# EUROPÄISCHE PATENTANMELDUNG

(11) **EP 0 521 368 A1**
(43) Veröffentlichungstag der Anmeldung: **07.01.1993**
(21) Anmeldenummer: 92110531.8
(22) Anmeldetag: 23.06.1992
(51) Int. Cl.: C07D 487/04, A61K 31/495, C07D 405/04, C07D 209/60, C07D 307/46, C07D 207/32

(54) **Bi- und tetracyclische Pyrrolopyrazine**

(30) Priorität: 02.07.1991 CH 1950/91; 22.05.1992 CH 1667/92
(71) Anmelder: F. HOFFMANN-LA ROCHE AG, 4002 Basel (CH)
(72) Erfinder: Röver, Stephan, W-7889 Grenzach-Wyhlen (DE)
(74) Vertreter: Poppe, Regina

(57) **Zusammenfassung**

Die neuen Pyrrolopyrazine der allgemeinen Formel 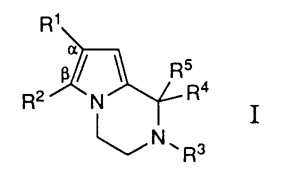
worin eines von R¹ und R² Aryl und das andere Wasserstoff, niederes Alkyl oder Aryl oder R¹ und R² zusammen mit den beiden mit α und β bezeichneten Kohlenstoff-Atomen die Gruppe A; 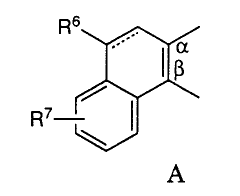
R³ Wasserstoff oder niederes Alkyl, R⁴ Wasserstoff, oder
R³ und R⁴ zusammen eine zusätzliche C/N-Bindung;
R⁵ Wasserstoff oder niederes Alkyl;
R⁶ Wasserstoff oder niederes Alkyl;
R⁷ Wasserstoff, Halogen, niederes Alkyl, gegebenenfalls substituiertes niederes Alkoxy, C₃₋₆-Cycloalkyl, C₄₋₆-Cycloalkenyl, C₃₋₆-Cycloalkyloxy, Hydroxy, Trifluormethansulfonat oder ein substituiertes Carbonat; und die punktierte Linie eine fakultative zusätzliche C/C-Bindung bedeuten, und pharmazeutisch annehmbare Säureadditionssalze der Verbindungen der Formel I können bei der Bekämpfung bzw. Verhütung von Krankheiten bzw. bei der Verbesserung der Gesundheit, insbesondere bei der Bekämpfung bzw. Verhütung von depressiven Zuständen, kognitiven Erkrankungen und neurodegenerativen Erkrankungen wie der Parkinsonschen Krankheit und der Alzheimerschen Krankheit verwendet werden.

## Beschreibung

Die vorliegende Erfindung betrifft Pyrrolopyrazine. Im speziellen betrifft sie Verbindungen der allgemeinen Formel 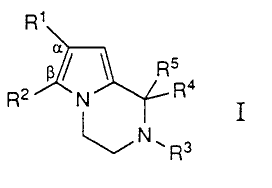
worin eines von R¹ und R² Aryl und das andere Wasserstoff, niederes Alkyl oder Aryl oder R¹ und R² zusammen mit den beiden mit α und β bezeichneten Kohlenstoff-Atomen die Gruppe A; 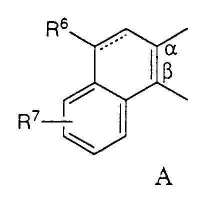
R³ Wasserstoff oder niederes Alkyl, R⁴ Wasserstoff, oder
R³ und R⁴ zusammen eine zusätzliche C/-N-Bindung;
R⁵ Wasserstoff oder niederes Alkyl;
R⁶ Wasserstoff oder niederes Alkyl;
R⁷ Wasserstoff, Halogen, niederes Alkyl, gegebenenfalls substituiertes niederes Alkoxy, C₃₋₆-Cycloalkyl, C₄₋₆-Cycloalkenyl, C₃₋₆-Cycloalkyloxy, Hydroxy, Trifluormethansulfonyloxy oder gegebenenfalls substituiertes Benzyloxycarbonyloxy und die punktierte Linie eine fakultative zusätzliche C/C-Bindung bedeuten, und pharmazeutisch annehmbare Säureadditionssalze der Verbindungen der Formel I.

Im Rahmen der vorliegenden Erfindung wurde überraschenderweise gefunden, dass die Verbindungen der Formel I bei geringer Toxizität interessante und therapeutisch verwertbare pharmakodynamische Eigenschaften aufweisen. In Tierversuchen konnte nämlich gezeigt werden, dass die Verbindungen der obigen Formel I sowie ihre pharmazeutisch annehmbaren Säureadditionssalze Monoaminooxidase (MAO) hemmende Eigenschaften besitzen.

Gegenstand der vorliegenden Erfindung sind die Verbindungen der allgemeinen Formel I sowie ihre pharmazeutisch annehmbaren Säureadditionssalze per se und als pharmazeutische Wirkstoffe, Arzneimittel, enthaltend eine Verbindung der allgemeinen Formel I oder ein pharmazeutisch annehmbares Säureadditionssalz davon, die Herstellung solcher Arzneimittel und die Verwendung von Verbindungen der allgemeinen Formel I sowie ihrer pharmazeutisch annehmbaren Säureadditionssalze bei der Bekämpfung bzw. Verhütung von Krankheiten bzw. bei der Verbesserung der Gesundheit, insbesondere bei der Bekämpfung bzw. Verhütung von depressiven Zuständen, kognitiven Erkrankungen und neurodegenerativen Erkrankungen wie der Parkinsonschen Krankheit und der Alzheimerschen Krankheit. Gegenstand vorliegender Erfindung sind schliesslich Verfahren zur Herstellung der Verbindungen der obigen Formel I und ihrer pharmazeutisch annehmbaren Säureadditionssalze sowie die in diesen Verfahren eingesetzten Zwischenprodukte.

Der Ausdruck "nieder" bezeichnet Reste und Verbindungen mit höchstens zwölf, vorzugsweise höchstens acht Kohlenstoffatomen. Der Ausdruck "Alkyl" bezeichnet gradkettige oder verzweigte, gesättigte Kohlenwasserstoffreste, wie beispielsweise Methyl, Ethyl, Propyl, Butyl, Isopropyl oder t-Butyl. Der Ausdruck "gegebenenfalls substituiertes Alkoxy" bezeichnet über ein Sauerstoffatom gebundene Alkylgruppen, wie beispielsweise Methoxy, Ethoxy, Propoxy, Butoxy, Octoxy, Isopropoxy oder tert.-Butoxy, welche ihrerseits durch Hydroxy oder einen über ein Stickstoffatom gebundenen fünf- oder sechsgliedrigen gesättigten Heterocyclus, welcher als Ringglied ein Sauerstoffatom enthalten kann oder durch eine Oxogruppe substituiert ist, substituiert sein können. Der Ausdruck "Cycloalkyl" bezeichnet cyclische gesättigte Kohlenwasserstoffe, wie beispielsweise Cyclopropyl oder Cyclohexyl. Der Ausdruck "Cycloalkenyl" bezeichnet cyclische ungesättigte Kohlenwasserstoffe, wie beispielsweise Cyclohexenyl. Der Ausdruck "Cycloalkyloxy" bezeichnet über ein Sauerstoffatom gebundene cyclische gesättigte Kohlenwasserstoffe, wie beispielsweise Cyclopentyloxy oder Cyclohexyloxy. Der Ausdruck "gegebenenfalls substituiertes Benzyloxycarbonyloxy" bezeichnet Benzyloxycarbonyloxy, welches gegebenenfalls am Phenylring substituiert ist, wie beispielsweise 4-Nitrobenzyloxycarbonyloxy. Der Ausdruck "Halogen" bezeichnet die Formen F, Cl und Br.

Der Ausdruck "Aryl" bezeichnet Phenyl oder durch Halogen, gegebenenfalls substituiertes niederes Alkoxy, niederes Alkyl, C₃₋₆-Cycloalkyl, C₄₋₆-Cycloalkenyl, Hydroxy, Trifluormethyl, Trifluormethansulfonyloxy, Phenyl-nieder-Alkoxy, C₃₋₆-Cycloalkyloxy oder durch gegebenenfalls substituiertes Benzyloxycarbonyloxy mono- oder disubstituiertes Phenyl oder durch niederes Alkylendioxy wie beispielsweise Methylendioxy oder durch Bicycloalkyloxy wie beispielsweise 2-Exonorbonyloxy substituiertes Phenyl.

Der Ausdruck "pharmazeutisch annehmbare Säureadditionssalze" umfasst Salze mit anorganischen und organischen Säuren, wie Salzsäure, Bromwasserstoffsäure, Salpetersäure, Schwefelsäure, Phosphorsäure, Zitronensäure, Ameisensäure, Fumarsäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Bevorzugte Verbindungen der Formel I sind solche, worin R¹ und R² zusammen die Gruppe A bedeutet, insbesondere solche, worin R⁷ C₃₋₆-Cycloalkyl, C₃₋₆-Cycloalkyloxy, Halogen, niederes Alkoxy oder niederes Alkyl bedeutet.

Bevorzugt sind auch Verbindungen, worin R¹ Wasserstoff und R² Aryl bedeutet.

Ganz besonders bevorzugte Verbindungen sind:
3,4-Dihydro-1-methyl-6-[p-(2-exonorbornyloxy)phenyl]pyrrolo[1,2-a]pyrazin.
3-Cyclopentyloxy-5,6,10,11- tetrahydro-8-methylbenzo[g]pyrazino[1,2-a]indol.
5,6,10,11-Tetrahydro-3-isopropoxy-8-methylbenzo[g]pyrazino[1,2-a]indol.
3-n-Butyl-5,6,10,11-tetrahydro-8-methylbenzo[g]pyrazino[1,2-a]indol.
3-Cyclohexyl-5,6,10,11-tetrahydro-8-methylbenzo[g]pyrazino[1,2-a]indol.
5,6,10,11-Tetrahydro-3,8-dimethylbenzo[g]pyrazino[1,2-a]indol.

Die Verbindungen der Formel I und deren pharmazeutisch verwendbare Säureadditionssalze lassen sich erfindungsgemäss herstellen, indem man
a) eine Verbindung der allgemeinen Formel 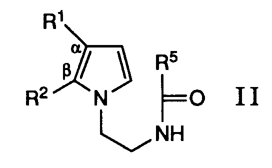 worin R¹ ,R² und R⁵ obige Bedeutung haben, mit der Massgabe, dass R¹ oder R² oder R¹ und R² zusammen keine durch Hydroxy, Trifluormethansulfonyloxy oder C₄₋₆-Cycloalkenyl substituierte Gruppe bedeuten, cyclisiert, oder
b) eine Verbindung der allgemeinen Formel 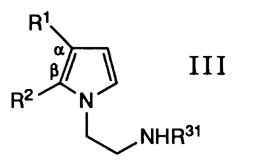 worin R¹ und R² obige Bedeutung haben und R³¹ Wasserstoff oder niederes Alkyl bedeutet, mit der Massgabe, dass R¹ oder R² oder R¹ und R² zusammen keine Gruppe bedeuten, welche durch Trifluormethansulfonyloxy oder C₄₋₆-Cycloalkenyl substituiert ist, mit einem Aldehyd der Formel

   R⁵-CHO IV

   wobei R⁵ die obige Bedeutung hat, umsetzt; oder
c) eine Verbindung der allgemeinen Formel 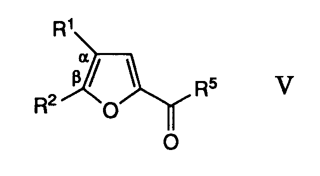 worin R¹,R² und R⁵ obige Bedeutung haben, mit 1,2-Ethandiamin umsetzt, oder
d) eine Verbindung der Formel 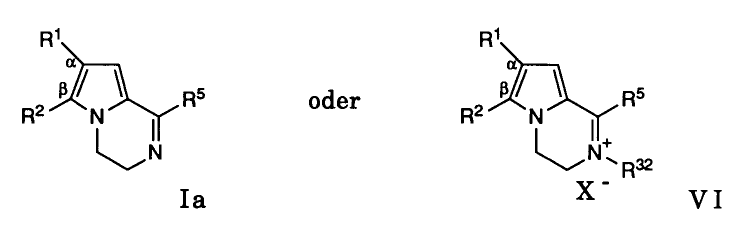 worin R¹,R² und R⁵ obige Bedeutung haben und R³² niederes Alkyl und X Halogen bedeutet, reduziert, oder
e) eine alkoxysubstituierte Verbindung der Formel I einer Etherspaltung unterzieht, oder
f) eine Verbindung der Formel I, worin R¹ und/oder R² oder R¹ und R² zusammen eine durch phenolisches Hydroxy substituierte Gruppe darstellen, in eine entsprechende Verbindung der Formel I überführt, worin R¹ und/oder R² oder R¹ und R² zusammen eine durch gegebenenfalls substituiertes Alkoxy, durch Phenylalkyloxy, durch C₃₋₆-Cycloalkyloxy oder durch Bicycloalkyloxy, substituierte Gruppe darstellen, oder
g) eine Verbindung der Formel I, worin R¹ und/oder R² oder R¹ und R² zusammen eine durch phenolisches Hydroxy substituierte Gruppe darstellen, in eine entsprechende durch Trifluormethansulfonyloxy substituierte Verbindung umwandelt, oder
h) eine Verbindung der Formel I, worin R¹ und/oder R² oder R¹ und R² zusammen eine durch Trifluormethansulfonyloxy substituierte Gruppe bedeuten, in eine entsprechende durch niederes Alkyl, Cyclopropyl oder C₄₋₆-Cycloalkenyl substituierte Verbindung überführt, oder
i)eine durch gegebenenfalls substituiertes Benzyloxycarbonyloxy substituierte Verbindung der Formel I spaltet, und
j) eine durch C₄₋₆-Cycloalken substituierte Verbindung der Formel I hydriert, und
k) erwünschtenfalls eine erhaltene Verbindung der Formel I in ein pharmazeutisch annehmbares Säureadditionssalz überführt.

Gemäss Verfahrensvariante a) können Verbindungen der Formel I hergestellt werden, wobei R¹, R² und R⁵ obige Bedeutung haben mit der Massgabe, dass R¹ oder R² oder R¹ und R² zusammen keine durch Hydroxy, Trifluormethansulfonyloxy oder C₄₋₆Cycloalkenyl substituierte Gruppe bedeuten und R³ und R⁴ zusammen eine zusätzliche C/N-Bindung bedeuten. Diese Cyclisation wird vorzugsweise durch Behandeln mit anorganischen Säurechloriden oder Säuren, beispielsweise mit Phosphoroxychlorid, durchgeführt. Die Reaktionstemperatur liegt vorzugsweise in einem Temperaturbereich von Raumtemperatur bis zur Rückflusstemperatur des Reaktionsgemisches, wobei man bevorzugt bei der Rückflusstemperatur arbeitet.

Gemäss Verfahrensvariante b) können Verbindungen der Formel I hergestellt werden, wobei R¹, R² und R⁵ obige Bedeutung haben, R³ Wasserstoff oder niederes Alkyl und R⁴ Wasserstoff bedeuten, mit der Massgabe, dass R¹ oder R² oder R¹ und R² zusammen keine Gruppe bedeuten, welche durch Trifluormethansulfonyloxy oder C₄₋₆-Cycloalken substituiert ist. Die Umsetzung erfolgt vorzugsweise in einem niederen Alkohol, wie beispielsweise Ethanol, und die Reaktion wird zweckmässigerweise bei erhöhter Temperatur, vorzugsweise bei der Rückflusstemperatur des Reaktionsgemisches, durchgeführt.

Gemäss Verfahrensvariante c) können Verbindungen der Formel I hergestellt werden, worin R¹, R² und R⁵ obige Bedeutung haben und R³ und R⁴ zusammen eine zusätzliche C/N-Bindung bedeuten. Diese Reaktion wird zweckmässigerweise bei erhöhter Temperatur, vorzugsweise bei der Rückflusstemperatur des Reaktionsgemisches, durchgeführt. Zweckmässigerweise dient als Lösungsmittel und gleichzeitig als Reagenz ein 1,2-Ethandiamin/Wasser-Gemisch, wobei das Verhältnis etwa 10:1 beträgt.

Gemäss Verfahrensvariante d) können Verbindungen der Formel I hergestellt werden, worin R¹, R² und R⁵ obige Bedeutung haben und R³ Wasserstoff oder niederes Alkyl und R⁴ Wasserstoff bedeuten. Für die Reduktion verwendet man vorzugsweise Natriumborhydrid und als Lösungsmittel vorzugsweise einen niederen Alkohol, wie Methanol oder Ethanol oder ein Gemisch eines niederen Alkohols mit Wasser, beispielsweise ein Methanol/Wasser-Gemisch. Die Reaktion wird in einem Temperaturbereich von Raumtemperatur bis zur Rückflusstemperatur der Reaktionsmischung durchgeführt, wobei die Raumtemperatur bevorzugt wird.

Gemäss Verfahrensvariante e) können Verbindungen der Formel I hergestellt werden, worin R¹ und/oder R² oder R¹ und R² zusammen eine durch Hydroxy substituierte Gruppe darstellen und R³, R⁴ und R⁵ die obige Bedeutung haben. Diese Reaktion wird beispielsweise durch Behandeln mit Bromwasserstoffsäure durchgeführt. Die Reaktionstemperatur liegt vorzugsweise in einem Temperaturbereich von Raumtemperatur bis zur Rückflusstemperatur des Reaktionsgemisches, wobei man vorzugsweise bei Raumtemperatur arbeitet.

Gemäss Verfahrensvariante f) können Verbindungen der Formel I hergestellt werden, worin R¹ und/oder R² oder R¹ und R² zusammen eine durch gegebenenfalls substituiertes Alkoxy, durch Phenylalkyloxy, durch C₃₋₆-Cycloalkyloxy oder durch Bicycloalkyloxy substituierte Gruppe darstellen und R³, R⁴ und R⁵ die obige Bedeutung haben. Die gewünschte Reaktion kann dadurch bewerkstelligt werden, dass man eine Verbindung der Formel I, worin R¹ und/oder R² oder R¹ und R² zusammen eine durch phenolisches Hydroxy substituierte Gruppe darstellen, mit einer Base, wie beispielsweise Natriumhydroxid, in Gegenwart eines Alkylierungsmittels behandelt. Als Alkylierungsmittel kommen beispielsweise Methyljodid, Benzylbromid, Bromcyclopentan, Bicyclo[2.2.1]-heptanbromid, 3-Hydroxy-1-butyltoluolsulfonat oder ähnliches in Frage. Geeignete Lösungsmittel sind beispielsweise Methylenchlorid, Dimethylformamid und dergleichen. Die Reaktion wird vorzugsweise in einem Temperaturbereich von Raumtemperatur bis zur Rückflusstemperatur des Reaktionsgemisches durchgeführt, wobei die Rückflusstemperatur besonders bevorzugt ist. Alternativ kann die Alkylierung auch in Gegenwart von Triphenylphosphin und einem Azodicarbonsäureester, wie beispielsweise Azodicarbonsäurediethylester, mit einem Alkohol als Alkylierungsmittel in einem inerten Lösungsmittel, wie Tetrahydrofuran bei einer Temperatur zwischen -20°C und Siedetemperatur des Lösungsmittel durchgeführt werden. Weiterhin kann die Alkylierung auch mit einer ungesättigten Verbindung wie Isobutylen in einem inerten Lösungsmittel wie Methylenchlorid in Gegenwart einer Säure wie Trifluormethansulfonsäure durchgeführt werden. Reaktionstemperatur ist bevorzugt -78°C bis -5°C.

Gemäss Verfahrensvariante g) können Verbindungen der Formel I hergestellt werden, worin R¹ und/oder R² oder R¹ und R² zusammen eine durch Trifluormethansulfonyloxy substituierte Gruppe darstellen, und R³, R⁴ und R⁵ die obige Bedeutung haben. Die gewünschte Reaktion kann dadurch bewerkstelligt werden, dass man eine Verbindung der Formel I, worin R¹ oder R² oder R¹ und R² zusammen eine durch phenolisches Hydroxy substituierte Gruppe bedeutet, mit einem reaktiven Trifluormethansulfonsäurederivat umsetzt. Vorzugsweise wird in einem inerten Lösungsmittel wie z.B. Dimethylacetamid mit einer starken Base wie z. B. Natriumhydrid das Phenolat erzeugt und dieses mit N-Phenyl-bis(trifluormethansulfonimid) umgesetzt. Die Reaktionstemperatur liegt zwischen 0°C und Raumtemperatur, vorzugsweise bei 0°C.

Gemäss Verfahrensvariante h) können Verbindungen der Formel I hergestellt werden, worin R¹ und/oder R² oder R¹ und R² zusammen eine durch niederes Alkyl, Cyclopropyl oder C₄₋₆-Cycloalkenyl substituierte Gruppe darstellen, und R³, R⁴ und R⁵ die obige Bedeutung haben. Die gewünschte Reaktion kann dadurch bewerkstelligt werden, dass man eilte Verbindung der Formel I, worin R¹ und/oder R² oder R¹ und R² zusammen eine durch Trifluormethansulfonyloxy substituierte Gruppe bedeutet, mit einer entsprechenden metallorganischen Verbindung umsetzt. Vorzugsweise wird die Reaktion in einem inerten Lösungsmittel wie Dimethylformamid bei erhöhter Temperatur, vorzugsweise bei 120°C, unter Zugabe eines Katalysators, vorzugsweise eines Palladiumkatalysators wie Bis(triphenylphosphin)palladiumdichlorid in Gegenwart von Cokatalysatoren wie Triphenylphosphin und Lithiumchlorid durchgeführt. Als metallorganische Verbindungen eignen sich beispielsweise Alkylzinnverbindungen wie Tetramethylzinn oder Tetra-n-butylzinn und ähnliche, oder Alkyl-cycloalkenylzinn-verbindungen wie Tri-n-butyl-1-cyclohexen-1-yl-zinn oder Tri-n-butyl-2-cyclohexen-1-yl-zinn.

Gemäss Verfahrensvariante i) können Verbindungen der Formel I hergestellt werden, worin R¹ und/oder R² oder R¹ und R² zusammen eine durch phenolisches Hydroxy substituierte Gruppe darstellen und R³, R⁴ und R⁵ die obige Bedeutung haben. Die gewünschte Reaktion kann dadurch bewerkstelligt werden, dass man eine Verbindung der Formel I, worin R¹ und/oder R² oder R¹ und R² zusammen eine durch gegebenenfalls substituiertes Benzyloxycarbonyloxy substituierte Gruppe darstellen, in einem inerten Lösungsmittel, wie Essigsäureethylester,, mit einem Hydrierkatalysator, wie Palladium auf Kohlenstoff, bei Raumtemperatur und Normaldruck hydriert oder aber indem man eine Verbindung der Formel I, worin R¹ und R² zusammen eine durch 4-Nitrobenzyloxycarbonyloxy substituierte Gruppe darstellen, in einem inerten Lösungsmittel wie Methylenchlorid mit einem Amin wie n-Propylamin bei Raumtemperatur spaltet.

Gemäss Verfahrensvariante j) können Verbindungen der Formel I hergestellt werden, worin R¹ und R² oder R¹ und R² zusammen eine durch C₄₋₆-Cycloalkyl substituierte Gruppe darstellen und R³,R⁴ und R⁵ die obige Bedeutung haben. Die gewünschte Reaktion kann dadurch bewerkstelligt werden, dass man eine durch C₄₋₆-Cycloalkenyl substituierte Verbindung der Formel I in einem inerten Lösungsmittel wie Methanol, unter Zusatz einer Säure wie Essigesäure oder Methansulfonsäure, mit einem Hydrierkatalysator wie Palladiumchlorid auf Kohle oder Tris(triphenylphosphin)rhodium(I)-chlorid vorzugsweise bei Raumtemperatur und Normaldruck hydriert.

Gemäss Verfahrensvariante k) können die Verbindungen der Formel I in pharmazeutisch annehmbare Säureadditionssalze übergeführt werden. Es kommen dabei sowohl Salze mit anorganischen als auch Salze mit organischen Säuren in Betracht. Beispiele derartiger Salze sind Hydrochloride, Hydrobromide, Nitrate, Sulfate, Phosphate, Citrate, Formiate, Fumarate, Maleate, Acetate, Succinate, Tartrate, Methansulfonate, p-Toluolsulfonate und dergleichen. Diese Salze können nach an sich bekannten und jedem Fachmann geläufigen Methoden hergestellt werden.

Die verschiedenen als Ausgangsstoffe verwendeten Verbindungen können beispielsweise gemäss den nachfolgenden Reaktionsschemata und den jeweils daran anschliessenden Beschreibungen der verschiedenen Reaktionen hergestellt werden. 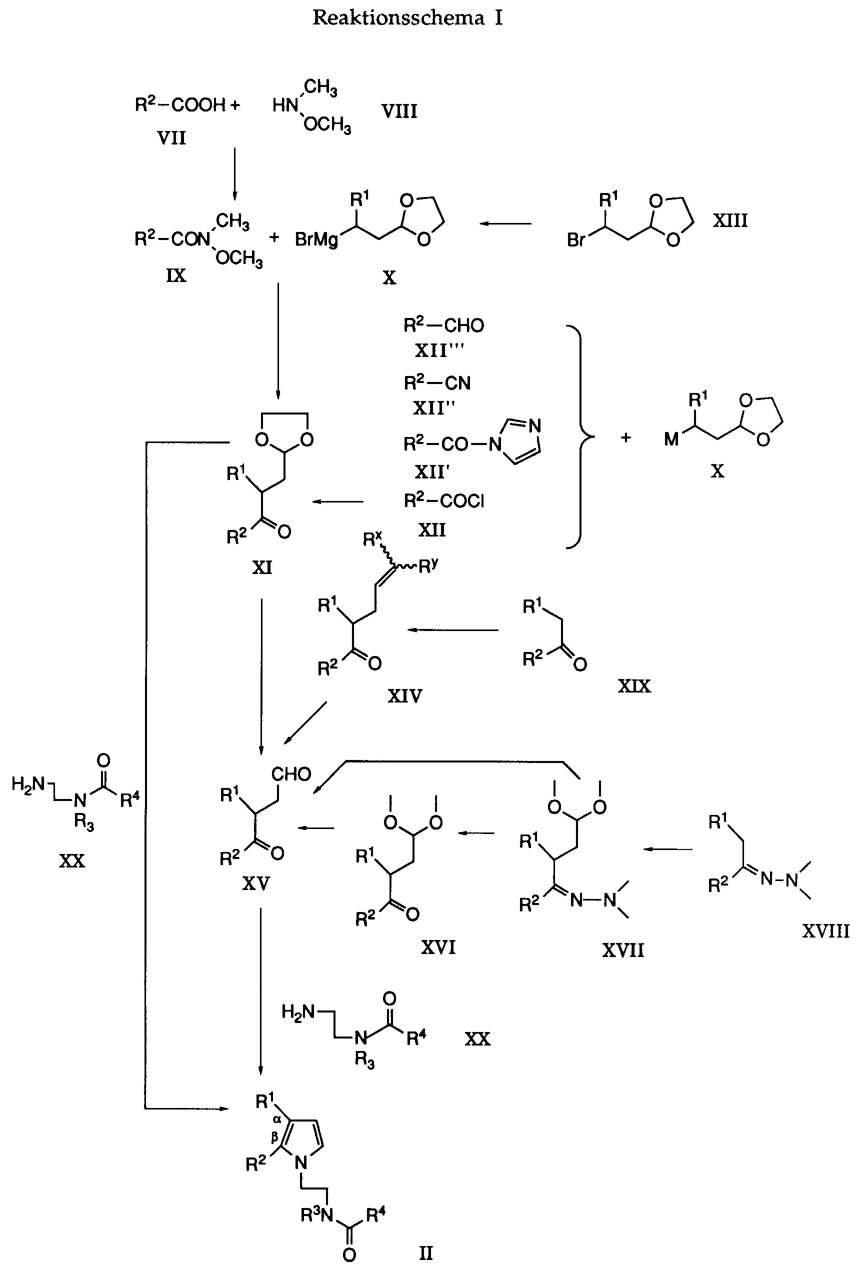
R¹, R² und R⁴ besitzen die oben angegebene Bedeutung, R³ bedeutet niederes Alkyl oder Wasserstoff, mit der Massgabe, dass R¹ oder R² oder R¹ und R² zusammen keine durch Hydroxy, Trifluormethansulfonyloxy, Benzyloxycarbonyloxy, substituiertes Benzyloxycarbonyloxy oder C₄₋₆-Cycloalkenyl substituierte Gruppe darstellen. Durch Behandeln einer Carbonsäure der Formel VII in Gegenwart eines geeigneten Kondensationsmittels mit N,O-Dimethylhydroxylamin (VIII) erhält man die N-Methoxy-N-methylamide IX. Geeignete Kondensationsmittel sind zum Beispiel Dicyclohexylcarbodiimid, 1,1'-Carbonyldiimidazol oder ähnliches. Die Umsetzung erfolgt in einem inerten Lösungsmittel, wie zum Beispiel Methylenchlorid, in einem Temperaturbereich von Raumtemperatur bis Siedetemperatur, vorzugsweise bei Raumtemperatur. Die Verbindungen der Formel IX gehören einer an sich bekannten Stoffklasse an. Derartige Verbindungen werden beispielsweise in Tetrahedron Letters 22, 3815 (1981) beschrieben.

Durch Umsetzung einer Verbindung der Formel IX mit einer Verbindung der Formel X erhält man eine Verbindung der Formel XI. Die Verbindungen der Formel X sind nach geläufigen Methoden aus Verbindungen der Formel XIII zugänglich. Diese Verbindungen der Formel XIII sind bekannt, siehe beispielsweise J. Org. Chem. 34, 1122 (1969) oder können auf analoge Weise hergestellt werden.

Die Verbindungen der Formel XI können aber auch durch Umsetzung eines reaktiven Carbonsäurederivates wie eines Carbonsäurechlorids der Formel XII, Imidazolids der Formel XII' oder Nitrils der Formel XII'' mit einer metallorganischen Verbindung des Typs X' auf im Prinzip bekannte Weise hergestellt werden.

Ebenso lassen sich die Verbindungen des Typs XI auf im Prinzip bekannte Weise auch durch Umsetzung eines Aldehyds der Formel XII''' mit einer metallorganischen Verbindung des Typs X und anschliessende Oxidation des sekundären Alkohols in einem inerten Lösungsmittel wie Aceton mit einem Oxidationsmittel wie Braunstein bevorzugt bei der Siedetemperatur des Lösungsmittels herstellen.

Verbindungen des Typs XI worin R¹ Alkyl bedeutet erhält man auch durch Alkylierung von Verbindungen des Typs XI worin R¹ Wasserstoff bedeutet, mit Alkylierungsreagentien wie Methyliodid in einem inerten Lösungsmittel wie Tetrahydrofuran und in Gegenwart von Basen wie Lithiumdiisopropylamin und Komplexierungsmitteln wie Hexamethylphosphorsäuretriamid.

Durch Hydrolyse einer Verbindung der Formel XI erhält man eine Verbindung der Formel XV. Diese Hydrolyse wird in Gegenwart einer Säure, beispielsweise Salzsäure, in einem pH-Bereich von etwa 0 - 2 durchgeführt. Diese Reaktion wird vorzugsweise in einem Temperaturbereich von ca. 0°C bis ca. 30°C durchgeführt. Als Lösungsmittel wird Tetrahydrofuran oder ähnliches verwendet.

Verbindungen der Formel XV sind auch durch Ozonolyse von Verbindungen der Formel XIV und anschliessende reduktive Aufarbeitung, wie zum Beispiel mit Dimethylsulfid, zugänglich. Diese Reaktion wird in Lösungsmitteln wie Methanol, Methylenchlorid/Methanol-Gemischen oder ähnlichem durchgeführt. Die Verbindungen der Formel XV gehören einer an sich bekannten Stoffklasse an und sind beispielsweise in Chem. Ber. 108, 1756 (1975) beschrieben und können auch nach der dort beschriebenen Methodik dargestellt werden.

Die Verbindungen der Formel XIV, worin eines von R^{x} und R^{y} Wasserstoff und das andere Wasserstoff oder niederes Alkyl bedeutet, sind durch Behandeln von Verbindungen der Formel XIX mit Allylalkohol oder gegebenenfalls alkylsubstituierten Allylalkoholen wie 3-Hydroxy-1-buten in Gegenwart eines sauren Katalysators wie zum Beispiel p-Toluolsulfonsäure und anschliessende sauer katalysierte Umlagerung nach bekannten Methoden zugänglich. Diese Reaktion wird vorzugsweise als Destillation mit einem Katalysator wie p-Toluolsulfonsäure und in Lösungsmitteln wie Toluol oder Benzol in Gegenwart von 2,2-Dimethoxypropan durchgeführt.

Durch Umwandlung eines Ketons der Formel XIX nach an sich bekannten Methoden in die Hydrazone XVIII und Alkylierung von Verbindungen der Formel XVIII erhält man Hydrazone der Formel XVII. Die Alkylierung wird in Gegenwart einer starken Base, beispielsweise n-Butyllithium, und mit Bromacetaldehyd-dimethylacetal als Alkylierungsmittel durchgeführt. Als Lösungsmittel wird vorzugsweise Tetrahydrofuran verwendet. Bevorzugt wird in einem Temperaturbereich von etwa -30°C bis Raumtemperatur gearbeitet.

Durch Hydrolyse von Verbindungen der Formel XVII erhält man Verbindungen der Formel XVI. Diese Reaktion wird unter oxidierenden Bedingungen, beispielsweise in Gegenwart eines Perjodats, in einem inerten Lösungsmittel wie Tetrahydrofuran oder ähnlichen Lösungsmitteln in Gegenwart von Wasser in einem Temperaturbereich von etwa 20 - 50°C durchgeführt. Die Verbindungen der Formeln XV, XVI, XVII und XVIII gehören an sich bekannten Stoffklassen an. Derartige Verbindungen werden beispielsweise in Heterocycles 26, 3141 (1987) beschrieben.

Die Ketone der Formel XIX sind bekannt, siehe beispielsweise J. Chem. Soc. 1959, 1017 oder können nach analogen Methoden hergestellt werden.

Verbindungen der Formel XVII können auch mit Übergangsmetallsalzen, beispielsweise Kupfer(II)chlorid, in Lösungsmitteln wie Tetrahydrofuran/Wasser-Gemischen und ähnlichen in Ketone der Formel XVI umgewandelt werden. Diese Reaktion wird bevorzugt in einem Temperaturbereich von Raumtemperatur bis zur Siedetemperatur des Gemisches durchgeführt, wobei die Raumtemperatur besonders bevorzugt ist.

Durch Hydrolyse von Verbindungen der Formel XVI erhält man Verbindungen der Formel XV. Die Hydrolyse wird in Lösungsmitteln wie Methylenchlorid und in Gegenwart von Oxalsäure/Wasser durchgeführt.

Verbindungen der Formel XV können auch direkt durch Hydrolyse aus Verbindungen der Formel XVII erhalten werden. Diese Hydrolyse wird in Gegenwart einer Säure, beispielsweise Salzsäure, in einem pH-Bereich von etwa 0 - 2 durchgeführt. Diese Reaktion wird vorzugsweise in einem Temperaturbereich von ca. 0° bis ca. 30°C durchgeführt. Als Lösungsmittel wird Tetrahydrofuran oder ähnliches verwendet.

Durch Behandeln einer Verbindung der Formel XV mit einem Amid der Formel XX erhält man die gewünschte Verbindung II. Diese Reaktion wird in Gegenwart einer Säure, beispielsweise Essigsäure, oder in einem inerten Lösungsmittel wie beispielsweise Methylenchlorid in Gegenwart von Molekularsieb durchgeführt. Bevorzugt wird bei Raumtemperatur bis zur Siedetemperatur des Reaktionsgemisches gearbeitet.

Die gewünschte Verbindung II kann auch durch Umsetzen einer Verbindung der Formel XI mit einem Amid der Formel XX erhalten werden. Diese Reaktion wird in Gegenwart von Essigsäure durchgeführt. Sie wird vorzugsweise bei der Rückflusstemperatur des Reaktionsgemisches durchgeführt.

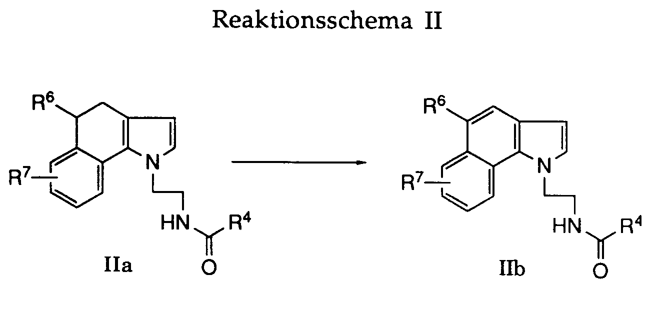

R⁴, R⁶ und R⁷ besitzen die oben angegebene Bedeutung.

Durch Behandeln einer Verbindung der Formel IIₐ mit einem Dehydrierungsmittel wie beispielsweise 2,3-Dichlor-5,6-dicyan-1,4-benzochinon in einem inerten Lösungsmittel wie Toluol, vorzugsweise bei der Siedetemperatur des Reaktionsgemisches, erhält man eine Verbindung der Formel II_{b}. Verbindungen der Formel IIa, d. h. Verbindungen der Formel II, worin R¹ und R² zusammen die Gruppe A bedeuten, jedoch ohne die fakultative zusätzliche C/C-Bindung, können gemäss Reaktionsschema I oder dergleichen hergestellt werden.

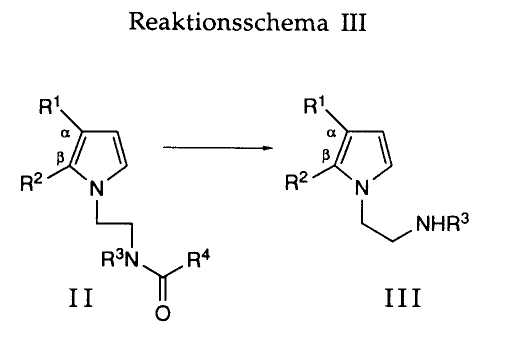

Durch Behandeln einer Verbindung der Formel II mit einem Alkalimetallhydroxid, z. B. Kaliumhydroxid, in einem Alkohol wie beispielsweise Ethylenglycol und in Gegenwart von Wasser, erhält man eine Verbindung der Formel III. Diese Reaktion wird vorzugsweise bei erhöhter Temperatur, insbesondere in einem Temperaturbereich von etwa 100°C bis etwa 120°C durchgeführt. Die Verbindungen der Formel II sind gemäss Reaktionsschema I oder dergleichen zugänglich.

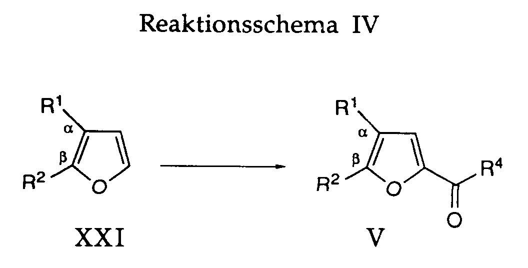

Die als Ausgangsstoffe verwendeten Verbindungen der Formel V können ausgehend von Verbindungen der allgemeinen Formel XXI, wobei R¹, R² und R⁴ obige Bedeutung besitzen, hergestellt werden, und zwar nach Methoden, welche an sich bekannt sind, vgl. zum Beispiel Heterocycles 4, 1021 (1976).

Verbindungen der Formel XXIa worin R² Methyl bedeutet, können ausgehend von Verbindungen der Formel XXIb, worin R² Wasserstoff bedeutet, durch Bromierung, Brom-Lithium-Austausch und Alkylierung mit Methyliodid hergestellt werden. Vorzugsweise wird die Bromierung in einem inerten Lösungsmittel wie Diethylether oder Tetrahydrofuran bei Raumtemperatur mit einem Bromierungsreagenz wie N-Bromsuccinimid durchgeführt. Der Brom-Lithium-Austausch wird vorzugsweise in den genannten Lösungsmitteln bei erniedrigter Temperatur, vorzugsweise bei - 78°C mit einer Lithiumalkylverbindung wie n-Butyllithium in Gegenwart eines Komplexierungsmittels wie N,N,N',N'-Tetramethylethylendiamin durchgeführt, vorteilhaft wird die Methylierung dann durch Zugabe von Methylierungsreagentien, wie Methyliodid, zur Reaktionslösung bei erniedrigter Temperatur, vorzugsweise zwischen -50 und -20°C erreicht.

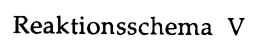

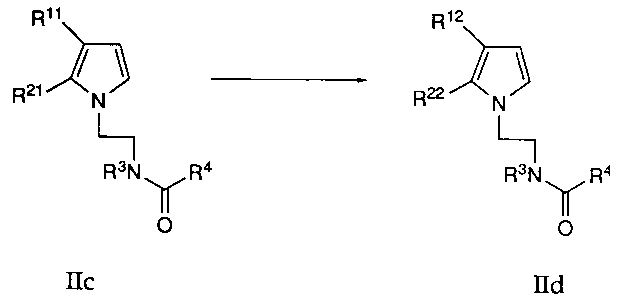

Verbindungen der Formel IId, worin R¹² oder R²² oder R¹² und R²² zusammen eine durch Hydroxy substituente Gruppe bedeuten und R³ und R⁴ die oben angegebene Bedeutung haben, können hergestellt werden indem man Verbindungen der Formel IIc worin R¹¹ oder R²¹ oder R¹¹ und R²¹ zusammen einen Alkoxysubstituenten tragen, einer Etherspaltung unterzieht. Vorzugsweise wird die Etherspaltung in einem inerten Lösungsmittel wie Methylenchlorid mit einer Lewissäure wie Bortribromid bei erniedrigter Temperatur, vorzugsweise bei -15°C bis Raumtemperatur durchgeführt.

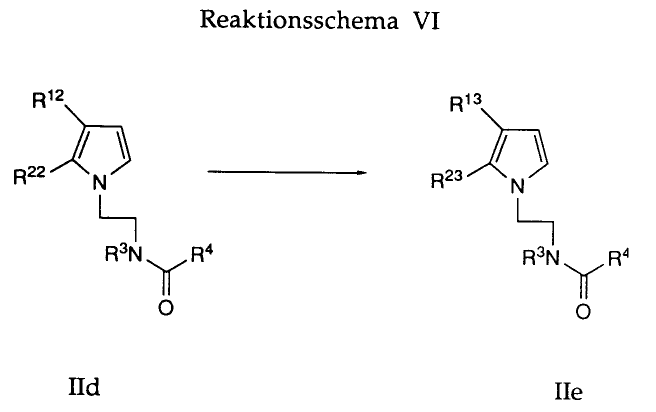

Verbindungen der Formel IIe, worin R¹³ oder R²³ oder R¹³ und R²³ zusammen ein Carbonat oder ein substituiertes Carbonat als Substituenten tragen, und R³ und R⁴ die oben angegebene Bedeutung haben, können hergestellt werden indem man Verbindungen der Formel IId, worin R¹² oder R²² oder R¹² und R²² zusammen einen Hydroxysubstituenten tragen, verestert. Vorzugsweise wird die Veresterung in inerten Lösungsmitteln wie Dioxan/Wasser-Gemischen mit einem reaktiven Kohlensäurederivat wie Benzylchloroformat oder 4-Nitrobenzylchloroformat in Gegenwart einer Base wie Natriumhydrogencarbonat bei Temperaturen zwischen Raumtemperatur und Siedetemperatur des Lösungsmittels, vorzugsweise bei Raumtemperatur durchgeführt.

Die als Zwischenprodukte verwendeten Verbindungen der Formeln II, III, V und VI sind neu und ebenfalls Gegenstand der vorliegenden Erfindung. Die übrigen als Ausgangsstoffe oder Zwischenprodukte verwendeten Verbindungen gehören an sich bekannten Stoffklassen an.

Die Verbindungen der Formel I und deren pharmazeutisch verwendbare Säureadditionssalze haben Monoaminoxidase (MAO) hemmende Aktivität. Auf Grund dieser Aktivität können die Verbindungen der Formel I und deren pharmazeutisch verwendbare Säureadditionssalze zur Behandlung von depressiven Zuständen, kognitiven Erkrankungen und neurodegenerativen Erkrankungen wie der Parkinsonschen Krankheit und der Alzheimerschen Krankheit verwendet werden.

Die MAO hemmende Aktivität der erfindungsgemässen Verbindungen kann unter Verwendung von Standardmethoden in vitro oder ex vivo bestimmt werden. Die zu prüfenden Präparate wurden in den nachfolgend beschriebenen Tests geprüft, welche sich an die von R.J. Wurtmann und J.A. Axelrod publizierte [Biochem. Pharmac. 12, 1439-41 (1963)] Methode anlehnen.

### in vitro:

Isolierte Rattengehirne werden im Verhältnis 1:4 (Gewicht/Volumen) in 0.1 molarem Kaliumphosphatpuffer (pH 7,4) homogenisiert, die Homogenate im Verhältnis 1:5 (Volumen/Volumen) mit demselben Puffer verdünnt und bei -20°C aufbewahrt. Zur Inkubation verwendet man eine Mischung folgender Zusammensetzung:
- 100 µl 1 M Phosphatpuffer (pH 7,4);
- 100 µl Lösung der zu prüfenden Substanz in Wasser oder in 10 %igem wässrigen Dimethylsulfoxid;
- 20 µl Rattenhirnhomogenat; und als Substrat
- 80 µl ¹⁴C-markiertes Serotonin (5-HT) bzw. ¹⁴C-markiertes Phenylethylamin (PEA) deren radioaktive Konzentration jeweils 100000 Zerfälle pro Minute beträgt und in einer Endkonzentration von 2 x 10⁻⁴ M bzw. 2 x 10⁻⁵ M eingesetzt wird.

Vor Zugabe des Substrates erfolgt während 30 Minuten eine Vorinkubation bei 37°C. Die Inkubation (in Gegenwart des Substrats) erfolgt ebenfalls bei 37°C und dauert 10 Minuten. Die Reaktion wird durch Zugabe von 200 µl 2N Salzsäure gestoppt.

Zur Extraktion der desaminierten Produkte wird, je nach Verwendung von 5-HT bzw. von PEA als Substrat, während 10 Minuten mit 5 ml Diethylether bzw. mit 5 ml n-Heptan geschüttelt, worauf man zentrifugiert, die Wasserphase im Trockeneisbad ausfriert, die organische Phase in Zählgläser überführt und die radioaktive Konzentration in einem β-Counter bestimmt.

Man erhält auf Grund der β-Zählerwerte die Aktivität der MAO im Vergleich zu Kontrollhomogenaten (ohne zu prüfende Substanz) und definiert als IC₅₀ diejenige Konzentration einer zu prüfenden Substanz, welche die Aktivität der MAO beim Substrat 5-HT bzw. PEA auf 50 % herabsetzt.

Die so ermittelte Aktivität einiger erfindungsgemässer Verbindungen wird aus den in der folgenden Tabelle aufgeführten IC₅₀-Werten ersichtlich:

| Verbindung | IC₅₀, µM | |
|---|---|---|
| | 5HT | PEA |
| 3,4-Dihydro-1-methyl-6-phenylpyrrolo[1,2-a]pyrazin | 0.3 | >>1 |
| 6-(p-Chlorphenyl)-3,4-dihydro-1-methylpyrrolo[1,2-a]pyrazin | 0.35 | 10 |
| 3,4-Dihydro-6-(p-methoxyphenyl)-1-methylpyrrolo[1,2a]pyrazin | 0.05 | >1 |
| 5,6,10,11-Tetrahydro-8-methylbenzo[g]pyrazino[1,2-a]indol | 0.03 | >1 |
| 5,6,8,9,10,11-Hexahydro-8-methylbenzo[g]pyrazino[1,2-a]-indol | 0.09 | >1 |
| 3,4-Dihydro-1-methyl-7-phenylpyrrolo[1,2-a]pyrazin | 0.09 | >>1 |
| 6-[p-(Cyclopentyloxy)phenyl]-3,4-dihydro-1-methylpyrrolo[1,2-a]pyrazin | 0.01 | <0.001 |
| 3,4-Dihydro-1-methyl-6-[p-(2-exonorbornyloxy)phenyl]pyrrolo[1,2-a]pyrazin | 0.02 | 0.003 |
| 3-Chlor-5,6,10,11-tetrahydro-8-methylbenzo[g]pyrazino[1,2-a]indol | 0.01 | >>1 |
| 5,6,10,11-Tetrahydro-3-methoxy-8-methyl-benzo[g]pyrazino[1,2-a]indol | 0.0008 | >1 |
| 5,6,10,11-Tetrahydro-3-isopropoxy-8-methyl-benzo[g]pyrazino[1,2-a]indol | 0.0004 | 0.014 |
| 3-Cyclopentyloxy-5,6,10,11-tetrahydro-8-methylbenzo[g]pyrazino[1,2-a]indol | 0.001 | 0.007 |
| 5,6,10,11-Tetrahydro-3,8-dimethyl-benzo[g]pyrazino[1,2-a]indol | 0.002 | >1 |
| 3-Cyclohexyl-5,6,10,11-tetrahydro-8-methylbenzo[g]pyrazino[1,2-a]indol | 0.002 | 0.16 |

### ex vivo:

Die zu testenden Verbindungen werden an männlichen Albino-Ratten (Fü-SPF, 100 - 140 g) auf Hemmung der MAO in Hirn und Leber geprüft. Die Testverbindungen werden als Lösung oder Suspension in Wasser in einer Dosis von 100 µmol pro Kilogramm Körpergewicht oral appliziert. Nach 2 Std. werden die Tiere betäubt und enthauptet. Das Hirn (ohne Cerebellum) sowie ein Teil der Leber werden präpariert und analog der oben beschriebenen Prozedur zur Bestimmung der MAO hemmenden Aktivität unterzogen, wobei die eingesetzte Enzymmenge im Falle des Hirns 20 µl eines 1:20- und im Falle der Leber 20 µl eines 1:80-Homogenates beträgt. Die Aktivität der Verbindungen wird aus dem Vergleich der β-Zählerwerte mit den Werten für mit Vehikel behandelte Kontrolltiere ermittelt und in Prozent der Kontrollwerte angegeben.

Die so ermittelte Aktivität einiger erfindungsgemässer Verbindungen wird aus den in der folgenden Tabelle aufgeführten Werten ersichtlich:

| | MAO-Hemmung in Prozent der Kontrolle | | | |
|---|---|---|---|---|
| Verbindung | Hirn | | Leber | |
| | 5HT | PEA | 5HT | PEA |
| 5,6,10,11-Tetrahydro-8-methylbenzo[g]pyrazino[1,2-a]indol | 48% | 87% | 47% | 81% |
| 5,6,8,9,10,11-Hexahydro-8-methylbenzo[g]pyrazino[1,2-a]indol | 69% | 95% | 55% | 78% |
| 6-[p-(Cyclopentyloxy)phenyl]-3,4-dihydro-1-methylpyrrolo-[1,2-a]pyrazin | 67% | 33% | 38% | 56% |
| 3-Chlor-5,6,10,11-tetrahydro-8-methylbenzo[g]pyrazino-[1,2-a]indol | 42% | 93% | 20% | 96% |
| ,4-Dihydro-1-methyl-6-[p-(2-exonorbornyloxy)phenyl]pyrrolo[1,2-a]pyrazin | 45% | 11% | 51% | 10% |
| 5,6,10,11-Tetrahydro-3-methoxy-8-methylbenzo[g]pyrazino[1,2-a]indol | 28% | 86% | 5% | 87% |
| 5,6,10,11-Tetrahydro-3-isopropoxy-8-methylbenzo[g]pyrazino[1,2-a]indol | 8% | 60% | 10% | 75% |
| 3-Cydopentyloxy-5,6,10,11-tetrahydro-8-methylbenzo[g]pyrazino[1,2-a]indol | 13% | 45% | 8% | 50% |
| 5,6,10,11-Tetrahydro-3,8-dimethyl-benzo[g]pyrazino[1,2-a]indol | 43% | 82% | 17% | 96% |

Die Toxizität der erfindungsgemässen Verbindungen wird geprüft, indem verschiedene Dosierungen der gelösten oder suspendierten Verbindungen Mäusen oral verabreicht werden. Pro Dosis wird je eine Maus eingesetzt. Dabei wird die Dosis schrittweise halbiert, so dass ein Dosisbereich von etwa 1,5 Zehnerpotenzen abgedeckt wird. Die Ergebnisse dieser preliminären Toxizitätsprüfung werden in Form von zwei Dosisangaben wiedergegeben.
Die höhere Dosis (LLD = lowest lethal dose) führte innerhalb von 24 Std. zum Tod, die niedrigere Dosis (HTD = highest tolerated dose) wurde überlebt. (Dosisangaben in Milligramm pro Kilogramm Körpergewicht, oral appliziert).

| Verbindung | HTD | LLD |
|---|---|---|
| 3,4-Dihydro-1-methyl-6-phenylpyrrolo[1,2-a]pyrazinfumarat (1:1) | 312 | 625 |
| 6-(p-Chlorphenyl)-3,4-dihydro-1-methylpyrrolo[1,2-a]pyrazin-fumarat (1:1) | 312 | 625 |
| 5,6,10,11-Tetrahydro-8-methylbenzo[g]pyrazino[1,2-a]indol-fumarat (2:3) | 156 | 312 |
| 5,6,8,9,10,11-Hexahydro-8-methylbenzo[g]pyrazino[1,2-a]indol-fumarat (1:1) | 312 | 625 |
| 6-[p-(Cyclopentyloxy)phenyl]-3,4-dihydro-1-methylpyrrolo[1,2-a]pyrazin-fumarat (1:1) | 312 | 625 |
| 3-Chlor-5,6,10,11-tetrahydro-8-methylbenzo[g]pyrazino[1,2-a]indol-fumarat (1:1) | 125 | 250 |
| 3-Chlor-5,6,8,9,10,11-hexahydro-8-methylbenzo[g]pyrazino[1,2-a]indol-fumarat (1:1) | 500 | 1000 |
| 3-Cyclopentyloxy-5,6,10,11-tetrahydro-8-methylbenzo[g]pyrazino[1,2-a]indol-fumarat (1:1) | 250 | 500 |

Die Verbindungen der Formel I und die pharmazeutisch annehmbaren Säureadditionssalze der Verbindungen der Formel I können als Heilmittel, z. B. in Form von pharmazeutischen Präparaten, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, planzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten. Arzneimittel enthaltend ein erfindungsgemässes Produkt und einen therapeutisch inerten Träger, sowie ein Verfahren zu deren Herstellung, das dadurch gekennzeichnet ist, dass man ein erfindungsgemässes Produkt und gegebenenfalls einen oder mehrere andere therapeutisch wertvolle Stoffe in eine galenische Darreichungsform bringt, sind ebenfalls Gegenstand der vorliegenden Erfindung.

Erfindungsgemäss kann man Verbindungen der allgemeinen Formel I sowie deren pharmazeutisch annehmbare Säureadditionssalze bei der Bekämpfung oder Verhütung von depressiven Zuständen, kognitiven Erkrankungen und neurodegenerativen Erkrankungen wie der Parkinsonschen Krankheit und der Alzheimerschen Krankheit verwenden. Die Dosierung kann innerhalb weiter Grenzen variieren und ist natürlich in jedem einzelnen Fall den individuellen Gegebenheiten anzupassen. Bei oraler Verabreichung liegt die tägliche Dosis in einem Bereich von etwa 50 - 500 mg einer Verbindung der allgemeinen Formel I, wobei aber die obere Grenze auch überschritten werden kann, wenn sich dies als angezeigt erweisen sollte.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

### Beispiel 1

a) Zu einer aus 44,9 g Magnesiumchlorid hergestellten Suspension von Riek-Magnesium in 1500 ml absolutem Tetrahydrofuran wurden unter Argon und Rühren binnen 15 Minuten bei maximal 30°C 40 ml 2-(2-Bromethyl)-1,3-dioxolan getropft. Die Suspension wurde 15 Minuten bei Raumtemperatur gerührt, auf 0°C abgekühlt und nach Zugabe von 43,5 g Kupfer(I)bromid 15 Minuten bei 5°C gerührt. Nach Abkühlen auf -70°C wurden binnen 10 Minuten 33 ml 3-Chlorbenzoylchlorid zugetropft. Das Reaktionsgemisch wurde 15 Minuten bei -70°C und 2 Stunden bei Raumtemperatur gerührt und anschliessend unter Kühlung bei maximal 20°C mit 1000 ml gesättigter Ammoniumchloridlösung hydrolysiert. Nach Zugabe von 500 ml Wasser wurde dreimal mit je 1000 ml Essigester extrahiert. Die vereinigten organischen Extrakte wurden mit 1000 ml gesättigter Natriumchloridlösung gewaschen, über MgSO₄ getrocknet und vom Lösungsmittel befreit. 59,2 g Oel wurden an 1500 g Kieselgel mit Essigester/Hexan (1:3) als Laufmittel chromatographiert.
   20,9 g (34%) 3'-Chlor-3-(1,3-dioxolan-2-yl)propiophenon wurden als gelbes Oel isoliert.
b) 10,0 g 3'-Chlor-3-(1,3-dioxolan-2-yl)propiophenon gelöst in 90 ml Tetrahydrofuran wurden unter Rühren und Argon bei Raumtemperatur mit 180 ml 2N Salzsäurelösung versetzt und über Nacht bei Raumtemperatur gerührt. Das Reaktionsgemisch wurde einmal mit 200 ml und zweimal mit je 100 ml Methylenchlorid extrahiert. Die organischen Extrakte wurden vereinigt, mit MgSO₄ getrocknet und vom Lösungsmittel befreit. Der Rückstand, 8,2 g gelbes Oel, wurde ohne weitere Reinigung in 20 ml Essigsäure gelöst und unter Argon und Rühren zu einer Lösung von 8,5 g N-Acetylethylendiamin in 50 ml Essigsäure gegeben. Die Reaktionslösung wurde 2 1/2 Stunden bei Raumtemperatur gerührt, anschliessend wurde die Essigsäure im Vakuum entfernt. Der Rückstand wurde in 200 ml Methylenchlorid aufgenommen und mit einem Gemisch aus 100 ml gesättigter Natriumhydrogencarbonatlösung und 60 ml 2N Natronlauge gewaschen. Die wässrige Phase wurde dreimal mit je 100 ml Methylenchlorid nachextrahiert. Die organischen Phasen wurden vereinigt, mit MgSO₄ getrocknet und vom Lösungsmittel befreit. Der Rückstand, 10,2 g braunes Oel, wurde an 500 g Kieselgel mit Essigester/Hexan (2:1) als Laufmittel chromatographiert.
   3,5 g (32%) N-[2-[2-(m-Chlorphenyl)pyrrol-1-yl]ethyl]acetamid wurden als gelbbraunes Oel isoliert.
   MS: m/e = 262(M⁺)
c) 3,5 g N-[2-[2-(m-Chlorphenyl)pyrrol-1-yl]ethyl]acetamid wurden unter Argon mit 15 ml Phosphoroxychlorid versetzt und 1 Stunde unter Rühren gekocht. Das Reaktionsgemisch wurde bei 0°C mit 100 ml 2N- und 50 ml 28%iger Natronlauge hydrolysiert, mit 1000 ml Wasser verdünnt und mit Diethylether (1 x 200ml, 2x100 ml) extrahiert. Die organischen Extrakte wurden vereinigt, einmal mit 100 ml Wasser gewaschen, über MgSO₄ getrocknet und vom Lösungsmittel befreit. 1,0 g von insgesamt 3,0 g Rohprodukt wurden in der 25fachen Menge Ethanol gelöst und mit 1,1 Aequivalenten einer gesättigten Lösung von Fumarsäure in Ethanol versetzt. Nach Zugabe von 30 ml Essigester kristallisierten 1,2 g (75%) 6-(m-Chlorphenyl)-3,4-dihydro-1-methylpyrrolo[1,2-a]pyrazin-fumarat; Schmp.: 158-160°C (Zers.).

### Beispiel 2

a) Zu einer aus 45,0 g Magnesiumchlorid hergestellten Suspension von Riek-Magnesium in 1500 ml absolutem Tetrahydrofuran wurden unter Argon und Rühren binnen 15 Minuten bei maximal 30°C 40 ml 2-(2-Bromethyl)-1,3-dioxolan getropft. Die Suspension wurde 15 Minuten bei Raumtemperatur gerührt, auf 0°C abgekühlt und nach Zugabe von 43,5 g Kupfer(I)bromid 15 Minuten bei 5°C gerührt. Nach Abkühlen auf -70°C wurden binnen 10 Minuten 33 ml 4-Chlorbenzoylchlorid zugetropft. Das Reaktionsgemisch wurde 15 Minuten bei -70°C und 2 Stunden bei Raumtemperatur gerührt und anschliessend unter Kühlung bei maximal 20°C mit 1000 ml gesättigter Ammoniumchloridlösung hydrolysiert. Nach Zugabe von 500 ml Wasser wurde dreimal mit je 1000 ml Essigester extrahiert. Die vereinigten organischen Extrakte wurden mit 1000 ml gesättigter Natriumchloridlösung gewaschen, über MgSO₄ getrocknet und vom Lösungsmittel befreit. 63,0 g Rückstand wurden an 2000 g Kieselgel mit Essigester/Hexan (1:1) als Laufmittel chromatographiert.
   36,5 g (59%) 4'-Chlor-3-(1,3-dioxolan-2-yl)propiophenon wurden als gelber, amorpher Feststoff isoliert.
b) 10,0 g 4'-Chlor-3-(1,3-dioxolan-2-yl)propiophenon gelöst in 100 ml Tetrahydrofuran wurden unter Rühren und Argon bei Raumtemperatur mit 180 ml 2N Salzsäurelösung versetzt und 5 Stunden bei Raumtemperatur gerührt. Das Reaktionsgemisch wurde einmal mit 250 ml und zweimal mit je 100 ml Methylenchlorid extrahiert. Die organischen Extrakte wurden vereinigt, mit MgSO₄ getrocknet und vom Lösungsmittel befreit. Der Rückstand, 8,1 g braunes Oel, wurde ohne weitere Reinigung in 30 ml Essigsäure gelöst und unter Argon und Rühren zu einer Lösung von 10,0 g N-Acetylethylendiamin in 60 ml Essigsäure gegeben. Die Reaktionslösung wurde 3 Stunden bei Raumtemperatur gerührt, anschliessend wurde die Essigsäure im Vakuum entfernt. Der Rückstand wurde in 150 ml Methylenchlorid aufgenommen und mit einem Gemisch aus 100 ml gesättigter Natriumhydrogencarbonatlösung und 200 ml 2N Natronlauge gewaschen. Die wässrige Phase wurde dreimal mit je 100 ml Methylenchlorid nachextrahiert. Die organischen Phasen wurden vereinigt, mit MgSO₄ getrocknet und vom Lösungsmittel befreit. Der Rückstand, 9,5 g braunes, amorphes Material, wurde an 500 g Kieselgel mit Essigester/Hexan (2:1) als Laufmittel chromatographiert.
   2,2 g (20%) N-[2-[2-(p-Chlorphenyl)pyrrol-1-yl]ethyl]acetamid wurden als braunes Oel isoliert.
   MS: m/e = 262 (M⁺)
c) 2,2 g N-[2-[2-(p-Chlorphenyl)pyrrol-1-yl]ethyl]acetamid wurden unter Argon mit 10 ml Phosphoroxychlorid versetzt und 45 Minuten unter Rühren gekocht. Das Reaktionsgemisch wurde bei 0°C mit 300 ml 2N Natronlauge hydrolysiert, mit 500 ml Wasser verdünnt und dreimal mit 100 ml Methylenchlorid extrahiert. Die organischen Extrakte wurden vereinigt, mit MgSO₄ getrocknet und vom Lösungsmittel befreit. 0,9 g von insgesamt 1,9 g Rohprodukt wurden in der 30fachen Menge Ethanol gelöst und mit 1,1 Aequivalenten einer gesättigten Lösung von Fumarsäure in Ethanol versetzt. Nach Zugabe von 25 ml Essigester kristallisierten 0,9 g (64%) 6-(p-Chlorphenyl)-3,4-dihydro-1-methylpyrrolo[1,2-a]pyrazin-fumarat (1:1); Schmp.: 187-189°C (Zers.).

### Beispiel 3

a) Zu einer aus 51,2 g Magnesiumchlorid hergestellten Suspension von Riek-Magnesium in 1300 ml absolutem Tetrahydrofuran wurden unter Argon und Rühren binnen 15 Minuten bei maximal 30°C 45 ml 2-(2-Bromethyl)-1,3-dioxolan getropft. Die Suspension wurde 15 Minuten bei Raumtemperatur gerührt, auf 0°C abgekühlt und nach Zugabe von 49,6 g Kupfer(I)bromid 15 Minuten bei 5°C gerührt. Nach Abkühlen auf -70°C wurden binnen 20 Minuten 50,0 g p-Anisoylchlorid gelöst in 150 ml absolutem Tetrahydrofuran zugetropft. Das Reaktionsgemisch wurde 30 Minuten bei -70°C und 2 Stunden bei Raumtemperatur gerührt und anschliessend unter Kühlung bei maximal 30°C mit 800 ml gesättigter Ammoniumchloridlösung hydrolysiert. Nach Zugabe von 500 ml Wasser wurde dreimal mit je 700 ml Essigester extrahiert. Die vereinigten organischen Extrakte wurden mit 1000 ml gesättigter Natriumchloridlösung gewaschen, über MgSO₄ getrocknet und vom Lösungsmittel befreit. 82,4 g dunkelbraunes Oel wurden an 2000 g Kieselgel mit Essigester/Hexan (1:1) als Laufmittel chromatographiert.
   48,8 g (71%) 3-(1,3-Dioxolan-2-yl)-4'-methoxypropiophenon wurden als gelboranges Oel isoliert.
b) 38,8 g 3-(1,3-Dioxolan-2-yl)-4'-methoxypropiophenon wurden in 100 ml Essigsäure gelöst und unter Rühren und Argon zu einer Lösung von 33,5 g N-Acetylethylendiamin in 500 ml Essigsäure gegeben. Das Reaktionsgemisch wurde über Nacht zum Rückfluss erhitzt, anschliessend wurde die Essigsäure im Vakuum entfernt. Der Rückstand wurde in 500 ml Methylenchlorid aufgenommen und mit einem Gemisch aus 200 ml gesättigter Natriumhydrogencarbonatlösung und 400 ml 2N Natronlauge gewaschen. Die wässrige Phase wurde dreimal mit je 100 ml Methylenchlorid extrahiert, die organischen Phasen wurden vereinigt, mit MgSO₄ getrocknet und vom Lösungsmittel befreit. Der Rückstand, 49,6 g braunes Oel, wurde an 500 g Kieselgel mit Essigester als Laufmittel chromatographiert.
   27,8 g (66%) N-[2-[2-(p-Methoxyphenyl)pyrrol-1-yl]ethyl]acetamid wurden als gelbbraunes Oel isoliert.
   MS: m/e =258 (M⁺)
c) 2,1 g N-[2-[2-(p-Methoxyphenyl)pyrrol-1-yl]ethyl]acetamid wurden unter Argon mit 10 ml Phosphoroxychlorid versetzt und 1/2 Stunde unter Rühren gekocht. Das Reaktionsgemisch wurde bei 0°C mit 100 ml 2N- und 50 ml 28%iger Natronlauge hydrolysiert, mit 600 ml Wasser verdünnt und dreimal mit je 100 ml Methylenchlorid extrahiert. Die organischen Extrakte wurden vereinigt, mit MgSO₄ getrocknet und vom Lösungsmittel befreit. 1,0 g von insgesamt 2,0 g Rohprodukt wurden in der 40fachen Menge Ethanol gelöst und mit 1,1 Aequivalenten einer gesättigten Lösung von Fumarsäure in Ethanol versetzt. Man erhielt 0,6 g (42%) 3,4-Dihydr-6-(p-methoxyphenyl)-1-methylpyrrolo[1,2-a]pyrazin-fumarat (1:1); Schmp.: 167-169°C (Zers.).

### Beispiel 4

a) 6,4 g 3-(1,3-Dioxolan-2-yl)-4'-(trifluormethyl)propiophenon wurden unter Rühren und Argon zu einer Lösung von 4,8 g N-Acetylethylendiamin in 70 ml Essigsäure gegeben. Das Reaktionsgemisch wurde über Nacht refluxiert, anschliessend wurde die Essigsäure im Vakuum entfernt. Der Rückstand wurde in 200 ml Methylenchlorid aufgenommen und mit einem Gemisch aus 100 ml gesättigter Natriumhydrogencarbonatlösung und 70 ml 2N Natronlauge gewaschen. Die wässrige Phase wurde zweimal mit je 100 ml Methylenchlorid nachextrahiert, die organischen Phasen wurden vereinigt, mit MgSO₄ getrocknet und vom Lösungsmittel befreit. Den Rückstand, 9,4 g gelbbrauner Feststoff, wurde an 500 g Kieselgel mit Essigester als Laufmittel chromatographiert. 5,4 g (78%) N-[2-[2-[4-(Trifluormethyl)phenyl]pyrrol-1-yl]ethyl]acetamid wurden als gelber amorpher Feststoff isoliert. MS: m/e = 296 (M⁺)
b) 5,4 g N-[2-[2-[4-(Trifluormethyl)phenyl]pyrrol-1-yl]ethyl]acetamid wurden unter Argon mit 25 ml Phosphoroxychlorid versetzt und 1 Stunde unter Rühren gekocht. Das Reaktionsgemisch wurde bei 0°C mit 100 ml 2N- und 150 ml 28%iger Natronlauge hydrolysiert, mit 1200 ml Wasser verdünnt und mit Methylenchlorid (1x300 ml, 2x100 ml) extrahiert. Die organischen Extrakte wurden vereinigt, mit MgSO₄ getrocknet und vom Lösungsmittel befreit. 2,5 g von insgesamt 5,0 g Rohprodukt wurden in der 30fachen Menge Ethanol gelöst und mit 1,1 Aequivalenten einer gesättigten Lösung von Fumarsäure in Ethanol versetzt. Man erhielt 2,7 g (76%) 3,4-Dihydro-1-methyl-6-[4-(trifluormethyl)phenyl]pyrrolo[1,2-a]pyrazin-fumarat (1:1); Schmp.: 165-167°C (Zers.).

### Beispiel 5

a) Zu einer aus 37,5 g Magnesiumchlorid hergestellten Suspension von Riek-Magnesium in 1200 ml absolutem Tetrahydrofuran wurden unter Argon und Rühren binnen 15 Minuten bei maximal 30°C 32,7 ml 2-(2-Bromethyl)-1,3-dioxolan getropft. Die Suspension wurde 15 Minuten bei Raumtemperatur gerührt, auf 0°C abgekühlt und nach Zugabe von 36,4 g Kupfer(I)bromid 15 Minuten bei 0°C gerührt. Nach Abkühlen auf -70°C wurden binnen 20 Minuten 45,0 g 3,-Dichlorbenzoylchlorid gelöst in 100 ml absolutem Tetrahydrofuran zugetropft. Das Reaktionsgemisch wurde 30 Minuten bei -70°C und 2 Stunden bei Raumtemperatur gerührt und anschliessend unter Kühlung bei maximal 20°C mit 800 ml gesättigter Ammoniumchloridlösung hydrolysiert. Nach Zugabe von 500 ml Wasser wurde dreimal mit Essigester (1x800 ml, 2x500 ml) extrahiert. Die vereinigten organischen Extrakte wurden über MgSO₄ getrocknet und vom Lösungsmittel befreit. Der Rückstand, 65,0 g braunes Oel, wurde an 1500 g Kieselgel mit Essigester/Hexan (1:3) als Laufmittel chromatographiert.
   30,4 g (51%) 3',4'-Dichlor-3-(1,3-dioxolan-2-yl)propiophenon wurden als gelboranges Oel isoliert.
b) 10,0 g 3',4'-Dichlor-3-(1,3-dioxolan-2-yl)propiophenon wurden in 20 ml Essigsäure gelöst und unter Rühren und Argon zu einer Lösung von 7,4 g N-Acetylethylendiamin in 80 ml Essigsäure gegeben. Das Reaktionsgemisch wurde über Nacht zum Rückfluss erhitzt, anschliessend wurde die Essigsäure im Vakuum entfernt. Der Rückstand wurde in 200 ml Methylenchlorid aufgenommen und mit einem Gemisch aus 100 ml gesättigter Natriumhydrogencarbonatlösung und 100 ml 2N Natronlauge gewaschen. Die wässrige Phase wurde zweimal mit je 100 ml Methylenchlorid nachextrahiert, die organischen Phasen vereinigt, mit MgSO₄ getrocknet und vom Lösungsmittel befreit. 12,5 g dunkelbraunes Oel wurden an 500 g Kieselgel mit Essigester als Laufmittel chromatographiert.
   9,1 g (84%) N-[2-[2-(3,4-Dichlorphenyl)pyrrol-1-yl]ethyl]acetamid wurden als gelbbraunes Oel isoliert.
   MS: m/e =296, 298, 300(9:6:1, M⁺)
c) 4,6 g N-[2-[2-(3,4-Dichlorphenyl)pyrrol-1-yl]ethyl]acetamid wurden unter Argon mit 25 ml Phosphoroxychlorid versetzt und 1 Stunde unter Rühren gekocht. Das Reaktlonsgemisch wurde bei 0°C mit 70 ml 2N- und 120 ml 28%iger Natronlauge hydrolysiert, mit 1400 ml Wasser verdünnt und mit Methylenchlorid (1x500 ml, 2x250 ml) extrahiert. Die organischen Extrakte wurden vereinigt, mit MgSO₄ getrocknet und vom Lösungsmittel befreit. 2,0 g von insgesamt 3,8 g Rohprodukt wurden in der 20fachen Menge Ethanol gelöst und mit 1,1 Aequivalenten einer gesättigten Lösung von Fumarsäure in Ethanol versetzt. Man erhielt 2,1 g (65%) 6-(3,4-Dichlorphenyl)-3,4-dihydro-1-methylpyrrolo-[1,2-a]pyrazin-fumarat (1:1); Schmp.: 193-195°C (Zers.).

### Beispiel 6

a) Zu einer aus 50,8 g Magnesiumchlorid hergestellten Suspension von Rieke-Magnesium in 1600 ml absolutem Tetrahydrofuran wurden unter Argon und Rühren binnen 15 Minuten bei maximal 30°C 44,3 ml 2-(2-Bromethyl)-1,3-dioxolan getropft. Die Suspension wurde 15 Minuten bei Raumtemperatur gerührt, auf 0°C abgekühlt und nach Zugabe von 49,3 g Kupfer(I)bromid 15 Minuten bei 0°C gerührt. Nach Abkühlen auf -70°C wurden binnen 20 Minuten 38,5 ml p-Toluoylchlorid zugetropft. Das Reaktionsgemisch wurde 30 Minuten bei -70°C und 2 Stunden bei Raumtemperatur gerührt und anschliessend unter Kühlung bei maximal 20°C mit 1000 ml gesättigter Ammoniumchloridlösung hydrolysiert. Nach Zugabe von 500 ml Wasser wurde einmal mit 1000 ml Essigester und zweimal mit je 500 ml Essigester extrahiert. Die vereinigten organischen Extrakte wurden über MgSO₄ getrocknet und vom Lösungsmittel befreit. Der Rückstand, 71,0 g braunes Oel, wurde an 1500 g Kieselgel mit Essigester/Hexan/Methylenchlorid (1:4:1) als Laufmittel chromatographiert.
   21,8 g (34%) 3-(1,3-Dioxolan-2-yl)-4'-methylpropiophenon wurden als gelboranges Oel isoliert.
b) 10,2 g 3-(1,3-Dioxolan-2-yl)-4'-methylpropiophenon wurden in 20 ml Essigsäure gelöst und unter Rühren und Argon zu einer Lösung von 9,4 g N-Acetylethylendiamin in 100 ml Essigsäure gegeben. Das Reaktionsgemisch wurde über Nacht zum Rückfluss erhitzt, anschliessend wurde die Essigsäure im Vakuum entfernt. Der Rückstand wurde in 200 ml Methylenchlorid aufgenommen und mit einem Gemisch aus 100 ml gesättigter Natriumhydrogencarbonatlösung und 150 ml 2N Natronlauge gewaschen. Die wässrige Phase wurde zweimal mit je 100 ml Methylenchlorid nachextrahiert. Die organischen Phasen wurden vereinigt, mit MgSO₄ getrocknet und vom Lösungsmittel befreit. Der Rückstand, 14,0 g dunkelbraunes Oel, wurde an 550 g Kieselgel mit Essigester/Hexan (2:1) als Laufmittel chromatographiert.
   7,8 g (70%) N-[2-(2-p-Tolylpyrrol-1-yl)ethyl]acetamid wurden als braunes Oel isoliert.
   MS: m/e = 242 (M⁺)
c) 3,9 g N-[2-(2-p-Tolylpyrrol-1-yl]ethyl]acetamid wurden unter Argon mit 20 ml Phosphoroxychlorid versetzt und 1/2 Stunde unter Rühren gekocht. Das Reaktionsgemisch wurde bei 0°C mit 100 ml 2N- und 100 mi 28%iger Natronlauge hydrolysiert, mit 1000 ml Wasser verdünnt und mit Methylenchlorid (1x200 ml, 2x100 ml) extrahiert. Die organischen Extrakte wurden vereinigt, mit MgSO₄ getrocknet und vom Lösungsmittel befreit. 1,7 g von insgesamt 3,5 g Rohprodukt wurden in der 25fachen Menge Ethanol gelöst und mit 1,1 Aequivalenten einer gesättigten Lösung von Fumarsäure in Ethanol versetzt. Man erhielt 1,8 g (68%) 3,4-Dihydro-1-methyl-6-p-tolylpyrrolo[1,2-a]pyrazin-fumarat (1:1); Schmp.: 186-189°C (Zers.).

### Beispiel 7

a) Zu einer Suspension von 50,0 g 3,4-Methylendioxy-benzoesäure in 500 ml Methylenchlorid unter Argon wurden portionenweise binnen 30 Minuten unter Rühren 48,8 g 1,1'-Carbonyl-diimidazol gegeben. Die entstandene klare Lösung wurde 2 Stunden bei Raumtemperatur gerührt und binnen 10 Minuten 42 ml Triethylamin zugetropft. Danach wurden 29,4 g N,O-Dimethylhydroxylamin-hydrochlorid zugegeben und über Nacht bei Raumtemperatur gerührt. Nach Zugabe von 800 mi 30%iger Zitronensäurelösung wurde zweimal mit je 300 ml Methylenchlorid extrahiert. Die organischen Phasen wurden sukzessive mit 300 ml 30%iger Zitronensäurelösung, 500 und 300 ml gesättigter Natriumhydrogencarbonatlösung gewaschen. Die organischen Phasen wurden vereinigt, mit MgSO₄ getrocknet und vom Lösungsmittel befreit. 60,9 g (97%) N-Methoxy-N-methyl-1,3-benzodioxol-5-carboxamid wurden als hellgelbes Oel isoliert.
b) Zu einer aus 5,1 g Magnesiumchlorid hergestellten Suspension von Rieke-Magnesium in 150 ml absolutem Tetrahydrofuran wurden unter Argon und Rühren binnen 20 Minuten bei maximal 30°C 4,5 ml 2-(2-Bromethyl)-1,3-dioxolan getropft. Die Suspension wurde 30 Minuten bei Raumtemperatur gerührt, auf -70°C abgekühlt und binnen 10 Minuten mit 6,1 g N-Methoxy-N-methyl-1,3-benzodioxol-5-carboxamid versetzt. Das Reaktionsgemisch wurde 30 Minuten bei -70°C und 2 Stunden bei Raumtemperatur gerührt und anschliessend unter Kühlung bei maximal 20°C mit 100 ml gesättigter Ammoniumchloridlösung hydrolysiert. Nach Zugabe von 50 ml Wasser wurde einmal mit 150 ml und zweimal mit je 100 ml Essigester extrahiert. Die vereinigten organischen Extrakte wurden über MgSO₄ getrocknet und vom Lösungsmittel befreit. Der Rückstand, 8,0 g gelbes Oel, wurde an 490 g Kieselgel mit Essigester/Hexan (1:5) als Laufmittel chromatographiert.
   5,3 g (72%) 1-(1,3-Benzodioxol-5-yl)-3-(1,3-dioxol-2-yl)-1-propanon wurden als farbloser Feststoff isoliert. Eine Probe kristallisierte farblos aus der 20fachen Menge Diethylether und schmolz bei 94-96°C.
c) 1,7 g 1-(1,3-Benzodioxol-5-yl)-3-(1,3-dioxol-2-yl)-1-propanon wurden unter Rühren und Argon zu einer Lösung von 1,4 g N-Acetylethylendiamin in 20 ml Essigsäure gegeben. Das Reaktionsgemisch wurde über Nacht refluxiert, anschliessend wurde die Essigsäure im Vakuum entfernt. Der Rückstand wurde in 80 ml Methylenchlorid aufgenommen und mit einem Gemisch aus 50 ml gesättigter Natriumhydrogencarbonatlösung und 10 ml 2N Natronlauge gewaschen. Die wässrige Phase wurde zweimal mit je 50 ml Methylenchlorid nachextrahiert, die organischen Phasen vereinigt, mit MgSO₄ getrocknet und vom Lösungsmittel befreit. Der Rückstand, 2,6 g braunes Oel, wurde an 50 g Kieselgel mit Essigester/Hexan (2:1) als Laufmittel chromatographiert.
   1,7 g (95%) N-[2-[2-(1,3-Benzodioxol-5-yl)pyrrol-1-yl]ethyl]acetamid wurden als gelbes Oel isoliert.
   MS: m/e = 272 (M⁺)
d) 1,7g N-[2-[2-(1,3-Benzodioxol-5-yl)pyrrol-1-yl]ethyl]acetamid wurden unter Argon mit 10 ml Phosphoroxychlorid versetzt und 45 Minuten unter Rühren gekocht. Das Reaktionsgemisch wurde bei 0°C mit 50 ml 2N- und 50 mi 28%iger Natronlauge hydrolysiert, mit 600 ml Wasser verdünnt und mit Methylenchlorid (1x100 ml, 2x60 ml) extrahiert. Die organischen Extrakte wurden vereinigt, mit MgSO₄ getrocknet und vom Lösungsmittel befreit. 1,4 g Rohprodukt wurden in der 20fachen Menge Ethanol gelöst und mit 1,1 Aequivalenten einer gesättigten Lösung von Fumarsäure in Ethanol versetzt. Man erhielt 1,5 g (66%) 6-(1,3-Benzodioxol-5-yl)-3,4-dihydro-1-methylpyrrolo[1,2-a]pyrazin-fumarat (1:1); Schmp.: 191-193°C (Zers.).

### Beispiel 8

a) Zu einer aus 43,5 g Magnesiumchlorid hergestellten Suspension von Rieke-Magnesium in 1300 ml absolutem Tetrahydrofuran wurden unter Argon und Rühren binnen 15 Minuten bei maximal 30°C 38 ml 2-(2-Bromethyl)-1,3-dioxolan getropft. Die Suspension wurde 15 Minuten bei Raumtemperatur gerührt, auf 0°C abgekühlt und nach Zugabe von 42,2 g Kupfer(I)bromid 15 Minuten bei 0°C gerührt. Nach Abkühlung auf -70°C wurden binnen 20 Minuten 50,0 g 3,4-Dimethoxybenzoylchlorid in 200 ml absolutem Tetrahydrofuran zugetropft. Das Reaktionsgemisch wurde 30 Minuten bei -70°C und 2 Stunden bei Raumtemperatur gerührt und anschliessend unter Kühlung bei maximal 20°C mit 1000 ml gesätrigter Ammoniumchloridlösung hydrolysiert. Nach Zugabe von 500 ml Wasser wurde dreimal mit Essigester (1x1000ml, 2x500 ml) extrahiert. Die vereinigten organischen Extrakte wurden mit 1000 ml gesättigter Natriumchloridlösung gewaschen, über MgSO₄ getrocknet und vom Lösungsmittel befreit. Der Rückstand, 69,5 g dunkelbraunes Oel, wurde an 1000 g Kieselgel mit Essigester/Hexan (1:1) als Laufmittel chromatographiert. Man erhielt 26,5 g Rohprodukt, die in der 12fachen Menge n-Hexan unter Zugabe von 100 ml Methylenchlorid gelöst wurden. Nach Abdestillieren von 50 ml Methylenchlorid fielen 22,3 g (34%) 3-(1,3-Dioxolan-2-yl)-3',4'-dimethoxypropiophenon als farblose Kristalle, die bei 103-105°C schmolzen.
b) 6,3 g 3-(1,3-Dioxolan-2-yl)-3',4'-dimethoxypropiophenon wurden in 30 ml Essigsäure gelöst und unter Rühren und Argon zu einer Lösung von 4,9 g N-Acetylethylendiamin in 50 ml Essigsäure gegeben. Das Reaktionsgemisch wurde über Nacht zum Rückfluss erhitzt, anschliessend wurde die Essigsäure im Vakuum entfernt. Der Rückstand wurde in 150 ml Methylenchlorid aufgenommen und mit einem Gemisch aus 100 ml gesättigter Natriumhydrogencarbonallösung und 150 ml 2N Natronlauge gewaschen. Die wässrige Phase wurde dreimal mit je 100 ml Methylenchlorid nachextrahiert, die organischen Phasen wurden vereinigt, mit MgSO₄ getrocknet und vom Lösungsmittel befreit. Der Rückstand, 7,3 g dunkelbraunes Oel, wurde an 250 g Kieselgel mit Essigester als Laufmittel chromatographiert.
   2,6 g (38%) N-[2-[2-(3,4-Dimethoxyphenyl)pyrrol-1-yl]ethyl]acetamid wurden als braunes Oel isoliert.
c) 2,6 g N-[2-[2-(3,4-Dimethoxyphenyl)pyrrol-1-yl]ethyl]acetamid wurden unter Argon mit 20 ml Phosphoroxychlorid versetzt und 1 Stunde unter Rühren gekocht. Das Reaktionsgemisch wurde bei 0°C mit 100 ml 2N- und 100 ml 28%iger Natronlauge hydrolysiert, mit 1200 ml Wasser verdünnt und mit Methylenchlorid (1x200 ml, 2x100 ml) extrahiert. Die organischen Extrakte wurden vereinigt, mit MgSO₄ getrocknet und vom Lösungsmittel befreit. 1,4 g von insgesamt 2,7 g Rohprodukt wurden an 30 g Kieselgel mit Methylenchlorid/Methanol (19:1) als Laufmittel chromatographiert. Das Produkt, 0,8 g braunes Oel, wurde in der 40fachen Menge Ethanol gelöst, mit 1,1 Aequivalenten einer gesättigten Lösung von Fumarsäure im Ethanol versetzt und auf ein Volumen von 10 ml eingeengt. Man erhielt 0,8 g 6-(3,4-Dimethoxyphenyl)-3,4-dihydro-1-methylpyrrolo[1,2-a]pyrazin-fumarat (1:1,3); Schmp.: 158-160°C (Zers.). Eine Probe wurde aus der 40fachen Menge Ethanol umkristallisiert und lieferte das (1:1)-Fumarat, das bei 160-161°C unter Zersetzung schmolz.

### Beispiel 9

a) 5,0 g 4,4-Dimethoxy-1-phenyl-1-butanon-(E/Z)-dimethylhydrazon gelöst in 20 ml Tetrahydrofuran wurden unter Argon mit 40 ml 2N Salzsäurelösung versetzt, so dass die Temperatur nicht über 10°C (Eisbad) stieg. Das Reaktionsgemisch wurde 3 Stunden bei Raumtemperatur gerührt und dann dreimal mit je 25 ml Essigester extrahiert. Die organischen Phasen wurden vereinigt, über MgSO₄ getrocknet und vom Lösungsmittel befreit. Der braune, ölige Rückstand (2,5 g) wurde ohne weitere Reinigung in 5 ml Essigsäure gelöst und unter Argon und Rühren bei Raumtemperatur zu einer Lösung von 3,2 g N-Acetylethylendiamin in 15 ml Essigsäure gegeben. Es wurde 1/2 Stunde bei Raumtemperatur gerührt und anschliessend die Essigsäure bei 37°C im Vakuum entfernt. Der Rückstand wurde in Methylenchlorid aufgenommen, mit gesättigter Natriumhydrogencarbonatlösung und Wasser gewaschen, mit MgSO₄ getrocknet und vom Lösungsmittel befreit. Das Rohprodukt, 3,0 g braunes Oel, wurde an 250 g Kieselgel mit Essigester/Hexan (1:1) als Laufmittel chromatographiert. 1,3 g (37%) N-[2-(2-Phenylpyrrol-1-yl)ethyl]-acetamid wurden als gelber, amorpher Feststoff isoliert.
   MS: m/e = 228 (M⁺)
b) 5.0 g N-[2-(2-Phenylpyrrol-1-yl)ethyl]acetamid wurden unter Argon zu 30 ml Phosphoroxychlorid gegeben und das Gemisch wurde 1 Stunde unter Rückfluss gekocht. Das abgekühlte Gemisch wurde mit 100 ml 2N- und 230 ml 28%iger Natronlauge hydrolysiert (Eiskühlung), mit 2000 ml Wasser verdünnt und mit Methylenchlorid (1x350 ml, 3x200 ml) extrahiert. Die organischen Phasen wurden vereinigt, mit MgSO₄ getrocknet und vom Lösungsmittel befreit. 1,0 g von insgesamt 4,5 g Rohprodukt wurden in der 30fachen Menge Ethanol gelöst und mit 1,1 Aequivalenten einer gesättigten Lösung von Fumarsäure in Ethanol versetzt. Man erhielt 0,9 g (57%) 3,4-Dihydro-1-methyl-6-phenylpyrrolo[1,2-a]pyrazin-fumarat (1:1); Schmp.: 175°C (Zers.).

### Beispiel 10

a) Unter Argon wurden zu einer Lösung von 27,5 g 1,2,3,4-Tetrahydro-1-oxo-2-naphtalinacetaldehyd in 275 ml Diethylether sukzessive 200 g Molekularsieb 4 Å und 14,9 g N-Acetylethylendiamin gegeben. Die Reaktionsmischung wurde 5 Stunden bei Siedetemperatur gerührt, und anschliessend abgekühlt. Das Molekularsieb wurde abfiltriert, das Filtrat vom Lösungsmittel befreit und das Rohprodukt an 600 g Kieselgel mit Essigester als Laufmittel chromatographiert. Man erhielt 25,3 g (79%) N-[2-(4,5-Dihydro-1H-benz[g]indol-1-yl)ethyl]acetamid, mit einem Schmelzpunkt von 95-96°C (Essigester).
b) Unter Argon wurden 5,3 g N-[2-(4,5-Dihydro-1H-benz[g]indol-1-yl)-ethyl]acetamid mit 60 ml Phosphoroxychlorid 1 Stunde bei Raumtemperatur gerührt. Das Reaktionsgemisch wurde bei 0°C mit 500 ml 28%iger Natronlauge hydrolysiert, mit 4000 ml Wasser verdünnt und dreimal mit je 300 ml Methylenchlorid extrahiert. Die organischen Phasen wurden vereinigt, mit MgSO₄ getrocknet und vom Lösungsmittel befreit. 2,0 g von insgesamt 5,0 g Rückstand wurden in der 30fachen Menge Ethanol gelöst und mit 1,1 Aequivalenten einer gesättigten Lösung von Fumarsäure in Ethanol versetzt. Man erhielt 2,3 g (67%) 5,6,10,11-Tetrahydro-8-methylbenzo[g]pyrazino[1,2-a]indol-fumarat (2:3); Schmp.: 181°C (Zers.).

### Beispiel 11

a) 44,1 g 5-Chlor-3,4-dihydro-1(2H)-naphtalenon wurden unter Argon in 200 ml Ethanol suspendiert, mit 55,7 ml N,N-Dimethylhydrazin (asym.) versetzt und während 96 Stunden unter Rückfluss gekocht. Das Reaktionsgemisch wurde im Vakuum vom Lösungsmittel und überschüssigem Dimethylhydrazin befreit. Der Rückstand wurde in 300 ml Methylenchlorid aufgenommen, mit MgSO₄ getrocknet und vom Lösungsmittel befreit.
   52,8 g (97%) 5-Chlor-3,4-dihydro-1(2H)-naphtalenon-N',N'-dimethylhydrazon wurden ohne weitere Reinigung weiterverwendet.
b) 52,8 g 5-Chlor-3,4-dihydro-1(2H)-naphtalenon-N',N'-dimethylhydrazon wurden unter Argon in 800 ml absolutem Tetrahydrofuran gelöst und mit 43 ml N,N,N',N'-Tetramethylethylendiamin versetzt. Die Reaktionslösung wurde auf -70°C abgekühlt und unter Rühren binnen 45 Minuten 150 ml einer 1,6 N Lösung von n-Butyllithium in Hexan zugetropft. Es wurde weitere 30 Minuten bei -70°C gerührt und dann bei -30°C binnen 15 Minuten 34 ml Bromacetaldehyd-dimethylacetal zugetropft. Anschliessend wurde 2 Stunden bei -30°C und 16 Stunden bei Raumtemperatur gerührt. Das Reaktionsgemisch wurde mit 1000 ml Wasser hydrolysiert, einmal mit 1000 ml und zweimal mit je 500 ml Essigester extrahiert. Die vereinigten organischen Extrakte wurden über MgSO₄ getrocknet und vom Lösungsmittel befreit. Das Rohprodukt, 81,1 g braunes Oel, wurde an 2000 g Kieselgel mit Hexan/Essigester-Gemischen (6:1 bis 1:2) als Laufmittel chromatographiert. Man erhielt 47,5 g (64%) 5-Chlor-1-(dimethylhydrazono)-1,2,3,4-tetrahydro-2-naphtalinacetaldehyd-dimethylacetal.
c) 47,5 g 5-Chlor-1-(dimethylhydrazono)-1,2,3,4-tetrahydro-2-naphtalinacetaldehyd-dimethylacetal wurden unter Argon in einem Gemisch von 2300 ml Tetrahydrofuran und 780 ml 0,067 M Phosphatpuffer vom pH-Wert 7 gelöst. Die Lösung wurde mit 26,1 g Kupfer(II)chlorid-dihydrat versetzt und bei Raumtemperatur 16 Stunden gerührt. Zum Reaktionsgemisch wurden 2000 ml Essigester gegeben und dreimal mit je 2000 ml eines Gemisches aus 25%iger Ammoniaklösung und gesättigter Ammoniumchloridlösung (1:19) gewaschen. Die Waschwasserphasen wurden einmal mit 1500 ml und einmal mit 1000 ml Essigester extrahiert, die organischen Phasen wurden vereinigt, über MgSO₄ getrocknet und vom Lösungsmittel befreit. Man erhielt 42,2 g rohes 5-Chlor1,2,3,4-tetrahydro-1-oxo-2-naphtalinacetaldehyd-dimethylacetal, das ohne Reinigung weiterverwendet wurde.
d) Zur Spaltung des Acetals wurden auf 1000 g Kieselgel 160 ml einer 10%igen wässrigen Oxalsäurelösung aufgezogen und an diesem Gemisch 42,2 g 5-Chlor-1,2,3,4-tetrahydro-1-oxo-2-naphtalinacetaldehyd-dimethylacetal mit Methylenchlorid als Laufmittel chromatographiert. Man erhielt 30,8 g (88% bezogen auf das Produkt aus Beispiel 11.b) 5-Chlor-1,2,3,4-tetrahydro-1-oxo-2-naphtalinacetaldehyd.
e) 15,5 g N-Acetylethylendiamin wurden unter Argon in 300 ml Methylenchlorid gelöst, sukzessive mit 260 g Molekularsieb 4 Å und einer Lösung von 30,8 g 5-Chlor-1,2,3,4-tetrahydro-1-oxo-2-naphtalinacetaldehyd in 150 ml Methylenchlorid versetzt und 23 Stunden unter Rühren refluxiert. Das Reaktionsgemisch wurde abgekühlt, über ein Celite®-Polster filtriert und das Filtrat vom Lösungsmittel befreit. Das Rohprodukt, 37,5 g braunes, amorphes Material, wurde in der 6fachen Menge Essigester heiss gelöst und nach Zugabe der 8fachen Menge Hexan kurz aufgekocht. Man erhielt 29,4 g (74%) N-[2-(6-Chlor-4,5-dihydro-1H-benz[g]indol-1-yl)-ethyl]acetamid; Schmp.: 124-126°C.
f) Unter Argon wurden 6,0 g N-(2-(6-Chlor-4,5-dihydro-1H-benz[g]indol-1-yl)ethyl]acetamid mit 30 ml Phosphoroxychlorid versetzt und das Gemisch für 30 Minuten bei 100°C gerührt. Die abgekühlte Mischung wurde auf 2500 g Eiswasser gegeben und mit 100 ml 2N- und 200 ml 28%iger Natronlauge versetzt. Das Gemisch wurde einmal mit 300 ml und zweimal mit je 100 ml Methylenchlorid extrahiert, die organischen Phasen wurden vereinigt, mit MgSO₄ getrocknet und vom Lösungsmittel befreit. 5,7 g Rückstand wurden in der 20fachen Menge Ethanol heiss gelöst und in der Hitze mit 1,1 Aequivalenten einer gesättigten Lösung von Fumarsäure in Ethanol versetzt. Man erhielt 6,6 g (82%) 4-Chlor-5,6,10,11-tetrahydro-8-methylbenzo[g]pyrazino[1,2-a]indol-fumarat (1:1); Schmp.: 191-192°C (Zers.).

### Beispiel 12

a) 11,0 g 6-Chlor-3,4-dihydro-1(2H)-naphtalenon wurden unter Argon in 150 ml Ethanol suspendiert, mit 18,5 ml N,N-Dimethylhydrazin (asym.) versetzt und während 72 Stunden unter Rückfluss gekocht. Das Reaktionsgemisch wurde im Vakuum vom Lösungsmittel und überschüssigem Dimethylhydrazin befreit. Der Rückstand wurde in 200 ml Methylenchlorid aufgenommen, mit MgSO₄ getrocknet und vom Lösungsmittel befreit. Der Rückstand wurde destilliert und lieferte 12,6 g (93%) 6-Chlor-3,4-dihydro1(2H)-naphtalenon-N',N'-dimethylhydrazon, vom Sdp.: 85-87°C/0,06 mbar.
b) 12,6 g 6-Chlor-3,4-dihydro-1(2H)-naphtalenon-N',N'-dimethylhydrazon wurden unter Argon in 200 ml absolutem Tetrahydrofuran gelöst und mit 10,2 ml N,N,N',N'-Tetramethylethylendiamin versetzt. Die Reaktionslösung wurde auf -70°C abgekühlt und unter Rühren binnen 15 Minuten 36 ml einer 1,6 N Lösung von n-Butyllithium in Hexan zugetropft. Es wurde weitere 15 Minuten bei -70°C gerührt und dann bei -30°C binnen 15 Minuten 8,0 ml Bromacetaldehyd-dimethylacetal zugetropft. Anschliessend wurde 2 Stunden bei -30°C und 18 Stunden bei Raumtemperatur gerührt. Das Reaktionsgemisch wurde mit 400 ml Wasser hydrolysiert, einmal mit 300 ml und zweimal mit je 150 ml Essigester extrahiert. Die vereinigten organischen Extrakte wurden über MgSO₄ getrocknet und vom Lösungsmittel befreit. Das Rohprodukt, 18,8 g braunes Oel, wurde an 400 g Kieselgel mit Essigester/Hexan (1:6) als Laufmittel chromatographiert. Man erhielt 10,8 g (61%) 6-Chlor-1-(dimethylhydrazono)-1,2,3,4-tetrahydro-2-naphtalinacetaldehyd-dimethylacetal.
c) 10,3 g 6-Chlor-1-(dimethylhydrazono)-1,2,3,4-tetrahydro-2-naphtalinacetaldehyd-dimethylacetal wurden unter Argon in einem Gemisch von 500 ml Tetrahydrofuran und 265 ml 0,067 M Phosphatpuffer vom pH-Wert 7 gelöst. Die Lösung wurde mit 8,7 g Kupfer(II)chlorid-dihydrat versetzt und bei Raumtemperatur 18 Stunden gerührt. Zum Reaktionsgemisch wurden 400 ml Essigester gegeben und zweimal mit je 500 ml eines Gemisches aus 25%iger Ammoniaklösung und gesättigter Ammoniumchloridlösung (1:19) gewaschen. Die Waschwasserphasen wurden dreimal mit je 200 ml Essigester extrahiert, die organischen Phasen vereinigt, über MgSO₄ getrocknet und vom Lösungsmittel befreit. Das Rohprodukt, 8,2 g braunes Oel, wurde an 330 g Kieselgel mit Essigester/Hexan-Gemischen (1:6 bis 1:4) als Laufmittel chromatographiert. Man erhielt 2,1 g (24%) 6-Chlor-1,2,3,4-tetrahydro-1-oxo-2-naphtalinacetaldehyd-dimethylacetal neben 3,2 g (43%) 6-Chlor-1,2,3,4-tetrahydro-1-oxo-2-naphtalinacetaldehyd.
d) Zur Spaltung des Acetals wurden auf 50 g Kieselgel 8 ml einer 10%igen wässrigen Oxalsäurelösung aufgezogen und an diesem Gemisch 2,1 g 6-Chlor-1,2,3,4-tetrahydro-1-oxo-2-naphtalinacetaldehyd-dimethylacetal mit Methylenchlorid als Laufmittel chromatographiert. Man erhielt 1,7 g (98%) 6-Chlor-1,2,3,4-tetrahydro-1-oxo-2-naphtalinacetaldehyd.
e) 2,4 g N-Acetylethylendiamin wurden unter Argon in 40 ml Methylenchlorid gelöst, sukzessive mit 40 g Molekularsieb 4 Å und einer Lösung von 4,8 g 6-Chlor-1,2,3,4-tetrahydro-1-oxo-2-naphtalinacetaldehyd in 20 ml Methylenchlorid versetzt und 40 Stunden unter Rühren refluxiert. Das Reaktionsgemisch wurde abgekühlt, über ein Celite®-Polster filtriert und das Filtrat vom Lösungsmittel befreit. Das Rohprodukt, 5,6 g brauner Feststoff, wurde in der 6fachen Menge Essigester heiss gelöst und nach Zugabe der 8fachen Menge Hexan kurz aufgekocht. Man erhielt 4,2g (67%) N-[2-(7-Chlor-4,5-dihydro-1H-benz[g]indol-1-yl)ethyl]acetamid; Schmp.: 144-145°C.
f) Unter Argon wurden 2,8 g N-[2-(7-Chlor-4,5-dihydro-1H-benz[g]indol-1-yl)ethyl]acetamid mit 20 ml Phosphoroxychlorid versetzt und das Gemisch für 30 Minuten bei 100°C gerührt. Die abgekühlte Mischung wurde auf 1500 g Eiswasser gegeben und mit 100 ml 2N- und 150 ml 28%iger Natronlauge versetzt. Das Gemisch wurde einmal mit 300 ml und zweimal mit je 100 ml Methylenchlorid extrahiert, die organischen Phasen wurden vereinigt, mit MgSO₄ getrocknet und vom Lösungsmittel befreit. Der Rückstand (2,6 g) wurde in der 20fachen Menge Ethanol heiss gelöst und in der Hitze mit 1,1 Aequivalenten einer gesättigten Lösung von Fumarsäure in Ethanol versetzt. Man erhielt 3,3 g (88%) 3-Chlor-5,6,10,11-tetrahydro-8-methylbenzo[g]pyrazino[1,2-a]indol-fumarat (1:1); Schmp.: 195-196°C (Zers.).

### Beispiel 13

a) 20,0 g 7-Chlor-3,4-dihydro-1(2H)-naphtalenon wurden unter Argon in 80 ml Ethanol suspendiert, mit 34 ml N,N-Dimethylhydrazin (asym.) versetzt und während 72 Stunden unter Rückfluss gekocht. Das Reaktionsgemisch wurde im Vakuum vom Lösungsmittel und überschüssigem Dimethylhydrazin befreit. Der Rückstand wurde in 130 ml Methylenchlorid aufgenommen, mit MgSO₄ getrocknet und vom Lösungsmittel befreit. Der Rückstand wurde destilliert und lieferte 20,3 g (82%) 7-Chlor-3,4-dihydro-1(2H)-naphtalenon-N',N'-dimethylhydrazon, vom Sdp.: 78-85°C/0,035 mbar.
b) 20,3 g 7-Chlor-3,4-dihydro-1(2H)-naphtalenon-N',N'dimethylhydrazon wurden unter Argon in 400 ml absolutem Tetrahydrofuran gelöst und mit 16,4 ml N,N,N',N'-Tetramethylethylendiamin versetzt. Die Reaktionslösung wurde auf -70°C abgekühlt und unter Rühren binnen 15 Minuten 60 ml einer 1,6 N Lösung von n-Butyllithium in Hexan zugetropft. Es wurde weitere 15 Minuten bei -70°C gerührt und dann bei -30°C binnen 15 Minuten 12,9 ml Bromacetaldehyd-dimethylacetal zugetropft. Anschliessend wurde 2 Stunden bei -30°C und 20 Stunden bei Raumtemperatur gerührt. Das Reaktionsgemisch wurde mit 500 ml Wasser hydrolysiert, einmal mit 400 mi und zweimal mit je 250 ml Essigester extrahiert. Die vereinigten organischen Extrakte wurden über MgSO₄ getrocknet und vom Lösungsmittel befreit. Das Rohprodukt, 29,9 g dunkelbraunes Oel, wurde an 550 g Kieselgel mit Essigester/Hexan-Gemischen (1:6 bis 1:5) als Laufmittel chromatographiert. Man erhielt 16,0 g (57%) 7-Chlor-1-(dimethylhydrazono)-1,2,3,4-tetrahydro-2-naphtalinacetaldehyddimethylacetal.
c) 16,0 g 7-Chlor-1-(dimethylhydrazono)-1,2,3,4-tetrahydro-2-naphtalinacetaldehyd-dimethylacetal wurden unter Argon in 700 ml Tetrahydrofuran und 180 ml Wasser gelöst. Die Lösung wurde sukzessive mit 30,8 g Natrium(meta)periodat, 11,8 g Natriumacetat und 120 mi Essigsäure versetzt und 18 Stunden bei 50°C gerührt. Anschliessend wurden 2000 ml Wasser zugegeben und dreimal mit je 500 ml Essigester extrahiert. Die organischen Phasen wurden mit 500 ml einer gesättigten Kochsalzlösung gewaschen, vereinigt, über MgSO₄ getrocknet und vom Lösungsmittel befreit. Das Rohprodukt, 18,4 g dunkelbraunes Oel, wurde an 500 g Kieselgel mit Essigester/Hexan (1:3) als Laufmittel chromatographiert. Man erhielt 4,8 g (35%) 7-Chlor-1,2,3,4-tetrahydro-1-oxo-2-naphtalinacetaldehyd-dimethylacetal.
d) Zur Acetalspaltung wurden auf 100 g Kieselgel 15 ml einer 10%igen wässrigen Oxalsäurelösung aufgezogen und an diesem Gemisch 4,8 g 7-Chlor-1,2,3,4-tetrahydro-1-oxo-2-naphtalinacetaldehyd-dimethylacetal mit Methylenchlorid als Laufmittel chromatographiert. Man erhielt 3,8 g (95%) 7-Chlor-1,2,3,4-tetrahydro-1-oxo-2-naphtalinacetaldehyd.
e) 2,9 g N-Acetylethylendiamin wurden unter Argon in 50 ml Methylenchlorid gelöst, sukzessive mit 45 g Molekularsieb 4 Å und einer Lösung von 5,7 g 7-Chlor-1,2,3,4-tetrahydro-1-oxo-2-naphtalinacetaldehyd in 20 ml Methylenchlorid versetzt und 16 Stunden unter Rühren zum Rückfluss erhitzt. Das Reaktionsgemisch wurde abgekühlt, über ein Celite®-Polster filtriert und das Filtrat vom Lösungsmittel befreit. Das Rohprodukt, 6,6 g brauner Feststoff, wurde in der 6fachen Menge Essigester heiss gelöst und nach Zugabe der 8fachen Menge Hexan kurz aufgekocht. Man erhielt 4,0 g (55%) N-[2-(8-Chlor-4,5-dihydro-1H-benz[g]indol-1-yl)ethyl]acetamid; Schmp.: 144-146°C.
f) Unter Argon wurden 2,9 g N-[2-(8-Chlor-4,5-dihydro-1H-benz[g]indol-1-yl)ethyl]acetamid mit 20 ml Phosphoroxychlorid versetzt und das Gemisch für 30 Minuten bei 100°C gerührt. Die abgekühlte Mischung wurde auf 1500 g Eiswasser gegossen und mit 100 ml 2N- und 150 ml 28%iger Natronlauge versetzt. Das Gemisch wurde einmal mit 250 ml und zweimal mit je 150 ml Methylenchlorid extrahiert, die organischen Phasen wurden vereinigt, mit MgSO₄ getrocknet und vom Lösungsmittel befreit. Der Rückstand (2,7g) wurde in der 20fachen Menge Ethanol heiss gelöst und in der Hitze mit 1,1 Aequivalenten einer gesättigten Lösung von Fumarsäure in Ethanol versetzt. Man erhielt 3,2 g (83%) 2-Chlor-5,6,10,11-tetrahydro-8-methylbenzo[g]pyrazino[1,2-a]indol-fumarat (1:1); Schmp.: 172-174°C (Zers.).

### Beispiel 14

a) 31,4 g 3,4-Dihydro-4-methyl-1(2H)-naphtalenon-N',N'-dimethylhydrazon wurden unter Argon in 500 ml absolutem Tetrahydrofuran gelöst und mit 28 ml N,N,N',N'-Tetramethylethylendiamin versetzt. Die Reaktionslösung wurde auf -70°C abgekühlt und unter Rühren binnen 30 Minuten 98 ml einer 1,6 N Lösung von n-Butyllithium in Hexan zugetropft. Es wurde weitere 30 Minuten bei -70°C gerührt und dann bei -30°C binnen 10 Minuten 22 ml Bromacetaldehyd-dimethylacetal zugetropft. Anschliessend wurde 2 Stunden bei -30°C und 18 Stunden bei Raumtemperatur gerührt. Das Reaktionsgemisch wurde mit 500 ml Wasser hydrolysiert und dreimal mit je 300 ml Essigester extrahiert. Die vereinigten organischen Extrakte wurden über MgSO₄ getrocknet und vom Lösungsmittel befreit. Das Rohprodukt, 50,3 g braunes Oel, wurde an 1000 g Kieselgel mit Hexan/Diethylether-Gemischen (14:1 bis 1:2) als Laufmittel chromatographiert. Man erhielt 23,1 g (51%) 1-(Dimethylhydrazono)-1,2,3,4-tetrahydro-4-methyl-2-naphtalinacetaldehyd-dimethylacetal.
b) 23,1 g 1-(Dimethylhydrazono)-1,2,3,4-tetrahydro-4-methyl-2-naphtalinacetaldehyd-dimethylacetal wurden unter Argon in einem Gemisch von 1000 ml Tetrahydrofuran und 350 ml 0,067 M Phosphatpuffer vom pH-Wert 7 gelöst. Die Lösung wurde mit 13,6 g Kupfer(II)chlorid-dihydrat versetzt und bei Raumtemperatur 16 Stunden gerührt. Zum Reaktionsgemisch wurden 500 ml Essigester gegeben und dreimal mit einem Gemisch aus 25%iger Ammoniaklösung und gesättigter Ammoniumchloridlösung (1:19) (1x800 ml, 2x500 ml) gewaschen. Die Waschwasserphasen wurden zweimal mit je 500 ml Essigester extrahiert, die organischen Phasen wurden vereinigt, über MgSO₄ getrocknet und vom Lösungsmittel befreit. Man erhielt 20,0 g rohes 1,2,3,4-Tetrahydro-4-methyl-1-oxo-2-naphtalinacetaldehyd-dimethylacetal, die ohne Reinigung weiterverwendet wurden.
c) Zur Acetalspaltung wurden auf 600 g Kieselgel 100 ml einer 10%igen wässrigen Oxalsäurelösung aufgezogen und an diesem Gemisch 20,0 g 1,2,3,4-Tetrahydro-4-methyl-1-oxo-2-naphtalinacetaldehyd-dimethylacetal mit Methylenchlorid als Laufmittel chromatographiert. Man erhielt 14,5 g (90% bezogen auf das Produkt aus Beispiel 14.a) 1,2,3,4-Tetrahydro-4-methyl-1-oxo-2-naphtalinacetaldehyd.
d) 8,1 g N-Acetylethylendiamin wurden unter Argon in 150 ml Methylenchlorid gelöst, sukzessive mit 140 g Molekularsieb 4 Å und einer Lösung von 14,5 g 1,2,3,4-Tetrahydro-4-methyl-1-oxo-2-naphtalinacetaldehyd in 100 ml Methylenchlorid versetzt und 18 Stunden unter Rühren zum Rückfluss erhitzt. Das Reaktionsgemisch wurde abgekühlt, über ein Celite®-Polster filtriert und das Filtrat vom Lösungsmittel befreit. Das Rohprodukt, 19,4 g dunkelbraunes Oel, wurde an 300 g Kieselgel mit Essigester als Laufmittel chromatographiert. Das Produkt, 14,2 g gelbbraunes Oel, wurde in 200 ml Ethanol gelöst, mit 0,7 g Norite® aufgekocht und lieferte nach Filtration und Entfernen des Lösungsmittels 13,9 g (72%) N-[2-(4,5-Dihydro-5-methyl-1H-benz[g]indol-1-yl)ethyl]acetamid, die ohne weitere Reinigung weiterverwendet wurden.
e) Unter Argon wurden 5,0 g N-[2-(4,5-Dihydro-5-methyl-1H-benz[g]indol-1-yl)ethyl]acetamid mit 20 ml Phosphoroxychlorid versetzt und das Gemisch für 30 Minuten bei 100°C gerührt. Die abgekühlte Mischung wurde auf 2000 ml Eiswasser gegeben und mit 200 ml 28%iger Natronlauge versetzt. Das Gemisch wurde einmal mit 300 ml und zweimal mit je 100 ml Methylenchlorid extrahiert, die organischen Phasen wurden vereinigt, mit MgSO₄ getrocknet und vom Lösungsmittel befreit. 4,6 g Rückstand wurden an 200 g Kieselgel mit Methylenchlorid/Methanol-Gemischen (49:1 bis 9:1) als Laufmittel chromatographiert. Vom Rohprodukt, 3,9 g braunem Oel, wurden 1,9 g in der 20fachen Menge Ethanol gelöst und mit 1,1 Aequivalenten einer gesättigten Lösung von Fumarsäure in Ethanol versetzt. Nach Einengen auf 10 ml und Zugabe von 10 ml Ethanol und 70 ml Essigester fielen ockergelbe Kristalle aus, die aus einem Ethanol/Essigester-Gemisch (1:2, 30fache Menge) umkristallisiert wurden. Man erhielt 1,7 g (43%) 5,6,10,11-Tetrahydro-5,8-dimethylbenzo[g]pyrazino[1,2-a]indol-fumarat (2:3); Schmp.: 156-157°C (Zers.).

### Beispiel 15

a) 4,0 g N-[2-(4,5-Dihydro-1H-benz[g]-indol-1-yl)ethyl]acetamid wurden unter Argon in 50 ml Toluol gelöst und mit 3,8 g 2,3-Dichlor-5,6-dicyan-1,4-benzochinon (DDQ) versetzt. Die Reaktionsmischung wurde 2 Stunden bei 80°C gerührt, dann wurden weitere 0,4 g DDQ zugegeben und weitere 2 Stunden bei 80°C gerührt. Der ausgefallene Niederschlag wurde bei Raumtemperatur abfiltriert und das Filtrat vom Lösungsmittel befreit. Das Rohprodukt, 1,5 g dunkelbrauner Feststoff, wurde an 60 g neutralem Aluminiumoxid mit Essigester als Laufmittel chromatographiert. Man erhielt 1,2 g (30%) N-[2-(1H-Benz[g]indol-1-yl)ethyl]acetamid die ohne weitere Reinigung weiterverwendet wurden.
b) Unter Argon wurden 1,4 g N-[2-(1H-Benz[g]indol-1-yl)ethyl]acetamid mit 20 ml Phosphoroxychlorid versetzt und das Gemisch für 30 Minuten bei 100°C gerührt. Die abgekühlte Mischung wurde auf 500 g Eiswasser gegossen und mit 150 ml 2N- und 50 ml 28%iger Natronlauge versetzt. Das Gemisch wurde einmal mit 200 ml und zweimal mit je 100 ml Methylenchlorid extrahiert, die organischen Phasen wurden vereinigt, mit MgSO₄ getrocknet und vom Lösungsmittel befreit. 1,4 g Rückstand wurden in der 20fachen Menge Ethanol gelöst und mit 1,1 Aequivalenten einer heissen gesättigten Lösung von Fumarsäure in Ethanol versetzt. Man erhielt 1,6 g (83%) 10,11-Dihydro-8-methylbenzo[g]pyrazino[1,2-a]indol-fumarat (1:1); Schmp.: 189-190°C (Zers.).

### Beispiel 16

a) Zu einer Lösung von 1,0 g Kaliumhydroxid in 10 ml Wasser wurden sukzessive 0,9 g N-[2-(2-Phenylpyrrol-1-yl)ethyl]acetamid und 5 ml Ethylenglycol gegeben. Das Reaktionsgemisch wurde 24 Stunden in der Siedehitze gerührt. Das kalte Gemisch wurde zweimal mit je 50 ml Methylenchlorid extrahiert, die organischen Phasen wurden vereinigt und zweimal mit je 50 ml 2N Salzsäure extrahiert. Die wässrigen Phasen wurden vereinigt, durch Zugabe von 28%iger Natronlauge auf einen pH-Wert von 12 gebracht und erneut zweimal mit je 50 ml Methylenchlorid extrahiert. Die organischen Phasen wurden vereinigt, mit MgSO₄ getrocknet und vom Lösungsmittel befreit. 0,3 g von insgesamt 0,6 g Rohprodukt wurden in der 20fachen Menge Ethanol gelöst und mit 2 Aequivalenten einer gesättigten Lösung von Fumarsäure in Ethanol versetzt. Man erhielt 0,3 g (64%) 1-(2-Aminoethyl)-2-phenylpyrrol-fumarat (1:1); Schmp.: 169-171°C.
b) 0,3 g rohes 1-(2-Aminoethyl)-2-phenylpyrrol wurden unter Argon in 5 ml Essigsäure gelöst und mit 0,1 ml einer 36,5%igen wässrigen Formaldehydlösung versetzt. Das Reaktionsgemisch wurde 64 Stunden bei Raumtemperatur gerührt und anschliessend im Vakuum eingeengt. Der Rückstand wurde kurz mit 50 ml Methylenchlorid und 20 ml 10%iger Ammoniaklösung gerührt. Die Phasen wurden getrennt, die organische Phase wurde mit MgSO₄ getrocknet und vom Lösungsmittel befreit. Der Rückstand, 0,4 g gelbes Oel, wurde an 70 g Kieselgel mit Methylenchlorid/Methanol (49:1) als Laufmittel chromatographiert. Das Produkt, 0,3 g gelbes Oel, wurde in der 20fachen Menge Ethanol aufgenommen und mit 2 Aequivalenten einer gesättigten Lösung von Fumarsäure in Ethanol versetzt. Die ausgefallenen Kristalle wurden aus Ethanol umkristallisiert. Man erhielt 0,2 g (40%) 1,2,3,4-Tetrahydro-6-phenylpyrrolo[1,2-a]pyrazin-fumarat (1:1); Schmp.: 182-184°C (Zers.).

### Beispiel 17

a) Unter Argon wurden 5,0 g N-[2-(4,5-Dihydro-1H-benz[g]indol-1-yl)ethyl]acetamid in 60 ml Ethylenglycol und 25 ml Wasser suspendiert und mit 5,0 g pulverisiertem Kaliumhydroxid versetzt. Das Gemisch wurde 18 Stunden bei 120°C gerührt, anschliessend abgekühlt und mit 150 ml 2N Salzsäure unter Kühlung versetzt. Die Lösung wurde mit 200 ml Methylenchlorid gewaschen, die organische Phase wurde dreimal mit je 100 ml 2N Salzsäure extrahiert, die wässrigen Phasen wurden vereinigt und unter Eiskühlung mit 28%iger Natronlauge stark alkalisch gestellt. Die wässrige Lösung wurde einmal mit 200 ml und zweimal mit je 100 ml Methylenchlorid extrahiert, die organischen Phasen wurden vereinigt, mit MgSO₄ getrocknet und vom Lösungsmittel befreit. Der Rückstand, 4,0 g braunes Oel, wurde an 250 g Kieselgel mit Methylenchlorid/Methanol-Gemischen (99:1 bis 19:1) als Laufmittel chromatographiert. Man erhielt 3,7 g (88%) 1-(2-Aminoethyl)-4,5-dihydro-1H-benz[g]indol, die ohne weitere Reinigung weiterverwendet wurden.
b) Unter Argon wurden zu einer Lösung von 3,7 g 1-(2-Aminoethyl)-4,5-dihydro-1H-benz[g]indol in 70 ml Ethanol 1,4 ml einer 36%igen wässrigen Formaldehydlösung gegeben. Die Reaktionslösung wurde 2 Stunden unter Rückfluss gerührt, anschliessend abgekühlt und vom Lösungsmittel befreit. Der Rückstand wurde an 250 g Kieselgel mit Methylenchlorid/Methanol (49:1) als Laufmittel chromatographiert. 1,3 g von insgesamt 2,8 g Produkt wurden in der 20fachen Menge Ethanol heiss gelöst und in der Hitze mit 1,1 Aequivalenten einer gesättigten Lösung von Fumarsäure in Ethanol versetzt. Der ausgefallene Feststoff wurde in der 150fachen Menge Methanol aufgekocht, heiss filtriert und auf die 60fache Menge an Lösungsmittel eingeengt. Man erhielt 1,2 g (53%) 5,6,8,9,10,11-Hexahydrobenzo[g]pyrazino[1,2-a]indol-fumarat (2:1); Schmp.: 183-184°C (Zers.).

### Beispiel 18

a) 4,3 g 3-Phenylfuran wurden unter Argon in 80 ml Acetonitril gelöst und unter Rühren mit 8,6 g des gemischten Anhydrids aus Essig- und p-Toluolsulfonsäure versetzt. Die Reaktionslösung wurde 64 Stunden bei Raumtemperatur gerührt und anschliessend mit 150 mi gesättigter Natriumhydrogencarbonatlösung und 150 ml Diethylether für 30 Minuten gerührt. Die Phasen wurden getrennt und die wässrige einmal mit weiteren 100 ml Diethylether extrahiert. Die organischen Phasen wurden vereinigt, mit 100 ml Wasser gewaschen, über MgSO₄ getrocknet und vom Lösungsmittel befreit. Der Rückstand wurde an 200 g Kieselgel mit Methylenchlorid/Hexan (1:1) als Laufmittel chromatographiert. Man erhielt 0,7 g (13%) 2-Acetyl-4-phenylfuran neben 2,4 g (43%) 2-Acetyl-3-phenylfuran. Ersteres wurde ohne weitere Reinigung weiterverwendet.
b) 0,7 g 2-Acetyl-4-phenylfuran wurden unter Argon mit 0,8 ml 1,2-Ethandiamin und 0,1 ml Wasser für 1 Stunde auf 100°C erhitzt. Nach Abkühlen wurden 50 ml Wasser zugegeben und das Gemisch dreimal mit je 50 ml Methylenchlorid extrahiert. Die organischen Phasen wurden vereinigt, mit MgSO₄ getrocknet und vom Lösungsmittel befreit. Der Rückstand, 0,7 g braunes Oel, wurde an 60 g Kieselgel mit Methylenchlorid/Methanol (19:1) als Laufmittel chromatographiert. Das Rohprodukt, 0,4 g brauner Feststoff, wurde in der 50fachen Menge Ethanol heiss gelöst und mit 1,1 Aequivalenten einer gesättigten Lösung von Fumarsäure in Ethanol versetzt. Man erhielt 0,4 g (31%) 3,4-Dihydro-1-methyl-7-phenylpyrrolo[1,2-a]pyrazin-fumarat (1:1); Schmp.: 181°C (Zers.).

### Beispiel 19

1,1 g 3,4-Dihydro-1-methyl-6-phenylpyrrolo[1,2-a]pyrazin wurden unter Argon in einem Gemisch aus 50 ml Methanol und 5 ml Wasser gelöst. Die Lösung wurde unter Rühren portionenweise mit 0,9 g Natriumborhydrid versetzt und über Nacht bei Raumtemperatur gerührt. Danach wurde das Methanol im Vakuum entfernt, der Rückstand in 100 ml Methylenchlorid aufgenommen und mit 100 ml 10%iger Ammoniaklösung gewaschen. Die Phasen wurden getrennt und die wässrige Phase wurde zweimal mit je 50 ml Methylenchlorid extrahiert. Die organischen Phasen wurden vereinigt, mit MgSO₄ getrocknet und vom Lösungsmittel befreit. Das Rohprodukt, 1,1 g gelbes Oel, wurde in der 10fachen Menge Ethanol gelöst und mit 1,1 Aequivalenten einer gesättigten Lösung von Fumarsäure in Ethanol kurz aufgekocht. Die ausgefallenen farblosen Kristalle wurden aus der 30fachen Menge Methanol umkristallisiert. Man erhielt 0,5 g (37%) 1,2,3,4-Tetrahydro-1-methyl-6-phenylpyrrolo[1,2-a]pyrazin-fumarat (1:1); Schmp.: 153-154°C (Zers.).

### Beispiel 20

2,0 g 6-(m-Chlorphenyl)-3,4-dihydro-1-methylpyrrolo[1,2-a]pyrazin wurden unter Argon in einem Gemisch aus 100 ml Methanol und 10 ml Wasser gelöst. Die Lösung wurde unter Rühren portionenweise mit 0,9 g Natriumborhydrid versetzt und über Nacht bei Raumtemperatur gerührt. Danach wurde das Methanol im Vakuum entfernt, der Rückstand in 150 ml Methylenchlorid aufgenommen und mit 50 ml 10%iger Ammoniaklösung gewaschen. Die Phasen wurden getrennt und die wässrige Phase wurde zweimal mit je 70 ml Methylenchlorid extrahiert. Die organischen Phasen wurden vereinigt, mit MgSO₄ getrocknet und vom Lösungsmittel befreit. Das Rohprodukt, 2,0 g gelbes Oel, wurde in der 20fachen Menge Ethanol gelöst und mit 1,1 Aequivalenten einer gesättigten Lösung von Fumarsäure in Ethanol versetzt. Die Lösung wurde auf 10 ml eingeengt und mit 100 ml Essigester kurz aufgekocht. Man erhielt 1,7 g (57%) 6-(m-Chlorphenyl)-1,2,3,4-tetrahydro-1-methylpyrrolo[1,2-a]pyrazin-fumarat (1:1); Schmp.: 165-167°C (Zers.).

### Beispiel 21

1,0 g 6-(p-Chlorphenyl)-3,4-dihydro-1-methylpyrrolo[1,2-a]pyrazin wurden unter Argon in einem Gemisch aus 100 ml Methanol und 10 ml Wasser gelöst. Die Lösung wurde unter Rühren portionenweise mit 0,5 g Natriumborhydrid versetzt und über Nacht bei Raumtemperatur gerührt. Danach wurde das Methanol im Vakuum entfernt, der Rückstand in 100 ml Methylenchlorid aufgenommen und mit 50 ml 10%iger Ammoniaklösung gewaschen. Die Phasen wurden getrennt und die wässrige Phase wurde zweimal mit je 70 ml Methylenchlorid extrahiert. Die organischen Phasen wurden vereinigt, mit MgSO₄ getrocknet und vom Lösungsmittel befreit. Das Rohprodukt, 1,1 g braunes Oel, wurde in der 30fachen Menge Ethanol unter Zusatz der 5fachen Menge Methylenchlorid gelöst und mit 1,1 Aequivalenten einer gesättigten Lösung von Fumarsäure in Ethanol versetzt. Man erhielt 1,1 g (74%) 6-(p-Chlorphenyl)-1,2,3,4-tetrahydro-1-methylpyrrolo[1,2-a]pyrazin-fumarat (1:1); Schmp.: 196-198°C (Zers.).

### Beispiel 22

1,0 g 3,4-Dihydro-6-(p-methoxyphenyl)-1-methylpyrrolo[1,2-a]pyrazin wurden unter Argon in einem Gemisch aus 60 ml Methanol und 6 ml Wasser gelöst. Die Lösung wurde unter Rühren portionenweise mit 0,5 g Natriumborhydrid versetzt und über Nacht bei Raumtemperatur gerührt. Danach wurde das Methanol im Vakuum entfernt, der Rückstand in 100 ml Methylenchlorid aufgenommen und mit 45 ml 10%iger Ammoniaklösung gewaschen. Die Phasen wurden getrennt und die wässrige Phase wurde zweimal mit je 50 ml Methylenchlorid extrahiert. Die organischen Phasen wurden vereinigt, mit MgSO₄ getrocknet und vom Lösungsmittel befreit. Das Rohprodukt, 1,0 g brauner Feststoff, wurde in der 30fachen Menge Ethanol gelöst und mit 1,1 Aequivalenten einer gesättigten Lösung von Fumarsäure in Ethanol versetzt. Man erhielt 0,7 g (47%) 1,2,3,4-Tetrahydro-6-(p-methoxyphenyl)-1-methylpyrrolo[1,2-a]pyrazin-fumarat (1:0,9); Schmp.: 162-165°C (Zers.).

### Beispiel 23

2,5 g 3,4-Dihydro-1-methyl-6-[4-(trifluormethyl)phenyl]pyrrolo[1,2-a]pyrazin wurden unter Argon in einem Gemisch aus 130 ml Methanol und 13 ml Wasser gelöst. Die Lösung wurde unter Rühren portionenweise mit 1,0 g Natriumborhydrid versetzt und über Nacht bei Raumtemperatur gerührt. Danach wurde das Methanol im Vakuum entfernt, der Rückstand in 100 ml Methylenchlorid aufgenommen und mit 80 ml 10%iger Ammoniaklösung gewaschen. Die Phasen wurden getrennt und die wässrige Phase wurde zweimal mit je 50 ml Methylenchlorid extrahiert. Die organischen Phasen wurden vereinigt, mit MgSO₄ getrocknet und vom Lösungsmittel befreit. Das Rohprodukt, 2,5 g gelbbrauner Feststoff, wurde in der 20fachen Menge Ethanol gelöst, mit 1,1 Aequivalenten einer gesättigten Lösung von Fumarsäure in Ethanol versetzt und auf 40 ml eingeengt. Man erhielt 2,5 g (70%) 1,2,3,4-Tetrahydro-1-methyl-6-[4-(trifluormethyl)phenyl]pyrrolo[1,2-a]pyrazin-fumarat (1:1); Schmp.: 180-183°C (Zers.).

### Beispiel 24

1,8 g 6-(3,4-Dichlorphenyl)-3,4-dihydro-1-methylpyrrolo[1,2-a]pyrazin wurden unter Argon in einem Gemisch aus 100 ml Methanol und 10 ml Wasser gelöst. Die Lösung wurde unter Rühren portionenweise mit 0,7 g Natriumborhydrid versetzt und über Nacht bei Raumtemperatur gerührt. Danach wurde das Methanol im Vakuum entfernt, der Rückstand in 150 ml Methylenchlorid aufgenommen und mit 65 ml 10%iger Ammoniaklösung gewaschen.

Die Phasen wurden getrennt und die wässrige Phase wurde zweimal mit je 70 ml Methylenchlorid extrahiert. Die organischen Phasen wurden vereinigt, mit MgSO₄ getrocknet und vom Lösungsmittel befreit. Das Rohprodukt, 1,8 g braunes Oel, wurde in der 20fachen Menge Ethanol gelöst und mit 1,1 Aequivalenten einer gesättigten Lösung von Fumarsäure in Ethanol versetzt. Man erhielt 2,0 g (92%) 6-(3,4-Dichlorphenyl)-1,2,3,4-tetrahydro-1-methylpyrrolo[1,2-a]pyrazin-fumarat (2:1); Schmp.: 224-226°C (Zers.).

### Beispiel 25

1,8 g 3,4-Dihydro-1-methyl-6-p-tolylpyrrolo[1,2-a]pyrazin wurden unter Argon in einem Gemisch aus 100 ml Methanol und 10 ml Wasser gelöst. Die Lösung wurde unter Rühren portionenweise mit 0,9 g Natriumborhydrid versetzt und über Nacht bei Raumtemperatur gerührt. Danach wurde das Methanol im Vakuum entfernt, der Rückstand in 150 ml Methylenchlorid aufgenommen und mit 100 ml 10%iger Ammoniaklösung gewaschen. Die Phasen wurden getrennt und die wässrige Phase wurde zweimal mit je 70 ml Methylenchlorid extrahiert. Die organischen Phasen wurden vereinigt, mit MgSO₄ getrocknet und vom Lösungsmittel befreit. Das Rohprodukt, 1,7 g braunes Oel, wurde in der 25fachen Menge Ethanol heiss gelöst und mit 1,1 Aequivalenten einer heissen, gesättigten Lösung von Fumarsäure in Ethanol versetzt. Man erhielt 2,1 g (79%) 1,2,3,4-Tetrahydro-1-methyl-6-p-tolylpyrrolo[1,2-a]pyrazin-fumarat (1:1); Schmp.: 196-198°C (Zers.).

### Beispiel 26

1,3 g 6-(3,4-Dimethoxyphenyl)-3,4-dihydro-1-methylpyrrolo[1,2-a]pyrazin wurden unter Argon in einem Gemisch aus 80 ml Methanol und 8 ml Wasser gelöst. Die Lösung wurde unter Rühren portionenweise mit 0,6 g Natriumborhydrid versetzt und über Nacht bei Raumtemperatur gerührt. Danach wurde das Methanol im Vakuum entfernt, der Rückstand in 100 ml Methylenchlorid aufgenommen und mit 100 ml 10%iger Ammoniaklösung gewaschen. Die Phasen wurden getrennt und die wässrige Phase wurde zweimal mit je 50 ml Methylenchlorid extrahiert. Die organischen Phasen wurden vereinigt, mit MgSO₄ getrocknet und vom Lösungsmittel befreit. Das Rohprodukt, 1,3 g gelbbraunes Oel, wurde an 60 g Kieselgel mit Methylenchlorid/Methanol (49:1) als Laufmittel chromatographiert. Das Produkt, 0,9 g gelbes Oel, wurde in der 20fachen Menge Ethanol gelöst, mit 1,1 Aequivalenten einer gesättigten Lösung von Fumarsäure in Ethanol versetzt, auf 10 ml eingeengt und mit 40 ml Essigester versetzt. Man erhielt 0,5 g (37%) 6-(3,4-Dimethoxyphenyl)-1,2,3,4-tetrahydro-1-methylpyrrolo[1,2-a]pyrazin-fumarat (1:0,6); Schmp.: 172-175°C (Zers.). Eine Probe wurde aus der 40fachen Menge Ethanol umkristallisiert und lieferte das (2:1)-Fumarat; Schmp.: 178-180°C (Zers.).

### Beispiel 27

2,8 g rohes 6-[p-(Benzyloxy)phenyl]-3,4-dihydro-1-methylpyrrolo[1,2-a]pyrazin wurden unter Argon in einem Gemisch aus 150 ml Methanol, 15 ml Wasser und 25 ml Methylenchlorid gelöst. Die Lösung wurde unter Rühren portionenweise mit 1,0 g Natriumborhydrid versetzt und über Nacht bei Raumtemperatur gerührt. Danach wurde das Lösungsmittel im Vakuum entfernt, der Rückstand in 150 ml Methylenchlorid aufgenommen und mit 100 ml 10%iger Ammoniaklösung 1/2 Stunde bei Raumtemperatur gerührt. Die Phasen wurden getrennt und die wässrige Phase wurde zweimal mit je 70 ml Methylenchlorid extrahiert. Die organischen Phasen wurden vereinigt, mit MgSO₄ getrocknet und vom Lösungsmittel befreit. Das Rohprodukt, 2,7 g braunes Oel, wurde an 60 g Kieselgel mit Methylenchlorid/Methanol (49:1) als Laufmittel chromatographiert. Das Produkt, 1,0 g beigefarbener Feststoff, wurde in der 35fachen Menge Ethanol und 5fachen Menge Methylenchlorid heiss gelöst und mit 1,1 Aequivalenten einer gesättigten Lösung von Fumarsäure in Ethanol versetzt. Man erhielt 0,8 g (24%) 6-[p-(Benzyloxy)phenyl]-1,2,3,4-tetrahydro-1-methylpyrrolo[1,2-a]pyrazin fumarat (2:1); Schmp.: 172-174°C (Zers.).

### Beispiel 28

1,5 g 6-[p-(Cyclopentyloxy)phenyl]-3,4-dihydro-1-methylpyrrolo[1,2-a]pyrazin wurden unter Argon in einem Gemisch aus 100 ml Methanol, 10 ml Wasser und 25 ml Methylenchlorid gelöst. Die Lösung wurde unter Rühren portionenweise mit 0,6 g Natriumborhydrid versetzt und über Nacht bei Raumtemperatur gerührt. Danach wurde das Lösungsmittel im Vakuum entfernt, der Rückstand in 100 ml Methylenchlorid aufgenommen und mit 100 ml 10%iger Ammoniaklösung gewaschen. Die Phasen wurden getrennt und die wässrige Phase wurde zweimal mitje 60 ml Methylenchlorid extrahiert. Die organischen Phasen wurden vereinigt, mit MgSO₄ getrocknet und vom Lösungsmittel befreit. Das Rohprodukt 1,6 g braunes Oel, wurde in der 20fachen Menge Ethanol heiss gelöst und in der Hitze mit 1,1 Aequivalenten einer gesättigten Lösung von Fumarsäure in Ethanol versetzt. Man erhielt 1,2 g (66%) 6-[p-(Cyclopentyloxy)phenyl]-1,2,3,4-tetrahydro-1-methylpyrrolo[1,2-a]pyrazin-fumarat (2:1); Schmp.: 172-174°C (Zers.).

### Beispiel 29

0,6 g 5,6,10,11-Tetrahydro-8-methylbenzo[g]pyrazino[1,2-a]indol wurden unter Argon in einem Gemisch aus 25 ml Ethanol und 2,5 ml Wasser gelöst. Die Lösung wurde unter Rühren portionenweise mit 0,3 g Natriumborhydrid versetzt und über Nacht bei Raumtemperatur gerührt. Danach wurde das Lösungsmittel im Vakuum entfernt, der Rückstand in 50 ml Methylenchlorid aufgenommen und mit 50 ml 10%iger Ammoniaklösung gewaschen. Die Phasen wurden getrennt und die wässrige Phase wurde zweimal mit je 20 ml Methylenchlorid extrahiert. Die organischen Phasen wurden vereinigt, mit MgSO₄ getrocknet und vom Lösungsmittel befreit. Das Rohprodukt, 0,5 g brauner Feststoff, wurde in der 20fachen Menge Ethanol gelöst und mit 1,1 Aequivalenten einer gesättigten Lösung von Fumarsäure in Ethanol versetzt. Man erhielt 0,2 g (24%) 5,6,8,9,10,11-Hexahydro-8-methylbenzo[g]pyrazino[1,2-a]indol-fumarat (1:1); Schmp.: 181-184°C (Zers.).

### Beispiel 30

2,1 g 4-Chlor-5,6,10,11-tetrahydro-8-methylbenzo[g]pyrazino[1,2-a]indol wurden unter Argon in einem Gemisch aus 70 ml Methanol und 7 ml Wasser gelöst. Die Lösung wurde unter Rühren portionenweise mit 0,9 g Natriumborhydrid versetzt und über Nacht bei Raumtemperatur gerührt. Danach wurde das Lösungsmittel im Vakuum entfernt, der Rückstand in 70 ml Methylenchlorid aufgenommen und mit 100 ml 10%iger Ammoniaklösung gewaschen. Die Phasen wurden getrennt und die wässrige Phase wurde zweimal mit je 40 ml Methylenchlorid extrahiert. Die organischen Phasen wurden vereinigt, mit MgSO₄ getrocknet und vom Lösungsmittel befreit. Das Rohprodukt, 1,9 g gelbes, amorphes Material, wurde in der 20fachen Menge Ethanol heiss gelöst und in der Hitze mit 1,1 Aequivalenten einer gesättigten Lösung von Fumarsäure in Ethanol versetzt. Man erhielt 2,3 g (78%) 4-Chlor-5,6,8,9,10,11-hexahydro-8-methylbenzo[g]pyrazino[1,2-a]indol-fumarat (1:1); Schmp.: 199°C (Zers.).

### Beispiel 31

0,9 g 3-Chlor-5,6,10,11-tetrahydro-8-methylbenzo[g]pyrazino[1,2-a]indol wurden unter Argon in einem Gemisch aus 40 ml Methanol und 4 ml Wasser gelöst. Die Lösung wurde unter Rühren portionenweise mit 0,4 g Natriumborhydrid versetzt und über Nacht bei Raumtemperatur gerührt. Danach wurde das Lösungsmittel im Vakuum entfernt, der Rückstand in 50 ml Methylenchlorid aufgenommen und mit 50 ml 10%iger Ammoniaklösung gewaschen. Die Phasen wurden getrennt und die wässrige Phase wurde zweimal mit je 30 ml Methylenchlorid extrahiert. Die organischen Phasen wurden vereinigt, mit MgSO₄ getrocknet und vom Lösungsmittel befreit. Das Rohprodukt, 0,9 g oranger Feststoff, wurde in der 40fachen Menge Ethanol heiss gelöst und in der Hitze mit 1,1 Aequivalenten einer gesättigten Lösung von Fumarsäure in Ethanol versetzt. Man erhielt 1,0 g (78%) 3-Chlor-5,6,8,9,10,11-hexahydro-8-methylbenzo[g]pyrazino[1,2-a]indol-fumarat (1:1); Schmp.: 202-203°C (Zers.).

### Beispiel 32

1,1 g 2-Chlor-5,6,10,11-tetrahydro-8-methylbenzo[g]pyrazino[1,2-a]indol wurden unter Argon in einem Gemisch aus 50 ml Methanol und 5 ml Wasser gelöst. Die Lösung wurde unter Rühren portionenweise mit 0,5 g Natriumborhydrid versetzt und über Nacht bei Raumtemperatur gerührt. Danach wurde das Lösungsmittel im Vakuum entfernt, der Rückstand in 50 ml Methylenchlorid aufgenommen und mit 50 ml 10%iger Ammoniaklösung gewaschen. Die Phasen wurden getrennt und die wässrige Phase wurde zweimal mit je 30 ml Methylenchlorid extrahiert. Die organischen Phasen wurden vereinigt, mit MgSO₄ getrocknet und vom Lösungsmittel befreit. Das Rohprodukt` 1,1 g gelber, amorpher Feststoff, wurde in der 20fachen Menge Ethanol heiss gelöst und in der Hitze mit 1,1 Aequivalenten einer gesättigten Lösung von Fumarsäure in Ethanol versetzt. Die ausgefallenen Kristalle wurden aus der 50fachen Menge Ethanol umkristallisiert. Man erhielt 1,0 g (74%) 2-Chlor-5,6,8,9,10,11-hexahydro-8-methylbenzo[g]pyrazino[1,2-a]indol-fumarat (2:1); Schmp.: 198-200°C (Zers.).

### Beispiel 33

2,0 g 5,6,10,11-Tetrahydro-5,8-dimethylbenzo[g]pyrazino[1,2-a]indol wurden unter Argon in einem Gemisch aus 80 ml Methanol und 8 ml Wasser gelöst. Die Lösung wurde unter Rühren portionenweise mit 0,9 g Natriumborhydrid versetzt und über Nacht bei Raumtemperatur gerührt. Danach wurde das Lösungsmittel im Vakuum entfernt, der Rückstand in 100 ml Methylenchlorid aufgenommen und mit 100 ml 10%iger Ammoniaklösung gewaschen. Die Phasen wurden getrennt und die wässrige Phase wurde zweimal mit je 50 ml Methylenchlorid extrahiert. Die organischen Phasen wurden vereinigt, mit MgSO₄ getrocknet und vom Lösungsmittel befreit. Das Rohprodukt, 2,0 g gelbbraunes Oel, wurde in der 25fachen Menge Ethanol heiss gelöst und mit 1,1 Aequivalenten einer heissen gesättigten Lösung von Fumarsäure in Ethanol versetzt. Man erhielt 2,4 g (81%) 5,6,8,9,10,11-Hexahydro-5,8-dimethylbenzo[g]pyrazino[1,2-a]indol-fumarat (1:1); Schmp.: 196-199°C (Zers.).

### Beispiel 34

a) 0,6 g 3,4-Dihydro-1-methyl-6-phenylpyrrolo[1,2-a]pyrazin wurden in 10 ml Ethanol gelöst und unter Argon mit 0,9 ml Methyliodid versetzt. Die Lösung wurde über Nacht gekocht, abgekühlt und die ausgefallenen Kristalle wurden abfiltriert. Man erhielt 0,8 g (78%) 3,4-Dihydro-1,2-dimethyl-6-phenylpyrrolo[1,2-a]pyraziniumiodid; Schmp.: 213-216°C (Zers.).
b) 0,4 g 3,4-Dihydro-1,2-dimethyl-phenylpyrrolo[1,2-a]pyraziniumiodid wurden unter Argon in 20 ml Methanol und 0,5 ml Wasser gelöst und nach Zusatz von 0,2 g Natriumborhydrid 15 Minuten bei Raumtemperatur gerührt. Das Methanol wurde im Vakuum entfernt und der Rückstand kurz mit 50 ml Methylenchlorid und 20 ml 10%iger Ammoniaklösung gerührt. Die wässrige Phase wurde zweimal mit je 20 ml Methylenchlorid extrahiert, die organischen Phasen wurden vereinigt, mit MgSO₄ getrocknet und vom Lösungsmittel befreit. Das Rohprodukt, 0,2 g gelbes Oel, wurde in der 25fachen Menge Ethanol aufgenommen, mit 2 Aequivalenten einer gesättigten Lösung von Fumarsäure in Ethanol versetzt, auf 2 ml eingeengt und mit 5 ml Essigester aufgekocht. Man erhielt 0,2 g (51%) 1,2,3,4-Tetrahydro-1,2-dimethyl-6-phenylpyrrolo[1,2-a]pyrazin-fumarat (1:1); Schmp.: 127-128°C (Zers.).

### Beispiel 35

In einer Argon-Atmosphäre wurden 20,9 g 3,4-Dihydr-6-(p-methoxyphenyl)-1-methylpyrrolo[1,2-a]pyrazin mit 500 ml 48%iger Bromwasserstoffsäure unter Rühren für 1 1/2 Stunden gekocht. Die Bromwasserstoffsäure wurde im Vakuum entfernt und der Rückstand in 900 ml Essigester unter Zugabe von 100 ml Methanol gelöst. Die Lösung wurde mit einem Gemisch aus 500 ml gesättigter Natriumhydrogencarbonatlösung und 100 ml 2N Natronlauge 15 Minuten gerührt. Die Wasserphase wurde mit 2N Salzsäure auf einen pH-Wert von 8-9 gebracht und viermal mit je 500 ml eines Essigester/Methanol-Gemisches (9:1) extrahiert. Die Wasserphase wurde mit 2N Salzsäure neutralisiert (pH = 7) und über Nacht mit 1000 ml Essigester kontinuierlich extrahiert. Die organischen Phasen wurden filtriert, vereinigt und auf 150 ml eingeengt. Dann wurden 150 ml Essigester zugegeben, die Suspension aufgekocht und über Nacht im Kühlschrank aufbewahrt. 16,3 g Rohprodukt wurden abfiltriert. 1,6 g davon wurden in der 50fachen Menge Methanol und 10fachen Menge Methylenchlorid gelöst und mit 1,1 Aequivalenten Fumarsäure in 20 ml Methanol versetzt. Man erhielt 1,8 g (62%) 3,4-Dihydro-6-(4-hydroxyphenyl)-1-methylpyrrolo[1,2-a]pyrazin-fumarat (1:1); Schmp.: 209-213°C (Zers.).

### Beispiel 36

Zu einer Lösung von 1,8 g Natriumhydroxid in 100 ml Wasser wurden unter Argon sukzessive 5,0 g 3,4-Dihydro-6-(4-hydroxyphenyl)-1-methylpyrrolo[1,2-a]pyrazin, 100 ml Methylenchlorid, 5,3 ml Benzylbromid und 0,8 g Benzyltributylammoniumbromid gegeben. Das Gemisch wurde über Nacht unter Rückfluss gekocht und gerührt und anschliessend abgekühlt. Nach Zugabe von 100 ml Methylenchlorid und 100 ml Wasser wurden die Phasen getrennt und die wässrige wurde zweimal mit je 100 ml Methylenchlorid extrahiert. Die organischen Phasen wurden vereinigt, mit MgSO₄ getrocknet und vom Lösungsmittel befreit. Der Rückstand, 10,3 g braunes Oel, wurde an 500 g Kieselgel mit Methylenchlorid/Methanol-Gemischen (99:1 bis 9:1) als Laufmittel chromatographiert. Das Rohprodukt, 4,9 g braunes, amorphes Material, wurde in der 10fachen Menge Ethanol heiss gelöst und mit 1,1 Aequivalenten einer gesättigten Lösung von Fumarsäure in Ethanol versetzt. Das Produkt, 2,2 g gelbe Kristalle, wurde aus der 20fachen Menge Ethanol umkristallisiert. Man erhielt 1,8 g (19%) 6-[p-(Benzyloxy)phenyl]-3,4-dihydro-1-methylpyrrolo[1,2-a]pyrazin-fumarat (1:1); Schmp.: 165-168°C (Zers.).

### Beispiel 37

Zu einer Lösung von 4,3 g 3,4-Dihydro-6-(4-hydroxyphenyl)-1-methylpyrrolo[1,2-a]pyrazin in 80 ml Dimethylformamid wurden unter Rühren und Argon sukzessive 5,3 g Kaliumcarbonat und 4,1 ml Bromcyclopentan gegeben. Das Gemisch wurde 65 Stunden bei 80°C, dann 24 Stunden bei 100°C gerührt und anschliessend abgekühlt. Nach Zugabe von 600 ml Wasser wurde dreimal mit je 250 ml Essigester extrahiert. Die organischen Phasen wurden mit 300 ml eines Gemisches von Wasser und gesättigter Kochsalzlösung (1:1) gewaschen, vereinigt, über MgSO₄ getrocknet und vom Lösungsmittel befreit. Der Rückstand, 5,3 g braunes Oel, wurde an 150 g Kieselgel mit Methylenchlorid/Methanol-Gemischen (49:1 bis 24:1) als Laufmittel chromatographiert. 1,5 g von insgesamt 3,0 g Rohprodukt wurden in der 30fachen Menge Ethanol heiss gelöst und mit 1,1 Aequivalenten einer heissen gesättigten Lösung von Fumarsäure in Ethanol versetzt. Nach Zugabe von 30 ml Essigester kristallisierten 1,4 g (36%) 6-[p-(Cyclopentyloxy)phenyl]-3,4-dihydro-1-methylpyrrolo[1,2-a]pyrazin-fumarat (1:1); Schmp.: 155-157°C (Zers.).

### Beispiel 38

a) 45,7 g 1,2-Diphenylethanon-(E/Z)-dimethylhydrazon wurden unter Argon in 500 ml absolutem Tetrahydrofuran gelöst und mit 34,5 ml N,N,N',N'-Tetramethylethylendiamin versetzt. Die Reaktionslösung wurde auf -70°C abgekühlt und unter Rühren binnen 30 Minuten 125 ml einer 1,6 N Lösung von n-Butyllithium in Hexan zugetropft. Es wurde weitere 30 Minuten bei -70°C gerührt und dann bei -30°C binnen 15 Minuten 27,1 ml Bromacetaldehyd-dimethylacetal zugetropft. Anschliessend wurde 2 Stunden bei -30°C und 16 Stunden bei Raumtemperatur gerührt. Das Reaktionsgemisch wurde mit 500 ml Wasser hydrolysiert und zweimal mit 250 ml Essigester extrahiert. Die vereinigten organischen Extrakte wurden über MgSO₄ getrocknet und vom Lösungsmittel befreit. Das Rohprodukt, 71 g gelbes Oel, wurde destilliert. Man erhielt 20,9 g (33%) 4,4-Dimethoxy-1,2-diphenyl-1-butanon-(E/Z)-dimethylhydrazon; Sdp.: 114-116°C/0.03 mbar.
b) 18,5 g 4,4-Dimethoxy-1,2-diphenyl-1-butanon-(E/Z)-dimethylhydrazon, gelöst in 125 ml Tetrahydrofuran, wurden unter Rühren und Argon bei Raumtemperatur mit 250 ml 2N Salzsäurelösung versetzt und über Nacht bei Raumtemperatur gerührt. Das Reaktionsgemisch wurde einmal mit 400 ml und zweimal mit je 200 ml Methylenchlorid extrahiert. Die organischen Extrakte wurden vereinigt, mit MgSO₄ getrocknet und vom Lösungsmittel befreit. Der Rückstand, 15,2 g gelbes Oel, wurde ohne weitere Reinigung in 60 ml Essigsäure gelöst und unter Argon und Rühren zu einer Lösung von 6,5 g N-Acetylethylendiamin in 40 ml Essigsäure gegeben. Nach Zugabe von 350 ml Ethanol wurde die Reaktionslösung 1 Stunde bei Raumtemperatur gerührt, anschliessend wurden Lösungsmittel und Essigsäure im Vakuum entfernt. Der Rückstand wurde in 250 ml Methylenchlorid aufgenommen und mit einem Gemisch aus 250 ml gesättigter Natriumhydrogencarbonatlösung und 130 ml 2N Natronlauge gewaschen. Die wässrige Phase wurde dreimal mit je 100 ml Methylenchlorid nachextrahiert. Die organischen Phasen wurden vereinigt, mit MgSO₄ getrocknet und vom Lösungsmittel befreit. Der Rückstand, 16,8 g braunes Oel, wurde an 500 g Kieselgel mit Essigester/Hexan (2:1) bzw. Essigester als Laufmittel chromatographiert.
   5,8 g (30%) N-[2-[2,3-Diphenylpyrrol-1-yl]ethyl]acetamid wurden als hellgelber, amorpher Feststoff isoliert.
   MS: m/e = 304 (M⁺)
c) 5,8 g N-[2-[2,3-Diphenylpyrrol-1-yl]ethyl]acetamid wurden unter Argon mit 25 ml Phosphoroxychlorid versetzt und 1 Stunde unter Rühren gekocht. Das Reaktionsgemisch wurde bei 0°C mit 100 ml 2N- und 150 ml 28%iger Natronlauge hydrolysiert, mit 1500 ml Wasser verdünnt und mit Methylenchlorid (1 x 300 ml, 3x150 ml) extrahiert. Die organischen Extrakte wurden vereinigt, einmal mit 100 ml gesättigter Natriumchloridlösung gewaschen, mit MgSO₄ getrocknet und vom Lösungsmittel befreit. 3,5 g von insgesamt 5,5 g Rohprodukt wurden in der 25fachen Menge Ethanol gelöst und mit 1,1 Aequivalenten einer gesättigten Lösung von Fumarsäure in Ethanol versetzt. Nach Zugabe von 50 ml Essigester kristallisierten 3,6 g (74%) 3,4-Dihydro-6,7-diphenyl-1-methylpyrrolo[1,2-a]pyrazin-fumarat (1:1); Schmp.: 168-170°C (Zers.).

### Beispiel 39

2,0 g 3,4-Dihydro-6,7-diphenyl-1-methylpyrrolo[1,2-a]pyrazin wurden unter Argon in einem Gemisch aus 100 ml Methanol und 10 ml Wasser gelöst. Die Lösung wurde unter Rühren portionenweise mit 0,8 g Natriumborhydrid versetzt und über Nacht bei Raumtemperatur gerührt. Danach wurde das Methanol im Vakuum entfernt, der Rückstand in 150 ml Methylenchlorid aufgenommen und mit 100 ml 10%iger Ammoniaklösung gewaschen. Die Phasen wurden getrennt und die wässrige Phase wurde zweimal mit je 60 ml Methylenchlorid extrahiert. Die organischen Phasen wurden vereinigt, mit MgSO₄ getrocknet und vom Lösungsmittel befreit. Das Rohprodukt, 2,0 g gelbes Harz, wurde in der 20fachen Menge Ethanol gelöst und mit 1,1 Aequivalenten einer gesättigten Lösung von Fumarsäure in Ethanol kurz aufgekocht. Man erhielt 2,1 g (86%) 6,7-Diphenyl-1,2,3,4-tetrahydro-1-methylpryrolo[1,2-a]pyrazin-fumarat (2:1); Schmp.: 187-190°C (Zers.).

### Beispiel 40

a) Zu einer Lösung von 7,1 ml Diisopropylamin in 200 ml absolutem Tetrahydrofuran wurden unter Argon und Rühren bei -70°C binnen 15 Minuten 32 ml einer 1,6 N Lösung von n-Butyllithium in n-Hexan getropft. Das Reaktionsgemisch wurde bei 0°C 15 Minuten gerührt, dann erneut auf -70°C abgekühlt, mit 30 ml Hexamethylphosphorsäuretriamid und 9,5 g 3-(1,3-Dioxolan-2-yl)propiophenon, gelöst in 10 ml absolutem Tetrahydrofuran, versetzt und 15 Minuten gerührt. Nach Zugabe von 3,2 ml Methyliodid bei -70°C wurde das Gemisch auf Raumtemperatur gebracht und 2 Stunden gerührt. Die Reaktionslösung wurde auf 100 ml gesättigte Ammoniumchloridlösung gegossen und einmal mit 200 ml und zweimal mit je 150 ml Essigester extrahiert. Die organischen Phasen wurden mit 100 ml Wasser gewaschen, vereinigt, mit MgSO ₄ getrocknet und filtriert. Das Filtrat wurde eingeengt und der Rückstand, 23,4 braunes Oel, wurde an 750 g Kieselgel mit Hexan/Essigester-Gemischen (4:1 bis 2:1) chromatographiert.
   7,1 g (70%) 3-(1,3-Dioxolan-2-yl)-2-methylpropiophenon wurden als gelbes Oel isoliert.
b) 7,1 g 3-(1,3-Dioxolan-2-yl)-2-methylpropiophenon wurden unter Argon und Rühren zu einer Lösung von 6,6 g N-Acetylethylendiamin in 100 ml Essigsäure gegeben. Die Reaktionslösung wurde 18 Stunden gekocht und anschliessend wurde die Essigsäure im Vakuum entfernt. Der Rückstand wurde in 200 ml Methylenchlorid aufgenommen und mit einem Gemisch aus 100 ml gesättigter Natriumhydrogencarbonatlösung und 160 ml 2N Natronlauge gewaschen. Die wässrige Phase wurde zweimal mit je 100 ml Methylenchlorid nachextrahiert. Die organischen Phasen wurden vereinigt, mit MgSO₄ getrocknet und vom Lösungsmittel befreit. Der Rückstand, 7,6 g braunes Oel, wurde an 350 g Kieselgel mit Essigester/Hexan-Gemischen (1:3 bis 2:1) als Laufmittel chromatographiert.
   4,6 g (59%) N-[2-[3-Methyl-2-phenylpyrrol-1-yl]ethyl]acetamid wurden als brauner, amorpher Feststoff isoliert.
   MS: m/e = 242 (M⁺)
c) 2,6 g N-[2-[3-Methyl-2-phenylpyrrol-1-yl]ethyl]acetamid wurden unter Argon mit 20 ml Phosphoroxychlorid versetzt und 1 Stunde unter Rühren gekocht. Das Reaktionsgemisch wurde bei 0°C mit 100 ml 2N- und 100 ml 28%iger Natronlauge hydrolysiert, mit 1000 ml Wasser verdünnt und mit Methylenchlorid (1 x 300ml, 2x150 ml) extrahiert. Die organischen Extrakte wurden vereinigt, einmal mit 100 ml Wasser gewaschen, mit MgSO₄ getrocknet und vom Lösungsmittel befreit. 2,1 g Rohprodukt wurden in der 25fachen Menge Ethanol in der Siedehitze gelöst und mit 1,1 Aequivalenten einer gesättigten Lösung von Fumarsäure in Ethanol versetzt. Nach Zugabe von 50 ml Essigester fielen 2,0 g hellgelbe Kristalle, die aus Essigester/Ethanol (1:1) umkristallisiert wurden. 1,3 g (30%) 3,4-Dihydro-1,7-dimethyl-6-phenylpyrrolo[1,2-a]pyrazin-fumarat (2:3); Schmp.: 157-159°C (Zers.).

### Beispiel 41

0,6 g 3,4-Dihydro-1,7-dimethyl-6-phenylpyrrolo[1,2-a]pyrazin wurden unter Argon in einem Gemisch aus 40 ml Methanol und 4 ml Wasser gelöst. Die Lösung wurde unter Rühren portionenweise mit 0,3 g Natriumborhydrid versetzt und über Nacht bei Raumtemperatur gerührt. Danach wurde das Methanol im Vakuum entfernt, der Rückstand in 50 ml Methylenchlorid aufgenommen und mit 50 ml 10%iger Ammoniaklösung gewaschen. Die Phasen wurden getrennt und die wässrige Phase wurde zweimal mit je 30 ml Methylenchlorid extrahiert. Die organischen Phasen wurden vereinigt, mit MgSO₄ getrocknet und vom Lösungsmittel befreit. Das Rohprodukt, 0,6 g gelber, amorpher Feststoff, wurde in der 25fachen Menge Ethanol gelöst und mit 1,1 Aequivalenten einer gesättigten Lösung von Fumarsäure in Ethanol versetzt. Man erhielt 0,5 g (63%) 1,2,3,4-Tetrahydro-1,7-dimethyl-6-phenylpyrrolo[1,2-a]pyrazin-fumarat (2:1); Schmp.: 152-154°C (Zers.).

### Beispiel 42

a) 4,2 g 3-Phenylfuran wurden unter Argon in 100 ml absolutem Tetrahydrofuran gelöst und portionenweise unter Rühren mit 5,2 g N-Bromsuccinimid versetzt. Die Lösung wurde nach 2 Stunden Rühren bei Raumtemperatur mit weiteren 0,2 g N-Bromsuccinimid versetzt und noch 1 Stunde gerührt. Anschliessend wurde die Lösung auf etwa 20 ml eingeengt, mit 100ml Pentan versetzt und das ausgefallene Succinimid wurde abfiltriert. Das Filtrat wurde zweimal mit je 100 ml 1%iger Natriumhydrogencarbonatlösung gewaschen und anschliessend mit Na₂SO₄ unter Zugabe von wenig Calciumcarbonat und Hydrochinon getrocknet. Es wurde filtriert, auf 10 ml eingeengt und mit absolutem Tetrahydrofuran auf 100 ml aufgefüllt. Diese Lösung wurde ohne weitere Reinigung unter Argon auf -78°C abgekühlt, mit 8,7 ml N,N,N,N'-Tetramethylethylendiamin und 3,7 ml einer 1,6 N Lösung von n-Butyllithium in n-Hexan unter Rühren versetzt und 1 Stunde bei -78°C gerührt. Dann wurden zwischen -50°C und -30°C 3,1 ml Metyliodid zugetropft und die Lösung 30 Minuten bei -20°C gerührt. Aufgearbeitet wurde durch Zugabe von 100 ml gesättigter Ammoniumchloridlösung und 40 ml Wasser. Die Mischung wurde dreimal mit je 100 ml Essigester extrahiert, die organischen Phasen wurden vereinigt, mit MgSO₄ getrocknet, filtriert und eingeengt. Der Rückstand, 4,6 g braunes Oel, wurde an 100 g Aluminiumoxid (neutral, III) mit Hexan als Laufmittel chromatographiert. 4,0 g gelbliches Oel wurden isoliert, in 80 ml trockenem Acetonitril unter Argon gelöst und 4 Stunden bei Raumtemperatur mit 7,9 g des gemischten Anhydrids aus Essig- und p-Toluolsulfonsäure gerührt. Anschliessend wurde mit 150 ml gesättigter Natriumhydrogencarbonatlösung und 150 ml Diethylether für 30 Minuten gerührt. Die Phasen wurden getrennt und die wässrige zweimal mit weiteren 100 ml Diethylether extrahiert. Die organischen Phasen wurden vereinigt, zweimal mit je 50 ml Wasser gewaschen, über MgSO₄ getrocknet und vom Lösungsmittel befreit. Der Rückstand, 4,2 g braunes Oel, wurde an 200 g Kieselgel mit Diethylether/Hexan (1:5) als Laufmittel chromatographiert. Man erhielt 1,9 g (18%) 2-Acetyl-5-methyl-4-phenylfuran.
   MS: (m/e) = 200 (M⁺)
b) 1,9 g 2-Acetyl-5-methyl-4-phenylfuran wurden unter Argon mit 6,3 ml 1,2-Ethandiamin und 0,6 ml Wasser für 1 Stunde auf 150°C erhitzt. Nach Abkühlen wurde auf 500 ml Wasser gegossen und das Gemisch dreimal mit je 80 ml Methylenchlorid extrahiert. Die organischen Phasen wurden vereinigt, mit MgSO₄ getrocknet und vom Lösungsmittel befreit. Der Rückstand, 1,9 g braunes Oel, wurde an 100 g Aluminiumoxid (neutral, III) mit Methylenchlorid als Laufmittel chromatographiert. 0,6 g von insgesamt 1,2 g (56%) Rohprodukt wurden in der 20fachen Menge Ethanol gelöst und mit 1,1 Aequivalenten einer gesättigten Lösung von Fumarsäure in Ethanol versetzt. Man erhielt 0,4 g 3,4-Dihydro-1,6-dimethyl-7-phenylpyrrolo[1,2-a]pyrazin-fumarat (1:1); Schmp.: 180-182°C (Zers.).

### Beispiel 43

0,5 g 3,4-Dihydro-1,6-dimethyl-7-phenylpyrrolo[1,2-a]pyrazin wurden unter Argon in einem Gemisch aus 20 ml Methanol und 2 ml Wasser gelöst. Die Lösung wurde unter Rühren portionenweise mit 0,25 g Natriumborhydrid versetzt und über Nacht bei Raumtemperatur gerührt. Danach wurde das Methanol im Vakuum entfernt, der Rückstand in 50 ml Methylenchlorid aufgenommen und mit 50 ml 10%iger Ammoniaklösung gewaschen. Die Phasen wurden getrennt und die wässrige Phase wurde zweimal mit je 30 ml Methylenchlorid extrahiert. Die organischen Phasen wurden vereinigt, mit MgSO₄ getrocknet und vom Lösungsmittel befreit. Das Rohprodukt, 0,6 g gelbes Oel, wurde in der 20fachen Menge Ethanol gelöst und mit 1,1 Aequivalenten einer gesättigten Lösung von Fumarsäure in Ethanol versetzt. Man erhielt 0,5 g (66%) 1,2,3,4-Tetrahydro-1,6-dimethyl-7-phenylpyrrolo[1,2-a]pyrazin-fumarat (1:1); Schmp.: 196-197°C (Zers.).

### Beispiel 44

Zu einer Lösung von 5,0 g 3,4-Dihydro-6-(4-hydroxyphenyl)-1-methylpyrrolo[1,2-a]pyrazin, 2,5 g endo-Norborneol und 8,7 g Triphenylphosphin in 250 ml absolutem Tetrahydrofuran unter Argon wurden bei Raumtemperatur und unter Rühren 6,3 ml Azodicarbonsäurediethylester getropft. Das Reaktionsgemisch wurde 24 Stunden gekocht, abgekühlt und mit 100 ml Wasser versetzt. Es wurde einmal mit 200 ml und zweimal mit je 100 ml Essigester extrahiert. Die organischen Phasen wurden zweimal mit je 100 ml gesättigter Natriumchloridlösung gewaschen, vereinigt, mit MgSO₄ getrocknet und vom Lösungsmittel befreit. Der Rückstand, 24,8 g braunes Oel, wurde an 500 g Kieselgel mit Methylenchlorid/Methanol-Gemischen (100:1 bis 10:1) als Laufmittel chromatographiert. 2,8 g von insgesamt 4,8 g Rohprodukt wurden in der 20fachen Menge Ethanol gelöst und mit 1,1 Aequivalenten einer gesättigten Lösung von Fumarsäure in Ethanol versetzt. Nach Zugabe von 50 ml Essigester erhielt man 3,1 g (55%) 3,4-Dihydro-1-methyl-6-[p-(2-exonorbornyloxy)phenyl-pyrrolo[1,2-a]pyrazin-fumarat (1:1); Schmp.: 162-163°C (Zers.).

### Beispiel 45

2,0 g 3,4-Dihydro-1-methyl-6-[p-(2-exonorbornyloxy)phenyl-pyrrolo[1,2-a]pyrazin wurden unter Argon in einem Gemisch aus 100 ml Methanol und 10 ml Wasser gelöst. Die Lösung wurde unter Rühren portionenweise mit 0,7 g Natriumborhydrid versetzt und über Nacht bei Raumtemperatur gerührt. Danach wurde das Methanol im Vakuum entfernt, der Rückstand in 100 ml Methylenchlorid aufgenommen und mit 100 ml 10%iger Ammoniaklösung gewaschen. Die Phasen wurden getrennt und die wässrige Phase wurde zweimal mit je 50 ml Methylenchlorid extrahiert. Die organischen Phasen wurden vereinigt, mit MgSO₄ getrocknet und vom Lösungsmittel befreit. Das Rohprodukt, 1,6 g amorpher, rosa Feststoff, wurde in der 20fachen Menge Ethanol gelöst und mit 1,1 Aequivalenten einer gesättigten Lösung von Fumarsäure in Ethanol versetzt. Man erhielt 1,2 g rosa gefärbte Kristalle, die aus 50 ml Ethanol umkristallisiert wurden. 0,9 g (38%) 1,2,3,4-Tetrahydro-1-methyl-6-[p-(2-exonorbornyloxy)-phenyl]-pyrrolo[1,2-a]pyrazin-fumarat (2:1); Schmp.: 177-180°C (Zers.).

### Beispiel 46

a) 10,4 g N-[2-[2-(p-Methoxyphenyl)pyrrol-1-yl]ethyl]acetamid wurden unter Argon in 300 ml Methylenchlorid gelöst, auf -15°C abgekühlt und unter Rühren bei dieser Temperatur mit einer Lösung von 11,6 ml Bortribromid in 90 ml Methylenchlorid versetzt. Es wurde 1 Stunde bei -15°C, 2 Stunden bei 0°C und über Nacht bei Raumtemperatur gerührt. Das Reaktionsgemisch wurde bei -10°C mit 270 ml 2N Natronlauge versetzt und 1 Stunde bei Raumtemperatur gerührt. Zu dieser Mischung wurden 200 ml Methylenchlorid gegeben und das Gemisch mit 70 ml 2N Salzsäure auf pH=8 eingestellt. Die Phasen wurden getrennt und die wässrige wurde zweimal mit je 150 ml Methylenchlorid extrahiert. Die organischen Phasen wurden vereinigt, mit MgSO₄ getrocknet und vom Lösungsmittel befreit. Der Rückstand, 8,2 g braunes Oel, wurde an 250 g Kieselgel mit Methylenchlorid/Methanol-Gemischen (50:1 bis 20:1) als Laufmittel chromatographiert. Man erhielt 5,4 g (55%) N-[2-[2-(p-Hydroxyphenyl)pyrrol-1-yl]ethyl]acetamid als farblosen amorphen Feststoff, der ohne weitere Reinigung weiterverwendet wurde. MS: (m/e) = 244(M⁺)
b) 36,0 g N-[2-[2-(p-Hydroxyphenyl)pyrrol-1-yl]ethyl]acetamid wurden unter Argon in 350 ml Dioxan und 350 ml Wasser gelöst und mit 44 ml Benzylchloroformat und 50 g Natriumhydrogencarbonat 4 Stunden bei Raumtemperatur gerührt. Anschliessend wurden 500 ml Essigester und 500 ml Wasser zugegeben, die Phasen wurden getrennt und die wässrige wurde zweimal mit je 300 ml Essigester extrahiert. Die organischen Extrakte wurden einmal mit 300 ml gesättigter Natriumchloridlösung gewaschen, vereinigt, mit MgSO₄ getrocknet und vom Lösungsmittel befreit. Der Rückstand, 80,1 g braunes Oel, wurde in 250 ml Essigester aufgenommen. Es fielen 35,0 g (63%) Kohlensäurebenzylester-4-[1-(2-N-acetylamino-ethyl)pyrrol-2-yl]phenylester; Schmp.: 114-117°C.
c) Unter Argon wurden 6,4 g Kohlensäurebenzylester-4-[1-(2-N-acetylamino-ethyl)pyrrol-2-yl]phenylester mit 20 ml Phosphoroxychlorid versetzt und das Gemisch für 30 Minuten gekocht. Die abgekühlte Mischung wurde auf 2500 g Eiswasser gegeben und mit 28%iger Natronlauge stark basisch gestellt. Das Gemisch wurde viermal mit je 200 ml Methylenchlorid extrahiert, die organischen Phasen wurden vereinigt, mit MgSO₄ getrocknet und vom Lösungsmittel befreit. 0,8 g von insgesamt 6,2 g Rohprodukt wurden in der 25fachen Menge Ethanol heiss gelöst und in der Hitze mit 1,1 Aequivalenten einer gesättigten Lösung von Fumarsäure in Ethanol versetzt. Man erhielt 0,7 g (67%) Kohlensäurebenzylester-4-(3,4-dihydro-1-methylpyrrolo[1,2-a]pyrazin-6-yl)-phenylester-fumarat (1:1); Schmp.: 165-167°C (Zers.).

### Beispiel 47

Unter Argon wurden 0,23 g 3,4-Dihydro-6-(4-hydroxyphenyl)-1-methylpyrrolo[1,2-a]pyrazin in 10 ml Dimethylacetamid auf 0°C abgekühlt. Unter Rühren wurden bei dieser Temperatur 42 mg Natriumhydriddispersion (60%ig) zugegeben und eine halbe Stunde bei 0°C gerührt. Anschliessend wurde binnen 5 Minuten eine Lösung von 0,36 g N-Phenyl-bis-(triflourmethansulfonimid) in 3 ml Tetrahydrofuran zugetropft und eine Stunde bei 0°C gerührt. Anschliessend wurden 20 ml gesättigte Ammoniumchloridlösung und 10 ml Wasser zugegeben. Das Gemisch wurde zweimal mit je 25 ml Essigester extrahiert, die organischen Phasen wurden mit 20 ml 1N Natriumcarbonatlösung und 30 ml gesättigter Natriumchloridlösung gewaschen, vereinigt, mit MgSO₄ getrocknet und vom Lösungsmittel befreit. Der Rückstand, 0,65 g orangegefärbtes Oel, wurde an 30 g Kieselgel mit Methylenchlorid/Methanol-Gemischen (50:1 bis 20:1) chromatographiert. Man erhielt 0,24 g hellgelbes Oel, das in der 20fachen Menge Ethanol gelöst und mit 1,1 Aequivalenten einer gesättigten Lösung von Fumarsäure in Ethanol versetzt wurde. Nach Zugabe von 10 ml Essigester erhielt man 0,2 g (42%) Triflourmethansulfonsäure-4-(3,4-dihydro-1-methylpyrrolo[1,2-a]pyrazin-6-yl)phenylester-fumarat (1:1); Schmp.: 146°C (Zers.).

### Beispiel 48

Zu einer Lösung von 1,5 g 3,4-Dihydro-6-(4-hydroxyphenyl)-1-methylpyrrolo[1,2-a]pyrazin in 20 ml 2-Butanon wurden unter Rühren und Argon bei Raumtemperatur 1,8 g Toluolsulfonsäure-3-hydroxybutan-1-yl-ester und 1,8 g Kaliumcarbonat gegeben und 20 Stunden gekocht, Das abgekühlte Reaktionsgemisch wurde mit 100 ml 2N Natronlauge versetzt und dreimal mit je 200 ml Methylenchlorid extrahiert. Die organischen Phasen wurden zweimal mit je 100 ml Wasser gewaschen, vereinigt, mit MgSO₄ getrocknet und vom Lösungsmittel befreit. Der Rückstand, 2,4 g braunes Oel, wurde an 50 g Kieselgel mit Methylenchlorid/Methanol-Gemischen (50:1 bis 20:1) als Laufmittel chromatographiert. Man erhielt 1,2 g orangegefärbten, amorphen Feststoff, der in der 25fachen Menge Ethanol heiss gelöst und in der Hitze mit 1,1 Aequivalenten einer gesättigten Lösung von Fumarsäure in Ethanol versetzt wurde Man erhielt 1.5 g (90%) 4-[4-(3,4-Dihydro-1-methylpyrrolo[1,2-a]pyrazin-6-yl)phenoxy]butan-2-ol-fumarat (1:1); Schmp.: 156-157°C (Zers.).

### Beispiel 49

a) 198 g 3,4-Dihydro-6-methoxy-1(2H)-naphtalenon wurden unter Argon in 2000 ml Toluol gelöst und und nacheinander mit 193 ml 3-Buten-2-ol, 206 ml Dimethoxypropan und 1,9 g p-Toluolsulfonsäure versetzt. Das Reaktionsgemisch wurde 20 Stunden am Rückfluss gekocht, mit weiteren 175 ml 3-Buten-2-ol und 180 ml Dimethoxypropan versetzt und weitere 50 Stunden gekocht. Nach Abkühlen wurde die Lösung mit 500 ml gesättigter Natriumhydrogencarbonatlösung gewaschen, die wässrige Phase wurde einmal mit 600 ml Essigester extrahiert, die organischen Phasen wurden vereinigt, mit MgSO₄ getrocknet und vom Lösungsmittel befreit. Der Rückstand, 269 g braunes Oel, wurde an 3000 g Kieselgel mit Hexan/Diethylether (5:1) als Laufmittel chromatographiert. Man erhielt 147 g (57%) 2-(2-Butenyl)-3,4-dihydro-6-methoxy-1-(2H)-naphtalenon, die ohne weitere Reinigung weiterverwendet wurden.
b) 65,3 g 2-(2-Butenyl)-3,4-dihydro-6-methoxy-1(2H)-naphtalenon wurden in einem Gemisch aus 2000 ml Methylenchlorid und 500 ml Methanol gelöst, auf -78°C abgekühlt und ozonisiert. Nach beendeter Reaktion wurden bei -78°C 29 ml Dimethylsulfid zugegeben und die Mischung wurde über Nacht auf Raumtemperatur gebracht. Anschliessend wurde das Lösungsmittel entfernt. Der Rückstand, 97,2 g orangegefärbtes Oel, wurde in 2000 ml Methylenchlorid gelöst, mit 400 g Kieselgel und 80 ml 10%iger Oxalsäure versetzt und über Nacht bei Raumtemperatur unter Argon gerührt. Die Mischung wurde filtriert und das Filtrat eingeengt. Der Rückstand, 71,8 g grünes Oel, wurde ohne weitere Reinigung unter Argon in 400 ml Methylenchlorid gelöst, mit 200 g Molekularsieb 4 Å und 37,1 g N-Acetylethylendiamin versetzt und über Nacht gekocht. Das Reaktionsgemisch wurde abgekühlt, über ein Celite®-Polster filtriert und das Filtrat vom Lösungsmittel befreit. Der Rückstand, 98,0 g brauner, kristalliner Feststoff, wurde über 1000 g Kieselgel mit Essigester als Laufmittel filtriert und das Filtrat auf 350 ml Volumen eingeengt. Es fielen 48,4 g (60%) N-[2-(4,5-Dihydro-7-methoxy-1H-benz[g]indol-1-yl)-ethyl]acetamid; Schmp.: 134°C.
c) Unter Argon wurden 14,5 g N-[2-(4,5-Dihydro-7-methoxy-1H-benz[g]indol-1-yl)ethyl]acetamid mit 50 ml Phosphoroxychlorid versetzt und das Gemisch für 30 Minuten bei 50°C gerührt. Die abgekühlte Mischung wurde auf 5000 g Eiswasser gegeben und mit 500 ml 28%iger Natronlauge versetzt. Das Gemisch wurde einmal mit 1000 ml und zweimal mit je 500 ml Methylenchlorid extrahiert, die organischen Phasen wurden vereinigt, mit MgSO₄ getrocknet und vom Lösungsmittel befreit. Der Rückstand, 14,6 g braunes Oel, wurde an 220 g Aluminiumoxid (neutral, III) mit Methylenchlorid als Laufmittel chromatographiert. 1,3 g von insgesamt 10,3 g Rohprodukt wurden in der 25fachen Menge Ethanol gelöst und mit 1,1 Aequivalenten einer gesättigten Lösung von Fumarsäure in Ethanol versetzt. Man erhielt 1,2 g (49%) 5,6,10,11-Tetrahydro-3-methoxy-8-methyl-benzo[g]pyrazino[1,2-a]indol-fumarat (1:1); Schmp.: 202-203°C (Zers.).

### Beispiel 50

a) 48,8 g N-[2-(4,5-Dihydro-7-methoxy-1H-benz[g]indol-3-yl)ethyl]-acetamid wurden unter Argon in 1000 ml Methylenchlorid gelöst, auf -15°C abgekühlt und unter Rühren bei dieser Temperatur mit einer Lösung von 50 ml Bortribromid in 300 ml Methylenchlorid versetzt. Es wurde 1 Stunde bei -15°C, 5 Stunden bei 0°C und über Nacht bei Raumtemperatur gerührt. Das Reaktionsgemisch wurde bei -10⁰C mit 1000 ml 2N Natronlauge versetzt und 1 Stunde bei Raumtemperatur gerührt. Zu dieser Mischung wurden 500 ml Methylenchlorid gegeben und das Gemisch mit 250 ml 2N Salzsäure auf pH=8 eingestellt. Die Phasen wurden getrennt und die wässrige wurde zweimal mit je 500 ml Methylenchlorid extrahiert. Die organischen Phasen wurden vereinigt, mit MgSO₄ getrocknet und vom Lösungsmittel befreit. Der Rückstand, 33,2 g gelbbraunes, amorphes Material wurde an 500 g Kieselgel mit Methylenchlorid/Methanol-Gemischen (50:1 bis 20:1) als Laufmittel chromatographiert. Man erhielt 30,2 g (65%) N-(2-[4,5-Dihydro-7-hydroxy-1H-benz[g]indol-1-yl)ethyl]acetamid, das ohne weitere Reinigung weiterverwendet wurde.
   MS: (m/e) = 270 (M⁺)
b) 29,7 g N-[2-[4,5-Dihydro-7-hydroxy-1H-benz[g]indol-1-yl)ethyl]-acetamid wurden unter Argon in 300 ml Dioxan und 300 ml Wasser gelöst und mit 28,4 g Chlorameisensäur-4-nitrobenzylester und 22,2 g Natriumhydrogencarbonat über Nacht bei Raumtemperatur gerührt. Danach wurden weitere 10,0 g Chlorameisensäur-4-nitrobenzylester und 7,8 g Natriumhydrogencarbonat zugegeben und noch 1 Stunde bei Raumtemperatur gerührt. Anschliessend wurden 1000 ml Essigester und 500 ml Wasser zugegeben, die Phasen wurden getrennt und die wässrige wurde zweimal mit je 1000 ml Essigester extrahiert. Die organischen Extrakte wurden vereinigt, mit MgSO₄ getrocknet und auf ein Volumen von 300 ml konzentriert. Es fielen 30,5 g (67%) Kohlensäure-1-(2-N-acetylamino-ethyl)-4,5-dihydro-1H-benz[g]indol-7-yl-ester-4-nitrobenzylester; Schmp.: 144-148°C.
c) Unter Argon wurden 41,2 g Kohlensäure-1-(2-N-acetylaminoethyl)-4,5-dihydro-1H-benz[g]indol-7-yl-ester-4-nitrobenzylester mit 100 ml Phosphoroxychlorid versetzt und das Gemisch für 30 Minuten auf 50°C erhitzt. Die abgekühlte Mischung wurde auf 20 1 Eiswasser gegeben und mit 700 ml 28%iger Natronlauge versetzt. Das Gemisch wurde einmal mit 3 1 und zweimal mit je 1 l Methylenchlorid extrahiert, die organischen Phasen wurden vereinigt, mit MgSO₄ getrocknet und vom Lösungsmittel befreit. 1,5 g von insgesamt 39,2 g Rohprodukt wurden in der 25fachen Menge Ethanol heiss gelöst und in der Hitze mit 1,1 Aequivalenten einer gesättigten Lösung von Fumarsäure in Ethanol versetzt. Man erhielt 1,5 g (78%) Kohlensäure-5,6,10,11-tetrahydro-8-methylbenzo[g]pyrazino[1,2-a]indol-3-yl-ester-4-nitrobenzylester-fumarat (1:1); Schmp.: 175-176°C (Zers.).

### Beispiel 51

37,8 g Kohlensäure-5,6,10,11-tetrahydro-8-methyl-benzo[g]pyrazino[1,2-a]indol-3-yl-ester-4-nitrobenzylester wurden unter Argon in 800 ml Methylenchlorid gelöst und nach Zugabe von 75 ml n-Propylamin über Nacht bei Raumtemperatur grührt. Das Produkt fiel in Form von 21,8 g (98%) ockerfarbenen Kristallen; Schmp.: >250°C. 0,4 g davon wurden in 20 ml Methanol suspendiert und mit 1 Aequivalent Methansulfonsäure versetzt. Nach Zugabe von 50 ml Diethylether fielen 0,4 g 5,6,10,11-Tetrahydro-8-methyl-benzo[g]pyrazino[1,2-a]indol-3-ol-methansulfonat (1:1); Schmp.: >240°C.

### Beispiel 52

a) Unter Argon wurden 5,0 g N-[2-(4,5-Dihydro-5-methyl-1H-benz[g]indol-1-yl)ethyl]acetamid in 80 ml Toluol suspendiert, mit 4,7 g 2,3-Dichlor-5,6-dicyan-1,4-benzochinon versetzt und 4 Stunden bei 80°C gerührt. Es wurden weitere 1,0 g 2,3-Dichlor-5,6-dicyan-1,4-benzochinon zugegeben und noch 1 Stunde bei 80°C gerührt. Die Lösung wurde abgekühlt und über 60 g Aluminiumoxid (neutral, III) mit Essigester als Laufmittel filtriert. Das Filtrat wurde eingeengt und lieferte 0,3 g (6%) N-[2-(5-Methyl-1H-benz[g]indol-1-yl)ethyl]acetamid, als amorphen, gelben Feststoff, der ohne weitere Reinigung weiterverwendet wurden.
b) Unter Argon wurden 0,3 g N-[2-(5-Methyl-1H-benz[g]indol-1-yl)ethyl]acetamid mit 3 ml Phosphoroxychlorid versetzt und das Gemisch für 30 Minuten auf 100°C erhitzt. Die abgekühlte Mischung wurde auf 250 g Eiswasser gegeben und mit 30 ml 28%iger Natronlauge versetzt. Das Gemisch wurde einmal mit 100 ml und zweimal mit je 50 ml Methylenchlorid extrahiert, die organischen Phasen wurden vereinigt, mit MgSO₄ getrocknet und vom Lösungsmittel befreit. Der Rückstand, 0,14 g braunes Oel, wurde an 10 g Aluminiumoxid (neutral, III) mit Methylenchlorid als Laufmittel chromatographiert. Das Rohprodukt, 90 mg gelbbrauner, amorpher Feststoff, wurde in 1 ml Ethanol und 3 ml Diethylether gelöst und mit 1,1 Aequivalenten einer gesättigten Lösung von Fumarsäure in Ethanol versetzt. Man erhielt 58 mg (17%) 10,11-Dihydro-5,8-dimethyl-benzo[g]pyrazino[1,2-a]indol-fumarat (2:1); Schmp.: 184-186°C (Zers.).

### Beispiel 53

Zu einer Lösung von 4,0 g 5,6,10,11-Tetrahydro-8-methyl-benzo[g]pyrazino[1,2-a]indol-3-ol, 1,5 ml Cyclopentanol und 6,2 g Triphenylphosphin in 250 ml absolutem Tetrahydrofuran unter Argon wurden bei Raumtemperatur und unter Rühren 3,7 ml Azodicarbonsäurediethylester getropft. Das Reaktionsgemisch wurde über Nacht gekocht und dann mit weiteren 2,0 g Triphenylphosphin und 1,2 ml Azodicarbonsäureethylester versetzt. Anschliessend wurde abgekühlt und mit 300 ml Wasser versetzt. Es wurde einmal mit 300 ml und zweimal mit je 250 ml Essigester extrahiert. Die organischen Phasen wurden einmal mit 250 ml gesättigter Natriumchloridlösung gewaschen, vereinigt, mit MgSO₄ getrocknet und vom Lösungsmittel befreit. Der Rückstand, 19,6 g braunes Oel, wurde an 500 g Kieselgel mit Methylenchlorid/Methanol-Gemischen (50:1 bis 20:1) als Laufmittel chromatographiert. 1,2 g von insgesamt 2,0 g Rohprodukt wurden in der 20fachen Menge Ethanol gelöst und mit 1,1 Aequivalenten einer gesättigten Lösung von Fumarsäure in Ethanol versetzt. Es fielen 1,1 g (26%) 3-Cyclopentyloxy-5,6,10,11-tetrahydro-8-methyl-benzo[g]pyrazino[1,2-a]indol-fumarat (1:1); Schmp.: 182-185°C (Zers.).

### Beispiel 54

0,8 g 3-Cyclopentyloxy-5,6,10,11-tetrahydro-8-methylbenzo[g]pyrazino[1,2-a]indol wurden unter Argon in einem Gemisch aus 50 ml Methanol und 50 ml Wasser gelöst. Die Lösung wurde unter Rühren portionsweise mit 0,3 g Natriumborhydrid versetzt und über Nacht bei Raumtemperatur gerührt. Danach wurde das Methanol im Vakuum entfernt, der Rückstand in 30 ml Methylenchlorid aufgenommen und mit 50 ml 10%iger Ammoniaklösung gewaschen. Die Phasen wurden getrennt und die wässrige Phase wurde zweimal mit je 30 ml Methylenchlorid extrahiert. Die organischen Phasen wurden vereinigt, mit MgSO₄ getrocknet und vom Lösungsmittel befreit. Der Rückstand, 0,8 g amorpher, brauner Feststoff, wurde an 30 g Kieselgel mit Methylenchlorid/Methanol-Gemischen (50:1 bis 20:1) chromatographiert. Das isolierte Rohprodukt wurde in der 25fachen Menge Ethanol heiss gelöst und mit 1,1 Aequivalenten einer gesättigten Lösung von Fumarsäure in Ethanol versetzt. Man erhielt 0,34 g (31%) 3-Cyclopentyloxy-5,6,8,9,10,11-hexahydro-8-methyl-benzo[g]pyrazino[1,2-a]indol-fumarat (2:1); Schmp.: 187-189°C (Zers.).

### Beispiel 55

Zu einer Lösung von 2,0 g 5,6,10,11-Tetrahydro-8-methyl-benzo[g]pyrazino[1,2-a]indo-3-ol, 0,9 ml Cyclohexanol und 3,1 g Triphenylphosphin in 120 ml absolutem Tetrahydrofuran unter Argon wurden bei Raumtemperatur und unter Rühren 1,9 ml Azodicarbonsäurediethylester getropft. Das Reaktionsgemisch wurde über Nacht gekocht und dann mit weiteren 1,5 g Triphenylphosphin und 1,0 ml Azodicarbonsäureethylester versetzt und erneut über Nacht gekocht. Anschliessend wurde abgekühlt und mit 150 ml Wasser versetzt. Es wurde dreimal mit je 150 ml Essigester extrahiert. Die organischen Phasen wurden einmal mit 100 ml gesättigter Natriumchloridlösung gewaschen, vereinigt, mit MgSO₄ getrocknet und vom Lösungsmittel befreit. Der Rückstand, 9,9 g braunes Oel, wurde an 200 g Kieselgel mit Methylenchlorid/Methanol-Gemischen (50:1 bis 10:1) als Laufmittel chromatographiert. 0,54 g Rohprodukt wurden in der 25fachen Menge Ethanol gelöst und mit 1,1 Aequivalenten einer gesättigten Lösung von Fumarsäure in Ethanol versetzt. Es fielen 0,45 g (12%) 3-Cyclohexyloxy-5,6,10,11-tetrahydro-8-methylbenzo[g]pyrazino[1,2-a]indol-fumarat (3:4); Schmp.: 199-202°C (Zers.).

### Beispiel 56

Zu einer Lösung von 1,0 g 5,6,10,11-Tetrahydro-8-methyl-benzo[g]pyrazino[1,2-a]indol-3-ol, 0,3 ml Isopropanol und 1,6 g Triphenylphosphin in 60 ml absolutem Tetrahydrofuran unter Argon wurden bei Raumtemperatur und unter Rühren 1,0 ml Azodicarbonsäurediethylester getropft. Das Reaktionsgemisch wurde über Nacht gekocht und dann mit weiteren 0,3 ml Isopropanol, 1,5 g Triphenylphosphin und 1,0 ml Azodicarbonsäureethylester versetzt und erneut über Nacht gekocht. Anschliessend wurde abgekühlt und mit 80 ml Essigester versetzt. Es wurde dreimal mit je 30 ml 2N Salzsäure extrahiert. Die wässrigen Phasen wurden einmal mit 80 ml Essigester gewaschen, vereinigt, mit 28%iger Natronlauge stark basisch gemacht und dreimal mit je 60 ml Methylenchlorid extrahiert. Die organischen Phasen wurden vereinigt, mit MgSO₄ getrocknet und vom Lösungsmittel befreit. Der Rückstand, 1,1 g braunes Oel, wurde an 80 g Aluminiumoxid (neutral, III) mit Methylenchlorid als Laufmittel chromatographiert. 0,8 g Rohprodukt wurden in der 20fachen Menge Ethanol heiss gelöst und mit 1,1 Aequivalenten einer gesättigten Lösung von Fumarsäure in Ethanol versetzt. Nach Zugabe von 40 ml Essigester fielen 0,55 g (34%) 5,6,10,11-Tetrahydro-3-isopropoxy-8-methyl-benzo[g]pyrazino[1,2-a]indol-fumarat (1:1); Schmp.: 176-177°C (Zers.).

### Beispiel 57

Zu einer Lösung von 1,0 g 5,6,10,11-Tetrahydro-8-methyl-benzo[g]pyrazino[1,2-a]indol-3-ol, 0,6 ml n-Octanol und 1,6 g Triphenylphosphin in 60 ml absolutem Tetrahydrofuran unter Argon wurden bei Raumtemperatur und unter Rühren 1,0 ml Azodicarbonsäurediethylester getropft. Das Reaktionsgemisch wurde über Nacht gekocht und anschliessend wurde abgekühlt und mit 100 ml Essigester versetzt. Es wurde dreimal mit je 30 ml 2N Salzsäure extrahiert. Die wässrigen Phasen wurden einmal mit 60 ml Essigester extrahiert. Die organischen Phasen wurden vereinigt, mit MgSO₄ getrocknet und vom Lösungsmittel befreit. Der Rückstand, 4,3 g braunes Oel, wurde an 200 g Aluminiumoxid (neutral, III) mit Methylenchlorid als Laufmittel und anschliessend über 40 g Kieselgel mit Methylenchlorid/Methanol-Gemischen (20:1 bis 10:1) chromatographiert. 0,5 g Rohprodukt wurden in der 20fachen Menge Ethanol gelöst und mit 1,1 Aequivalenten einer gesättigten Lösung von Fumarsäure in Ethanol versetzt. Es fielen 0,4 g (21%) 5,6,10,11-Tetrahydro-8-methyl-3-octyloxy-benzo[g]pyrazino[1,2-a]indol-fumarat (1:1); Schmp.: 151-152°C (Zers.).

### Beispiel 58

Zu einer Lösung von 1,0 g 5,6,10,11-Tetrahydro-8-methyl-benzo[g]pyrazino[1,2-a]indol-3-ol, 0,5 g 4-(2-Hydroxyethyl)morpholin und 1,6 g Triphenylphosphin in 60 ml absolutem Tetrahydrofuran unter Argon wurden bei Raumtemperatur und unter Rühren 1,0 ml Azodicarbonsäurediethylester getropft. Das Reaktionsgemisch wurde über Nacht gekocht und anschliessend wurde abgekühlt und mit 100 ml Essigester versetzt. Es wurde dreimal mit je 50 ml 2N Salzsäure extrahiert. Die wässrigen Phasen wurden einmal mit 60 ml Essigester gewaschen, vereinigt, mit 28%iger Natronlauge stark basisch gestellt und dreimal mit je 60 ml Essigester extrahiert. Die organischen Phasen wurden vereinigt, mit MgSO₄ getrocknet und vom Lösungsmittel befreit. Der Rückstand, 1,7 g braunes Oel, wurde an 40 g Kieselgel mit Methylenchlorid/Methanol-Gemischen (100:1 bis 10:1) chromatographiert. 0,15 g Rohprodukt wurden in der 20fachen Menge Ethanol gelöst und mit 1,1 Aequivalenten einer gesättigten Lösung von Fumarsäure in Ethanol versetzt. Es fielen 0,11 g (5%) 5,6,10,11-Tetrahydro-8-methyl-3-(2-morpholin-4-yl-ethoxy)-benzo[g]pyrazino[1,2-a]indol-fumarat (1:2); Schmp.: 195-197°C (Zers.).

### Beispiel 59

Zu einer Lösung von 1,0 g 5,6,10,11-Tetrahydro-8-methyl-benzo[g]pyrazino[1,2-a]indol-3-ol, 0,45 ml 1-(2-Hydroxethyl)-2-pyrrolidon und 1,6 g Triphenylphosphin in 60 ml absolutem Tetrahydrofuran unter Argon wurden bei Raumtemperatur und unter Rühren 1,0 ml Azodicarbonsäurediethylester getropft. Das Reaktionsgemisch wurde über Nacht gekocht und anschliessend wurde abgekühlt und mit 100 ml Essigester versetzt. Es wurde dreimal mit je 30 ml 2N Salzsäure extrahiert. Die wässrigen Phasen wurden einmal mit 80 ml Essigester gewaschen, vereinigt, mit 28%iger Natronlauge stark basisch gestellt und dreimal mit je 60 ml Essigester extrahiert. Die organischen Phasen wurden vereinigt, mit MgSO₄ getrocknet und vom Lösungsmittel befreit. Der Rückstand, 2,4 g braunes Oel, wurde an 150 g Aluminiumoxid (neutral, III) mit Methylenchlorid und Methylenchlorid/Methanol (100:1) als Laufmittel chromatographiert. 1,0 g Rohprodukt wurden in der 20fachen Menge Ethanol gelöst und mit 1,1 Aequivalenten einer gesättigten Lösung von Fumarsäure in Ethanol versetzt. Nach Zugabe von 70 ml Essigester fielen 0,8 g (42%) 1-[2-(5,6,10,11-Tetrahydro-8-methyl-benzo[g]pyrazino[1,2-a]indol-3-yloxy)ethyl]pyrrolidin-2-on-fumarat (1:1); Schmp.: 183-185°C (Zers.).

### Beispiel 60

Zu einer Lösung von 1,0 g 5,6,10,11-Tetrahydro-8-methyl-benzo[g]pyrazino[1,2-a]indol-3-ol in 20 ml 2-Butanon wurden unter Rühren und Argon bei Raumtemperatur 1,1 g Toluolsulfonsäure-3-hydroxybutan-1-yl-ester und 1,0 g Kaliumcarbonat gegeben und über Nacht gekocht, Das abgekühlte Reaktionsgemisch wurde mit 100 ml 2N Natronlauge versetzt und dreimal mit je 200 ml Methylenchlorid extrahiert. Die organischen Phasen wurden zweimal mit je 50 ml Natronlauge gewaschen, vereinigt, mit MgSO₄ getrocknet und vom Lösungsmittel befreit. Der Rückstand, 1,1 g braunes, amorphes Material , wurde an 80 g Aluminiumoxid (neutral, III) mit Methylenchlorid und Methylenchlorid/Methanol (100:1) als Laufmitteln chromatographiert. Man erhielt 0,8 g gelbes Oel, das in der 20fachen Menge Ethanol gelöst und mit 1,1 Aequivalenten einer gesättigten Lösung von Fumarsäure in Ethanol versetzt wurde Man erhielt 0,8 g (46%) 4-(5,6,10,11-Tetrahydro-8-methyl-benzo[g]pyrazino[1,2-a]indol-3-yl-oxy)butan-2-ol-fumarat (1:1); Schmp.: 180-182°C (Zers.).

### Beispiel 61

Unter Argon wurden in eine Lösung von 1,0 g 5,6,10,11-Tetrahydro-8-methyl-benzo[g]pyrazino[1,2-a]indol-3-ol in 50 ml Methylenchlorid 11,0 g Isobutylen einkondensiert. Die Lösung wurde auf -78°C abgekühlt und unter Rühren mit 0,4 ml Triflourmethansulfonsäure versetzt. Es wurde 8 Stunden bei -78°C und 30 Minuten bei -5°C gerührt. Anschliessend wurden 50 ml Methanol zugegeben und mit Natriumhydroxidpulver neutralisiert. Das Reaktionsgemisch wurde vom Lösungsmittel befreit und über 100 g Aluminiumoxid (neutral, III) mit Methylenchlorid als Laufmittel chromatographiert. Das Rohprodukt, 0,3 g gelbes Oel, wurde in der 20fachen Menge Ethanol gelöst und mit 1,1 Aequivalenten einer gesättigten Lösung von Fumarsäure in Ethanol versetzt. Die Lösung wurde auf die Hälfte eingeengt und mit 5 ml Essigester versetzt. Man erhielt 0,11 g (6%) 3-tert-Butoxy-5,6,10,11-tetrahydro-8-methyl-benzo[g]pyrazino[1,2-a]indol-fumarat (1:1); Schmp.: 179-181°C (Zers.).

### Beispiel 62

Unter Argon wurden 2,0 g 5,6,10,11-Tetrahydro-8-methyl-benzo[g]pyrazino[1,2-a]indol-3-ol in 200 ml Dimethylacetamid heiss gelöst und auf 0°C abgekühlt. Unter Rühren wurden bei dieser Temperatur 0,33 g Natriumhydriddispersion (60%ig) zugegeben und 1 Stunde bei 0°C gerührt. Anschliessend wurde binnen 10 Minuten eine Lösung von 2,8 g N-Phenyl-bis-(triflourmethansulfonimid) in 25 ml Tetrahydrofuran zugetropft und eine Stunde bei 0°C gerührt. Anschliessend wurden 400 ml gesättigte Ammoniumchloridlösung und 250 ml Wasser zugegeben. Das Gemisch wurde dreimal mit je 200 ml Essigester extrahiert, die organischen Phasen wurden mit 250 ml Wasser, 200 ml 1N Natriumcarbonatlösung und 200 ml gesättigter Natriumchloridlösung gewaschen, vereinigt, mit MgSO₄ getrocknet und vom Lösungsmittel befreit. Der Rückstand, 4,9 g braunes Oel, wurde an 250 g Kieselgel mit Methylenchlorid/Methanol-Gemischen (50:1 bis 20:1) chromatographiert. Man erhielt 2,1 g (69%) Triflourmethansulfonsäure-5,6,10,11-tetrahydro-8-methyl-benzo[g]pyrazino[1,2-a]indol-3-yl-ester. 0,5 g davon wurden in der 20fachen Menge Ethanol gelöst und mit 1,1 Aequivalenten einer gesättigten Lösung von Fumarsäure in Ethanol versetzt. Es fielen 0,4 g Triflourmethansulfonsäure-5,6,10,11-tetrahydro-8-methyl-benzo[g]pyrazino[1,2-a]indol-3-yl-ester-fumarat (1:1); Schmp.: 165-167°C (Zers.).

### Beispiel 63

a) 50,9 g 3,4-Dihydro-7-methoxy-1(2H)-naphtalenon wurden unter Argon in 200 ml Ethanol suspendiert, mit 44 ml N,N-Dimethylhydrazin (asym.) versetzt und während 168 Stunden unter Rückfluss gekocht. Das Reaktionsgemisch wurde im Vakuum vom Lösungsmittel und überschüssigem Dimethylhydrazin befreit. Der Rückstand wurde in 400 ml Essigester aufgenommen und bei 0°C mit 100 ml 3,3 N HCl in Essigester versetzt. Es wurde 30 Minuten bei 0°C gerührt, die ausgefallenen Kristalle wurden abfiltriert und erneut in Essigester suspendiert. Zu dieser Suspension wurden 500 ml gesättigte Natriumhydrogencarbonatlösung gegeben und 30 Minuten gerührt. Die Phasen wurden getrennt und die wässrige wurde zweimal mit Essigester extrahiert. Die organischen Phasen wurden vereinigt, mit MgSO₄ getrocknet und vom Lösungsmittel befreit. 56,8 g (90%) 3,4-Dihydro-7-methoxy-1(2H)-naphtalenon-N',N'-dimethylhydrazon wurden ohne weitere Reinigung weiterverwendet.
b) 56,8 g 3,4-Dihydro-7-methoxy-1(2H)-naphtalenon-N',N'-dimethylhydrazon wurden unter Argon in 1100 ml absolutem Tetrahydrofuran gelöst und mit 47 ml N,N,N',N'-Tetramethylethylendiamin versetzt. Die Reaktionslösung wurde auf -70°C abgekühlt und unter Rühren binnen 45 Minuten 170 ml einer 1,6 N Lösung von n-Butyllithium in Hexan zugetropft. Es wurde weitere 30 Minuten bei -70°C gerührt und dann bei -30°C binnen 15 Minuten 37 ml Bromacetaldehyd-dimethylacetal zugetropft. Anschliessend wurde 2 Stunden bei -30⁰C und 16 Stunden bei Raumtemperatur gerührt. Das Reaktionsgemisch wurde mit 1400 ml Wasser hydrolysiert, einmal mit 1200 ml und zweimal mit je 800 ml Essigester extrahiert. Die vereinigten organischen Extrakte wurden über MgSO₄ getrocknet und vom Lösungsmittel befreit. Das Rohprodukt wurde an 2000 g Kieselgel mit Hexan/Essigester-Gemischen (4:1 bis 2:1) als Laufmittel chromatographiert. Man erhielt 46,6 g (59%) 1,2,3,4-Tetrahydro-1-(dimethylhydrazono)-7-methoxy-2-naphtalinacetaldehyd-dimethylacetal.
c) 45,0g 1,2,3,4-Tetrahydro-1-(dimethylhydrazono)-7-methoxy-2-naphtalinacetaldehyd-dimethylacetal wurden unter Argon in einem Gemisch von 1800 ml Tetrahydrofuran und 645 ml 0,067 M Phosphatpuffer vom pH-Wert 7 gelöst. Die Lösung wurde mit 19,8 g Kupfer(II)chlorid-dihydrat versetzt und bei Raumtemperatur 16 Stunden gerührt. Zum Reaktionsgemisch wurden 1000 ml Essigester gegeben und dreimal mit je 1500 ml eines Gemisches aus 25%iger Ammoniaklösung und gesättigter Ammoniumchloridlösung (1:19) gewaschen. Die Waschwasserphasen wurden zweimal mit 1000 ml Essigester extrahiert, die organischen Phasen wurden vereinigt, über MgSO₄ getrocknet und vom Lösungsmittel befreit. Man erhielt 39,7 g rohes 1,2,3,4-Tetrahydro-7-methoxy-1-oxo-2-naphtalinacetaldehyd-dimethylacetal, das ohne Reinigung weiterverwendet wurde.
d) Zur Spaltung des Acetals wurden auf 1000 g Kieselgel 200 ml einer 10%igen wässrigen Oxalsäurelösung aufgezogen und an diesem Gemisch 39,7 g 7-Methoxy-1,2,3,4-tetrahydro-1-oxo-2-naphtalinacetaldehyd-dimethylacetal mit Methylenchlorid als Laufmittel chromatographiert. Man erhielt 28,1 g (88% bezogen auf das Produkt aus Beispiel 64.b) 1,2,3,4-Tetrahydro-7-methoxy-1-oxo-2-naphtalinacetaldehyd.
e) 14,5 g N-Acetylethylendiamin wurden unter Argon in 250 ml Methylenchlorid gelöst, sukzessive mit 250 g Molekularsieb 4 Å und einer Lösung von 28,1 g 1,2,3,4-Tetrahydro-7-methoxy-1-oxo-2-naphtalinacetaldehyd in 190 ml Methylenchlorid versetzt und 21 Stunden unter Rühren refluxiert. Das Reaktionsgemisch wurde abgekühlt, über ein Celite®-Polster filtriert und das Filtrat vom Lösungsmittel befreit. Das Rohprodukt, 35,3 g braunes, amorphes Material, wurde an 600 g Kieselgel mit Essigester als Laufmittel chromatographiert. Man erhielt 29,6 g (81%) N-[2-(4,5-Dihydro-8-methoxy-1H-benz[g]indol-1-yl)-ethyl]acetamid, die ohne weitere Reinigung weiterverwendet wurden.
f) Unter Argon wurden 4,7 g N-[2-(4,5-Dihydro-8-methoxy-1H-benz[g]indol-1-yl)-ethyl]acetamid mit 15 ml Phosphoroxychlorid versetzt und das Gemisch für 30 Minuten bei 50°C gerührt. Die abgekühlte Mischung wurde auf 2000 g Eiswasser gegeben und mit 200 ml 28%iger Natronlauge versetzt. Das Gemisch wurde einmal mit 300 ml und zweimal mit je 100 ml Methylenchlorid extrahiert, die organischen Phasen wurden vereinigt, mit MgSO₄ getrocknet und vom Lösungsmittel befreit. 4,2 g Rückstand wurden an 250 g Aluminiumoxid (neutral, III) mit Methylenchlorid als Laufmittel chromatographiert. 3,8 g Rohprodukt wurden in der 20fachen Menge Ethanol heiss gelöst und in der Hitze mit 1,1 Aequivalenten einer gesättigten Lösung von Fumarsäure in Ethanol versetzt. Man erhielt 4,7 g (84%) 5,6,10,11-Tetrahydro-2-methoxy-8-methylbenzo[g]pyrazino[1,2-a]indol-fumarat (1:1); Schmp.: 175-177°C (Zers.).

### Beispiel 64

Unter Argon wurden 0,4 g Triflourmethansulfonsäure-5,6,10,11-tetrahydro-8-methyl-benzo[g]pyrazino[1,2-a]indol-3-yl-ester, 0,37 g wasserfreies Lithiumchlorid, 85 mg Bis(triphenylphosphin)palladiumdichlorid, 0,16 g Triphenylphoshin und 0,3 g Tetramethylzinn in 8 ml Dimethylformamid suspendiert und während 3 h unter Rühren auf 120°C erhitzt. Das Reaktionsgemisch wurde auf 120 ml Wasser gegeben und mit Essigester (1x 60 ml, 1x 50 ml) extrahiert. Die organischen Phasen wurden mit 120 ml gesättigter Natriumchloridlösung gewaschen, vereinigt, mit MgSO₄ getrocknet und vom Lösungsmittel befreit. Der Rückstand, 0,5 g braunes Oel, wurde an 30 g Kieselgel mit Methylenchlorid/Methanol-Gemischen (20:1 bis 10:1) chromatographiert. Man erhielt 0.16 g Rohprodukt, die in der 20fachen Menge Ethanol gelöst und mit 1,1 Aequivalenten einer gesättigten Lösung von Fumarsäure in Ethanol versetzt wurden. Nach Einengen auf ein Volumen von etwa 3 ml und Zugabe von 5 ml Essigester fielen 78 mg (20%) 5,6,10,11-Tetrahydro-3,8-dimethyl-benzo[g]pyrazino[1,2-a]indol-fumarat (1:1); Schmp.: 210-211°C (Zers.).

### Beispiel 65

Unter Argon wurden 0,7 g Triflourmethansulfonsäure-5,6,10,11-tetrahydro-8-methyl-benzo[g]pyrazino[1,2-a]indol-3-yl-ester, 0,65 g wasserfreies Lithiumchlorid, 0,15 g Bis(triphenylphosphin)palladiumdichlorid, 0,29 g Triphenylphoshin und 1,4 g Cyclohex-1-enyl-tri-n-butylzinn in 12 ml Dimethylformamid suspendiert und während 3 h unter Rühren auf 120°C erhitzt. Das Reaktionsgemisch wurde auf 100 ml Wasser gegeben und zweimal mit je 100 ml Essigester extrahiert. Die organischen Phasen wurden mit 50 ml gesättigter Natriumchloridlösung gewaschen, vereinigt, mit MgSO₄ getrocknet und vom Lösungsmittel befreit. Der Rückstand, 1,6 g braunes Oel, wurde an 50 g Kieselgel mit Methylenchlorid/Methanol/Ammoniak-Gemischen (100:3:0,3 bis 100:5:0,5) chromatographiert. 0,15 g von 0,65 g Rohprodukt wurden in der 25fachen Menge Ethanol gelöst und mit 1,1 Aequivalenten einer gesättigten Lösung von Fumarsäure in Ethanol versetzt wurden. Nach Zugabe von 5 ml Essigester fielen 40 mg (19%) 3-Cyclohex-1-enyl-5,6,10,11-tetrahydro-8-methyl-benzo[g]pyrazino[1,2-a]indol-fumarat (2:3); Schmp.: 181-183°C (Zers.).

### Beispiel 66

a) Unter Argon wurden 2,0 g Triflourmethansulfonsäure-5,6,10,11-tetrahydro-8-methyl-benzo[g]pyrazino[1,2-a]indol-3-yl-ester, 1,85 g wasserfreies Lithiumchlorid, 0,44 g Bis(triphenylphosphin)palladiumdichlorid, 0,88 g Triphenylphoshin und 3,9 g Tri-n-butyl-2-cyclohexen-1-yl-zinn in 30 ml Dimethylformamid suspendiert und während 4 h unter Rühren auf 120°C erhitzt. Das Reaktionsgemisch wurde abgekühlt und mit 5 ml Pyridin und 0,6 g Flourwasserstoff-Pyridin-Komplex (60% HF) über Nacht bei Raumtemperatur gerührt. Das Reaktionsgemisch wurde auf 100 ml 2N Natronlauge gegeben und mit 500 ml Diethylether 10 Minuten gerührt. Anschliessend wurde über Celite® filtriert, die wässrige Phase wurde zweimal mit je 100 ml Diethylether extrahiert und die organischen Phasen wurden sukzessive mit 100 ml 2N Natronlauge und 100 ml gesättigter Natriumchloridlösung gewaschen, vereinigt, mit MgSO₄ getrocknet und vom Lösungsmittel befreit. Der Rückstand, 6,8 g gelbes Oel, wurde an 60 g Kieselgel mit Methylenchlorid und Methylenchlorid/Methanol/Ammoniak-Gemischen (30:1:0.1 bis 20:1:0.1) chromatographiert. Man erhielt 1,05 g (64%) 3-Cyclohex-2-enyl-5,6,10,11-tetrahydro-8-methyl-benzo[g]pyrazino[1,2-a]indol als Rohprodukt, das ohne weitere Reinigung weiterverwendet wurde.
b) 1,05 g 3-Cyclohex-2-enyl-5,6,10,11-tetrahydro-8-methyl-benzo[g]pyrazino[1,2-a]indol wurden in 100 ml Methanol gelöst und mit 0,2 ml Methansulfonsäure versetzt. Nach Zugabe von 100 mg Tris(triphenylphosphin)rhodium(I)chlorid wurde 24 Stunden bei Raumtemperatur hydriert. Die Gemisch wurde vom Lösungsmittel befreit, mit 100 ml 10%iger Ammoniaklösung versetzt und dreimal mit je 100 ml Methylenchlorid extrahiert. Die organischen Phasen wurden vereinigt, mit MgSO₄ getrocknet und vom Lösungsmittel befreit. Der Rückstand, 0,95 g gelbes Oel, wurde in der 20fachen Menge Ethanol gelöst und mit 1,1 Aequivalenten einer gesättigten Lösung von Fumarsäure in Ethanol versetzt wurde. Nach Einengen auf ein Volumen von etwa 16 ml und Zugabe von 30 ml Essigester fielen 0,57 g gelbe Kristalle, die aus einem Ethanol/Essigester-Gemisch (1:2) umkristallisiert wurden. Man erhielt 0,39 g (37%) 3-Cyclohexyl-5,6,10,11-tetrahydro-8-methyl-benzo[g]pyrazino[1,2-a]indol-fumarat (2:3); Schmp.: 165-167°C (Zers.).

### Beispiel 67

Unter Argon wurden 2,0 g Triflourmethansulfonsäure-5,6,10,11-tetrahydro-8-methyl-benzo[g]pyrazino[1,2-a]indol-3-yl-ester, 1,85 g wasserfreies Lithiumchlorid, 0,44 g Bis(triphenylphosphin)palladiumdichlorid, 0,88 g Triphenylphoshin und 3,4 ml Tetra-n-butylzinn in 40 ml Dimethylformamid suspendiert und während 6 h unter Rühren auf 120°C erhitzt. Das Reaktionsgemisch wurde abgekühlt und mit 5 ml Pyridin und 0,6 g Flourwasserstoff-Pyridin-Komplex (60% HF) über Nacht bei Raumtemperatur gerührt. Das Reaktionsgemisch wurde auf 100 ml 2N Natronlauge gegeben und mit 500 ml Diethylether 10 Minuten gerührt. Anschliessend wurde über Celite® filtriert, die wässrige Phase wurde zweimal mit je 100 ml Diethylether extrahiert und die organischen Phasen wurden sukzessive mit 100 ml 2N Natronlauge, 100 ml Wasser und 100 ml gesättigter Natriumchloridlösung gewaschen, vereinigt, mit MgSO₄ getrocknet und vom Lösungsmittel befreit. Der Rückstand, 2,2 g gelbes Oel, wurde an 50 g Kieselgel mit Methylenchlorid/Methanol/Ammoniak-Gemischen (30:1:0.1 bis 20:1:0.1) chromatographiert. Man erhielt 0.8 g Rohprodukt, das in der 20fachen Menge Ethanol gelöst und mit 1,1 Aequivalenten einer gesättigten Lösung von Fumarsäure in Ethanol versetzt wurde. Nach Einengen auf ein Volumen von etwa 10 ml und Zugabe von 30 ml Essigester fielen 0,38 g (16%) 3-Butyl-5,6,10,11-tetrahydro-8-methyl-benzo[g]pyrazino[1,2-a]indol-fumarat (2:3); Schmp.: 146-148°C (Zers.).

### Beispiel 68

0,34 g 3-Cyclohexyl-5,6,10,11-tetrahydro-8-methyl-benzo[g]pyrazino[1,2-a]indol wurden unter Argon in einem Gemisch aus 15 ml Methanol und 1,5 ml Wasser gelöst. Die Lösung wurde unter Rühren portionenweise mit 0,15 g Natriumborhydrid versetzt und 3 Stunden bei Raumtemperatur gerührt. Danach wurde das Methanol im Vakuum entfernt und der Rückstand mit 20 ml Ether und 20 ml 10%iger Ammoniaklösung gerührt. Die Phasen wurden getrennt und die wässrige Phase wurde zweimal mit je 20 ml Ether extrahiert. Die organischen Phasen wurden vereinigt, mit MgSO₄ getrocknet und vom Lösungsmittel befreit. Das Rohprodukt, 0,35 g farbloses Oel, wurde in der 20fachen Menge Ethanol gelöst und mit 1,1 Aequivalenten einer gesättigten Lösung von Fumarsäure in Ethanol kurz aufgekocht. Man erhielt 0,11 g (27%) 3-Cyclohexyl-5,6,8,9,10,11-hexahydro-8-methyl-benzo[g]pyrazino[1,2-a]indol-fumarat (2:1); Schmp.: 199-202°C (Zers.).

### Beispiel 69

0,47 g 3-n-Butyl-5,6,10,11-tetrahydro-8-methyl-benzo[g]pyrazino[1,2-a]indol wurden unter Argon in einem Gemisch aus 20 ml Methanol und 2 ml Wasser gelöst. Die Lösung wurde unter Rühren portionenweise mit 0,18 g Natriumborhydrid versetzt und 4 Stunden bei Raumtemperatur gerührt. Danach wurde das Methanol im Vakuum entfernt und der Rückstand mit 50 ml Ether und 20 ml 10%iger Ammoniaklösung gerührt. Die Phasen wurden getrennt und die wässrige Phase wurde einmal mit 50 ml Ether extrahiert. Die organischen Phasen wurden vereinigt, mit MgSO₄ getrocknet und vom Lösungsmittel befreit. Das Rohprodukt, 0,47 g gelbliches Oel, wurde in der 20fachen Menge Ethanol gelöst und mit 1,1 Aequivalenten einer gesättigten Lösung von Fumarsäure in Ethanol kurz aufgekocht. Man erhielt 0,11 g (19%) 3-n-Butyl-5,6,8,9,10,11-hexahydro-8-methyl-benzo[g]pyrazino[1,2-a]indol-fumarat (2:1); Schmp.: 174-176°C (Zers.).

### Beispiel 70

Unter Argon wurden 2,0 g Triflourmethansulfonsäure-5,6,10,11-tetrahydro-8-methyl-benzo[g]pyrazino[1,2-a]indol-3-yl-ester, 1,85 g wasserfreies Lithiumchlorid, 0,44 g Bis(triphenylphosphin)palladiumdichlorid, 0,88 g Triphenylphoshin und 3,5 g Tri-n-butyl-cyclopropyl-zinn in 25 ml Dimethylformamid suspendiert und während 6 h unter Rühren auf 120°C erhitzt. Das Reaktionsgemisch wurde auf 500 ml Wasser gegeben und einmal mit 200 ml und zweimal mit je 100 ml Essigester extrahiert. Die Essigesterextrakte wurden viermal mit 2N Salzsäure (1x100 ml, 3x50 ml) extrahiert, die wässrigen Phasen wurden vereinigt, mit 120 ml 28%iger Natronlauge basisch gestellt und dreimal mit je 100 ml Methylenchlorid extrahiert. Die vereinigten Methylenchlorid-Extrakte wurden zweimal mit je 60 ml gesättigter Kaliumfluoridlösung und einmal mit 500 ml Wasser gewaschen. Die organische Phase wurde mit MgSO₄ getrocknet und vom Lösungsmittel befreit. Der Rückstand, 1,1 g braunes Oel, wurde an 60 g Aluminiumoxid (neutral, III) mit Methylenchlorid als Laufmittel chromatograhiert. Man erhielt 0,67 g Rohprodukt die nochmals an 8 g Kieselgel mit Methylenchlorid/Methanol-Gemischen (50:1 bis 20:1) chromatographiert wurden. Das Produkt, 0,14 g braunes Oel, wurde in der 10fachen Menge Ethanol gelöst und mit 1,1 Aequivalenten einer gesättigten Lösung von Fumarsäure in Ethanol versetzt wurde. Nach Einengen auf ein Volumen von etwa 1 ml und Zugabe von 3 ml Essigester fielen 0,11 g braune Kristalle, die aus 4 ml Isopropanol umkristallisiert wurden, Man erhielt 50 mg (4%) 3-Cyclopropyl-5,6,10,11-tetrahydro-8-methyl-benzo[g]pyrazino[1,2-a]indol-fumarat (1:1); Schmp.: 184-187°C (Zers.).

### Beispiel A

Es werden in üblicher Weise Tabletten folgender Zusammensetzung hergestellt:

| | mg/Tablette |
|---|---|
| Wirkstoff | 100 |
| Lactose pulv. | 95 |
| Maisstärke weiss | 35 |
| Polyvinylpyrrolidon | 8 |
| Na-carboxymethylstärke | 10 |
| Magnesiumstearat | 2 |
| | Tablettengewicht 250 |

### Beispiel B

Es werden in üblicher Weise Tabletten folgender Zusammensetzung hergestellt:

| | mg/Tablette |
|---|---|
| Wirkstoff | 200 |
| Lactose pulv. | 100 |
| Maisstärke weiss | 64 |
| Polyvinylpyrrolidon | 12 |
| Na-carboxymethylstärke | 20 |
| Magnesiumstearat | 4 |
| | Tablettengewicht 400 |

### Beispiel C

Es werden Kapseln folgender Zusammensetzung hergestellt:

| | mg/Kapsel |
|---|---|
| Wirkstoff | 50 |
| Lactose krist. | 60 |
| Mikrokristalline Cellulose | 34 |
| Talk | 5 |
| Magnesiumstearat | 1 |
| | Kapselfüllgewicht 150 |

Der Wirkstoff mit einer geeigneten Partikelgrösse, die Lactose kristallin und die mikrokristalline Cellulose werden homogen miteinander vermischt, gesiebt und danach Talk und Magnesiumstearat zugemischt. Die Endmischung wird in Hartgelatinekapseln geeigneter Grösse abgefüllt.

## Patentansprüche

1. Verbindungen der allgemeinen Formel 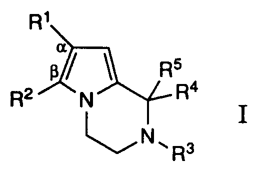 worin eines von R¹ und R² Aryl und das andere Wasserstoff, niederes Alkyl oder Aryl oder R¹ und R² zusammen mit den beiden mit α und β bezeichneten Kohlenstoff-Atomen die Gruppe A; 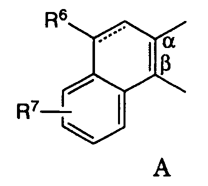 R³ Wasserstoff oder niederes Alkyl, R⁴ Wasserstoff, oder
R³ und R⁴ zusammen eine zusätzliche C/N-Bindung;
R⁵ Wasserstoff oder niederes Alkyl;
R⁶ Wasserstoff oder niederes Alkyl;
R⁷ Wasserstoff, Halogen, niederes Alkyl, gegebenenfalls substituiertes niederes Alkoxy, C₃₋₆-Cycloalkyl, C₄₋₆Cycloalkenyl, C₃₋₆-Cycloalkyloxy, Hydroxy, Trifluormethansulfonyloxy oder gegebenenfalls substituiertes Benzyloxycarbonyloxy; und die punktierte Linie eine fakultative zusätzliche C/C-Bindung bedeuten, und pharmazeutisch annehmbare Säureadditionssalze der Verbindungen der Formel I.

2. Verbindungen nach Anspruch 1, worin eines von R¹ und R² Aryl und das andere Wasserstoffl oder Aryl oder R¹ und R² zusammen mit den beiden mit α und β bezeichneten Kohlenstoff-Atomen die in Anspruch 1 definierte Gruppe A bedeuten, R³-R⁶ sowie die punktierte Linie die in Anspruch 1 angegebene Bedeutung haben und R⁷ Wasserstoff, Halogen, niederes Alkyl, Hydroxy, niederes Alkoxy oder C₃₋₆-Cycloalkyloxy bedeuten, wobei "Aryl" Phenyl oder durch Halogen, niederes Alkoxy, niederes Alkyl, Hydroxy, Trifluormethyl, Phenyl-nieder-Alkoxy, C₃₋₆-Cycloalkyloxy mono- oder oder disubstituiertes Phenyl oder durch niederes Alkylendioxy substituiertes Phenyl bezeichnet.

3. Verbindungen nach Anspruch 1 oder 2, worin R¹ und R² zusammen die Gruppe A bedeutet.

4. Verbindungen nach Anspruch 3, worin R⁷ C₃₋₆-Cycloalkyl, C₃₋₆-Cycloalkyloxy, Halogen, niederes Alkoxy oder niederes Alkyl bedeutet.

5. Verbindungen nach Anspruch 1 oder 2, worin R¹ Wasserstoff und R² Aryl bedeutet.

6. 3,4-Dihydro-1-methyl-6-[p-(2-exonorbornyloxy)phenyl]pyrrolo[1,2-a]pyrazin.

7. 3-Cyclopentyloxy-5,6,10,11-tetrahydro-8-methylbenzo[g]pyrazino[1,2-a]indol.

8. 5,6,10,11-Tetrahydro-3-isopropoxy-8-methylbenzo[g]pyrazino[1,2-a]indol.

9. 3-n-Butyl-5,6,10,11-tetrahydro-8-methylbenzo[g]pyrazino[1,2-a]indol.

10. 3-Cyclohexyl-5,6,10,11-tetrahydro-8-methylbenzo[g]pyrazino[1,2-a]indol.

11. 5,6,10,11-Tetrahydro-3,8-dimethylbenzo[g]pyrazino[1,2-a]indol.

12. Verbindungen der allgemeinen Formel 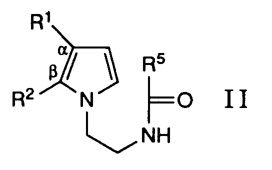 worin R¹,R² und R⁵ die in Anspruch 1 oder 2 angegebene Bedeutung haben, mit der Massgabe, dass R¹ oder R² oder R¹ und R² zusammen keine durch Hydroxy substituierte Gruppe bedeuten.

13. Verbindungen der allgemeinen Formel 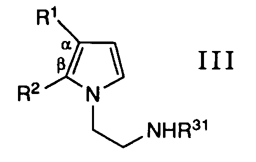 worin R¹ und R² die in Anspruch 1 oder 2 angegebene Bedeutung haben und R³¹ Wasserstoff oder niederes Alkyl bedeutet.

14. Verbindungen der allgemeinen Formel 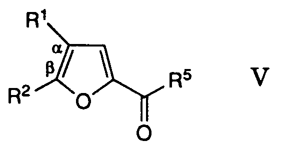 worin R¹, R² und R⁵ die in Anspruch 1 oder 2 angegebene Bedeutung haben.

15. Verbindungen der allgemeinen Formel 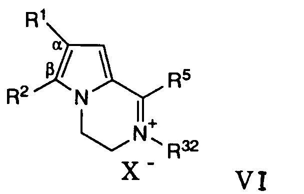 worin R¹, R² und R⁵ die in Anspruch 1 oder 2 angegebene Bedeutung haben und R³² niederes Alkyl und X Halogen bedeutet.

16. Verbindungen nach einem der Ansprüche 1-11 sowie pharmazeutisch annehmbare Säureadditionssalze davon zur Anwendung als therapeutische Wirkstoffe.

17. Verbindungen nach einem der Ansprüche 1-11 sowie pharmazeutisch annehmbare Säureadditionssalze davon zur Anwendung als Antidepressiva oder Mittel zur Behandlung bzw. Verhütung von kognitiven Erkrankungen oder zur Behandlung von neurodegenerativen Erkrankungen, als Antiparkinsonmittel oder als Antialzheimermittel.

18. Verfahren zur Herstellung von Verbindungen nach einem der Ansprüche 1-11 und von pharmazeutisch annehmbaren Säureadditionsalzen davon, dadurch gekennzeichnet, dass man
a) eine Verbindung der allgemeinen Formel 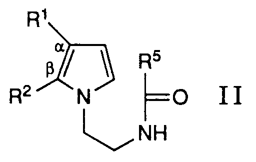 worin R¹ , R² und R⁵ obige Bedeutung haben, mit der Massgabe, dass R¹ oder R² oder R¹ und R² zusammen keine durch Hydroxy, Trifluormethansulfonyloxy oder C₄₋₆-Cycloalkenyl substituierte Gruppe bedeuten, cyclisiert, oder
b) eine Verbindung der allgemeinen Formel 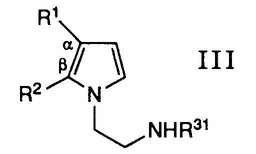 worin R¹ und R² obige Bedeutung haben und R³¹ Wasserstoff oder niederes Alkyl bedeutet, mit der Massgabe, dass R¹ oder R² oder R¹ und R² zusammen keine Gruppe bedeuten, welche durch Trifluormethansulfonyloxy oder C₄₋₆-Cycloalkenyl substitutiert ist, mit einem Aldehyd der Formel
R⁵-CHO IV
wobei R⁵ die obige Bedeutung hat, umsetzt; oder
c) eine Verbindung der allgemeinen Formel 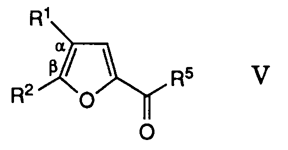 worin R¹, R² und R⁵ obige Bedeutung haben, mit 1,2-Ethandiamin umsetzt, oder
d) eine Verbindung der Formel 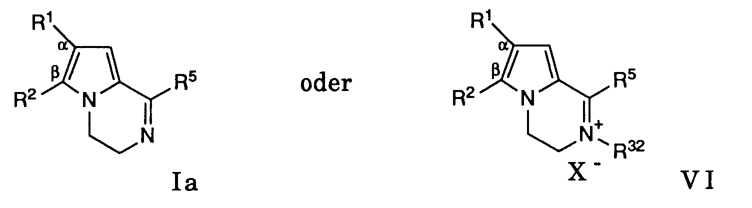 worin R¹, R² und R⁵ obige Bedeutung haben und R³² niederes Alkyl und X Halogen bedeutet, reduziert, oder
e) eine alkoxysubstituierte Verbindung der Formel I einer Etherspaltung unterzieht, oder
f) eine Verbindung der Formel I, worin R¹ und/oder R² oder R¹ und R² zusammen eine durch phenolisches Hydroxy substituierte Gruppe darstellen, in eine entsprechende Verbindung der Formel I überführt, worin R¹ und/oder R² oder R¹ und R² zusammen eine durch gegebenenfalls substituiertes Alkoxy, durch Phenylalkyloxy, durch C₃₋₆Cycloalkyoxy oder durch Bicycloalkyloxy, substituierte Gruppe darstellen , oder
g) eine Verbindung der Formel I, worin R¹ und/oder R² oder R¹ und R² zusammen eine durch phenolisches Hydroxy substituierte Gruppe darstellen, in eine entsprechende durch Trifluormethansulfonyloxy substituierte Verbindung überführt, oder
h) eine Verbindung der Formel I, worin R¹ und/oder R² oder R¹ und R² zusammen eine durch Trifluormethansulfonyloxy substituierte Gruppe bedeuten, in eine entsprechende durch niederes Alkyl, Cyclopropyl oder C₄₋₆-Cycloalkenyl substituierte Verbindung überführt, oder
i) eine durch gegebenenfalls substituiertes Benzyloxycarbonyloxy substituierte Verbindung der Formel I spaltet, oder
j) eine durch C₄₋₆-Cycloalken substituierte Verbindung der Formel I hydriert, und
k) erwünschtenfalls eine erhaltene Verbindung der Formel I in ein pharmazeutisch annehmbares Säureadditionssalz überführt.

19. Arzneimittel enthaltend eine Verbindung nach einem der Ansprüche 1-11 und ein therapeutisch inertes Trägermaterial.

20. Arzneimittel zur Bekämpfung oder Verhütung von depressiven Zuständen, kognitiven Erkrankungen und neurodegenerativen Erkrankungen, wie der Parkinsonschen Krankheit und der Alzheimerschen Krankheit, enthaltend eine Verbindung nach einem der Ansprüche 1-11 und ein therapeutisch inertes Trägermaterial.

21. Verwendung von Verbindungen nach einem der Ansprüche 1-11 zur Herstellung von Mitteln zur Bekämpfung oder Verhütung von depressiven Zuständen, kognitiven Erkrankungen und neurodegenerativen Erkrankungen wie der Parkinsonschen Krankheit und der Alzheimerschen Krankheit.

## Patentansprüche (Patentansprüche für folgende(n) Vertragsstaat(en): ES)

1. Verfahren zur Herstellung von Verbindungen der allgemeinen Formel 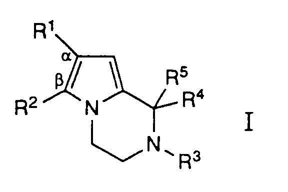 worin eines von R¹ und R² Aryl und das andere Wasserstoff, niederes Alkyl oder Aryl oder R¹ und R² zusammen mit den beiden mit α und β bezeichneten Kohlenstoff-Atomen die Gruppe A; 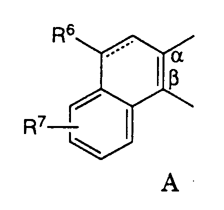 R³ Wasserstoff oder niederes Alkyl, R⁴ Wasserstoff, oder
R³ und R⁴ zusammen eine zusätzliche C/N-Bindung;
R⁵ Wasserstoff oder niederes Alkyl;
R⁶ Wasserstoff oder niederes Alkyl;
R⁷ Wasserstoff, Halogen, niederes Alkyl, gegebenenfalls substituiertes niederes Alkoxy, C₃₋₆-Cycloalkyl, C₄₋₆-Cycloalkenyl, C₃₋₆-Cycloalkyloxy, Hydroxy, Trifluormethansulfonyloxy oder gegebenenfalls substituiertes Benzyloxycarbonyloxy; und die punktierte Linie eine fakultative zusätzliche C/C-Bindung bedeuten, und von pharmazeutisch annehmbaren Säureadditionssalzen der Verbindungen der Formel I,
dadurch gekennzeichnet, dass man
a) eine Verbindung der allgemeinen Formel 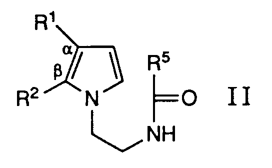 worin R¹ ,R² und R⁵ obige Bedeutung haben, mit der Massgabe, dass R¹ oder R² oder R¹ und R² zusammen keine durch Hydroxy, Trifluormethansulfonyloxy oder C₄₋₆-Cycloalkenyl substituierte Gruppe bedeuten, cyclisiert, oder
b) eine Verbindung der allgemeinen Formel 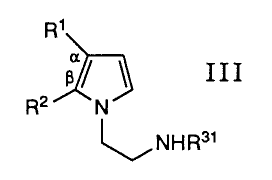 worin R¹ und R² obige Bedeutung haben und R³¹ Wasserstoff oder niederes Alkyl bedeutet,mit der Massgabe, dass R¹ oder R² oder R¹ und R² zusammen keine Gruppe bedeuten, welche durch Trifluormethansulfonyloxy oder C₄₋₆-Cycloalkenyl substituiert ist, mit einem Aldehyd der Formel
R⁵-CHO IV
wobei R⁵ die obige Bedeutung hat, umsetzt; oder
c) eine Verbindung der allgemeinen Formel 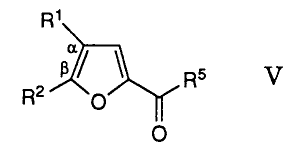 worin R¹, R² und R⁵ obige Bedeutung haben, mit 1,2-Ethandiamin umsetzt, oder
d) eine Verbindung der Formel 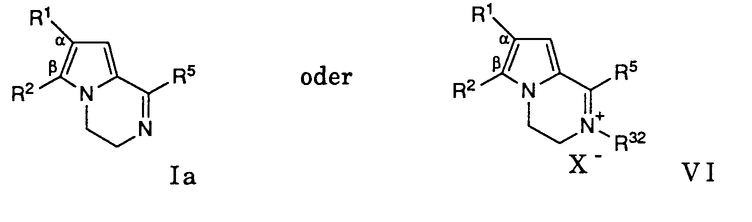 worin R¹, R² und R⁵ obige Bedeutung haben und R³² niederes Alkyl und X Halogen bedeutet, reduziert, oder
e) eine alkoxysubstituierte Verbindung der Formel I einer Etherspaltung unterzieht, oder
f) eine Verbindung der Formel I, worin R¹ und/oder R² oder R¹ und R² zusammen eine durch phenolisches Hydroxy substituierte Gruppe darstellen, in eine entsprechende Verbindung der Formel I überführt, worin R¹ und/oder R² oder R¹ und R² zusammen eine durch gegebenenfalls substituiertes Alkoxy, durch Phenylalkyloxy, durch C₃₋₆-Cycloalkyoxy oder durch Bicycloalkyloxy, substituierte Gruppe darstellen, oder
g) eine Verbindung der Formel I, worin R¹ und/oder R² oder R¹ und R² zusammen eine durch phenolisches Hydroxy substituierte Gruppe darstellen, in eine entsprechende durch Trifluormethansulfonyloxy substituierte Verbindung überführt, oder
h) eine Verbindung der Formel I, worin R¹ und/oder R² oder R¹ und R² zusammen eine durch Trifluormethansulfonyloxy substituierte Gruppe bedeuten, in eine entsprechende durch niederes Alkyl, Cyclopropyl oder C₄₋₆-Cycloalkenyl substituierte Verbindung überführt, oder
i) eine durch gegebenenfalls substituiertes Benzyloxycarbonyloxy substituierte Verbindung der Formel I spaltet, oder
j)eine durch C₄₋₆-Cycloalken substituierte Verbindung der Formel I hydriert, und
k) erwünschtenfalls eine erhaltene Verbindung der Formel I in ein pharmazeutisch annehmbares Säureadditionssalz überführt.

2. Verfahren nach Anspruch 1, dadurch gekennzeichnet, dass eines von R¹ und R² Aryl und das andere Wasserstoff oder Aryl oder R¹ und R² zusammen mit den beiden mit α und β bezeichneten Kohlenstoff-Atomen die in Anspruch 1 definierte Gruppe A bedeuten, R³-R⁶ sowie die punktierte Linie die in Anspruch 1 angegebene Bedeutung haben und R⁷ Wasserstoff, Halogen, niederes Alkyl, Hydroxy, niederes Alkoxy oder C₃₋₆-Cycloalkyloxy bedeuten, wobei "Aryl" Phenyl oder durch Halogen, niederes Alkoxy, niederes Alkyl, Hydroxy, Trifluormethyl, Phenyl-nieder-Alkoxy, C₃₋₆-Cycloalkyloxy mono- oder disubstituiertes Phenyl oder durch niederes Alkylendioxy substituiertes Phenyl bezeichnet, und dass man nach den in Anspruch 1 angegebenen Verfahrensvarianten a) bis f), und erwünschtenfalls k) arbeitet.

3. Verfahren nach Anspruch 1 oder 2, dadurch gekennzeichnet, dass R¹ und R² zusammen die Gruppe A bedeutet.

4. Verfahren nach Anspruch 3, dadurch gekennzeichnet, dass R⁷ C₃₋₆-Cycloalkyl, C₃₋₆-Cycloalkyloxy, Halogen, niederes Alkoxy oder niederes Alkyl bedeutet.

5. Verfahren nach Anspruch 1 oder 2, dadurch gekennzeichnet, dass R¹ Wasserstoff und R² Aryl bedeutet.

6. Verfahren nach Anspruch 1, dadurch gekennzeichnet, dass man 3,4-Dihydro-1-methyl-6-[p-(2-exonorbornyloxy)phenyl]pyrrolo-[1,2-a]pyrazin herstellt.

7. Verfahren nach Anspruch 1, dadurch gekennzeichnet, dass man 3-Cyclopentyloxy-5,6,10,11-tetrahydro-8-methylbenzo[g]pyrazino[1,2-a]indol herstellt.

8. Verfahren nach Anspruch 1, dadurch gekennzeichnet, dass man 5,6,10,11-Tetrahydro-3-isopropoxy-8-methylbenzo[g]pyrazin-[1,2-a]indol herstellt.

9. Verfahren nach Anspruch 1, dadurch gekennzeichnet, dass man 3-n-Butyl-5,6,10,11-tetrahydro-8-methylbenzo[g]pyrazino-[1,2-a]indol herstellt.

10. Verfahren nach Anspruch 1, dadurch gekennzeichnet, dass man 3-Cyclohexyl-5,6,10,11-tetrahydro-8-methylbenzo[g]pyrazino[1,2-a]indol herstellt.

11. Verfahren nach Anspruch 1, dadurch gekennzeichnet, dass man 5,6,10,11-Tetrahydro-3,8-dimethylbenzo[g]pyrazino[1,2-a]indol herstellt.

12. Verfahren zur Herstellung von Arzneimitteln, insbesondere zur Bekämpfung oder Verhütung von depressiven Zuständen, kognitiven Erkrankungen und neurodegenerativen Erkrankungen wie der Parkinsonschen Krankheit und der Alzheimerschen Krankheit, dadurch gekennzeichnet, dass man eine Verbindung der in Anspruch 1 angegebenen Formel I oder ein pharmazeutisch annehmbares Säureadditionssalz davon und erwünschtenfalls einen anderen therapeutisch wirksamen Stoff zusammen mit einem therapeutisch inerten Trägermaterial in eine galenische Darreichungsform bringt.

13. Verwendung von Verbindungen der in Anspruch 1 angegebenen Formel I oder von pharmazeutisch annehmbaren Säureadditionssalzen davon zur Herstellung von Mitteln zur Bekämpfung oder Verhütung von depressiven Zuständen, kognitiven Erkrankungen und neurodegenerativen Erkrankungen wie der Parkinsonschen Krankheit und der Alzheimerschen Krankheit.

## Patentansprüche (Patentansprüche für folgende(n) Vertragsstaat(en): GR)

1. Verfahren zur Herstellung von Verbindungen der allgemeinen Formel 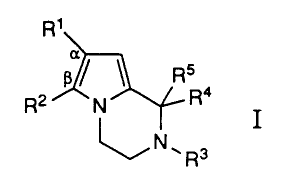 worin eines von R¹ und R² Aryl und das andere Wasserstoff, niederes Alkyl oder Aryl oder R¹ und R² zusammen mit den beiden mit α und β bezeichneten Kohlenstoff-Atomen die Gruppe A; 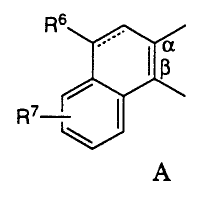 R³ Wasserstoff oder niederes Alkyl, R⁴ Wasserstoff, oder
R³ und R⁴ zusammen eine zusätzliche C/N-Bindung;
R⁵ Wasserstoff oder niederes Alkyl;
R⁶ Wasserstoff oder niederes Alkyl;
R⁷ Wasserstoff, Halogen, niederes Alkyl, gegebenenfalls substituiertes niederes Alkoxy , C₃₋₆-Cycloalkyl, C₄₋₆-Cycloalkenyl, C₃₋₆-Cycloalkyloxy, Hydroxy, Trifluormethansulfonyloxy oder gegebenenfalls substituiertes Benzyloxycarbonyloxy; und die punktierte Linie eine fakultative zusätzliche C/C-Bindung bedeuten, und von pharmazeutisch annehmbaren Säureadditionssalzen der Verbindungen der Formel I,
dadurch gekennzeichnet, dass man
a) eine Verbindung der allgemeinen Formel 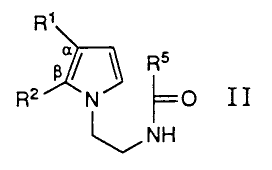 worin R¹ ,R² und R⁵ obige Bedeutung haben, mit der Massgabe, dass R¹ oder R² oder R¹ und R² zusammen keine durch Hydroxy, Trifluormethansulfonyloxy oder C₄₋₆-Cycloalkenyl substituierte Gruppe bedeuten, cyclisiert, oder
b) eine Verbindung der allgemeinen Formel 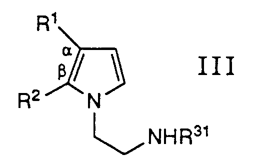 worin R¹ und R² obige Bedeutung haben und R³¹ Wasserstoff oder niederes Alkyl bedeutet,mit der Massgabe, dass R¹ oder R² oder R¹ und R² zusammen keine Gruppe bedeuten, welche durch Trifluormethansulfonyloxy oder C₄₋₆-Cycloalkenyl substituiert ist, mit einem Aldehyd der Formel
R⁵-CHO IV
wobei R⁵ die obige Bedeutung hat, umsetzt; oder
c) eine Verbindung der allgemeinen Formel 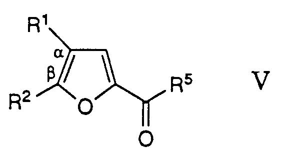 worin R¹, R² und R⁵ obige Bedeutung haben, mit 1,2-Ethandiamin umsetzt, oder
d) eine Verbindung der Formel 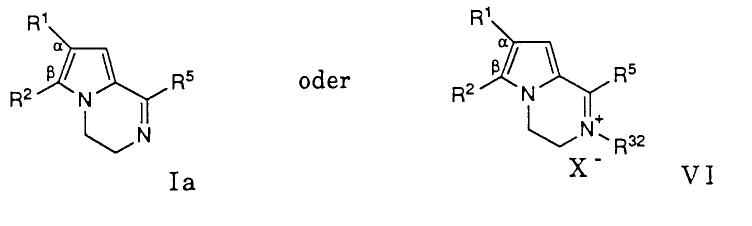 worin R¹, R² und R⁵ obige Bedeutung haben und R³² niederes Alkyl und X Halogen bedeutet, reduziert, oder
e) eine alkoxysubstituierte Verbindung der Formel I einer Etherspaltung unterzieht, oder
f) eine Verbindung der Formel I, worin R¹ und/oder R² oder R¹ und R² zusammen eine durch phenolisches Hydroxy substituierte Gruppe darstellen, in eine entsprechende Verbindung der Formel I überführt, worin R¹ und/oder R² oder R¹ und R² zusammen eine durch gegebenenfalls substituiertes Alkoxy, durch Phenylalkyloxy, durch C₃₋₆-Cycloalkyoxy oder durch Bicycloalkyloxy, substituierte Gruppe darstellen, oder
g) eine Verbindung der Formel I, worin R¹ und/oder R² oder R¹ und R² zusammen eine durch phenolisches Hydroxy substituierte Gruppe darstellen, in eine entsprechende durch Trifluormethansulfonyloxy substituierte Verbindung überführt, oder
h) eine Verbindung der Formel I, worin R¹ und/oder R² oder R¹ und R² zusammen eine durch Trifluormethansulfonyloxy substituierte Gruppe bedeuten, in eine entsprechende durch niederes Alkyl, Cyclopropyl oder C₄₋₆-Cycloalkenyl substituierte Verbindung überführt, oder
i) eine durch gegebenenfalls substituiertes Benzyloxycarbonyloxy substituierte Verbindung der Formel I spaltet, oder
j) eine durch C₄₋₆-Cycloalken substituierte Verbindung der Formel I hydriert, und
k) erwünschtenfalls eine erhaltene Verbindung der Formel I in ein pharmazeutisch annehmbares Säureadditionssalz überführt.

2. Verfahren nach Anspruch 1, dadurch gekennzeichnet, dass eines von R¹ und R² Aryl und das andere Wasserstoff oder Aryl oder R¹ und R² zusammen mit den beiden mit α und β bezeichneten Kohlenstoff-Atomen die in Anspruch 1 definierte Gruppe A bedeuten, R³-R⁶ sowie die punktierte Linie die in Anspruch 1 angegebene Bedeutung haben und R⁷ Wasserstoff, Halogen, niederes Alkyl, Hydroxy, niederes Alkoxy oder C₃₋₆-Cycloalkyloxy bedeuten, wobei "Aryl" Phenyl oder durch Halogen, niederes Alkoxy, niederes Alkyl, Hydroxy, Trifluormethyl, Phenyl-nieder-Alkoxy, C₃₋₆-Cycloalkyloxy mono- oder disubstituiertes Phenyl oder durch niederes Alkylendioxy substituiertes Phenyl bezeichnet, und dass man nach den in Anspruch 1 angegebenen Verfahrensvarianten a) bis f), und erwünschtenfalls k) arbeitet.

3. Verfahren nach Anspruch 1 oder 2, dadurch gekennzeichnet, dass R¹ und R² zusammen die Gruppe A bedeutet.

4. Verfahren nach Anspruch 3, dadurch gekennzeichnet, dass R⁷ C₃₋₆-Cycloalkyl, C₃₋₆-Cycloalkyloxy, Halogen, niederes Alkoxy oder niederes Alkyl bedeutet.

5. Verfahren nach Anspruch 1 oder 2, dadurch gekennzeichnet, dass R¹ Wasserstoff und R² Aryl bedeutet.

6. Verfahren nach Anspruch 1, dadurch gekennzeichnet, dass man 3,4-Dihydro-1-methyl-6-[p-(2-exonorbornyloxy)phenyl]pyrrolo-[1,2-a]pyrazin herstellt.

7. Verfahren nach Anspruch 1, dadurch gekennzeichnet, dass man 3-Cyclopentyloxy-5,6,10,11-tetrahydro-8-methylbenzo[g]pyrazino[1,2-a]indol herstellt.

8. Verfahren nach Anspruch 1, dadurch gekennzeichnet, dass man 5,6,10,11-Tetrahydro-3-isopropoxy-8-methylbenzo[g]pyrazino-[1,2-a]indol herstellt.

9. Verfahren nach Anspruch 1, dadurch gekennzeichnet, dass man 3-n-Butyl-5,6,10,11-tetrahydro-8-methylbenzo[g]pyrazino-[1,2-a]indol herstellt.

10. Verfahren nach Anspruch 1, dadurch gekennzeichnet, dass man 3-Cyclohexyl-5,6,10,11-tetrahydro-8-methylbenzo[g]pyrazino[1,2-a]indol herstellt.

11. Verfahren nach Anspruch 1, dadurch gekennzeichnet, dass man 5,6,10,11-Tetrahydro-3,8-dimethylbenzo[g]pyrazino[1,2-a]indol herstellt.

12. Verfahren zur Herstellung von Arzneimitteln, insbesondere zur Bekämpfung oder Verhütung von depressiven Zuständen, kognitiven Erkrankungen und neurodegenerativen Erkrankungen wie der Parkinsonschen Krankheit und der Alzheimerschen Krankheit, dadurch gekennzeichnet, dass man eine Verbindung der in Anspruch 1 angegebenen Formel I oder ein pharmazeutisch annehmbares Säureadditionssalz davon und erwünschtenfalls einen anderen therapeutisch wirksamen Stoff zusammen mit einem therapeutisch inerten Trägermaterial in eine galenische Darreichungsform bringt.

13. Verwendung von Verbindungen der in Anspruch 1 angegebenen Formel I oder von pharmazeutisch annehmbaren Säureadditionssalzen davon zur Herstellung von Mitteln zur Bekämpfung oder Verhütung von depressiven Zuständen, kognitiven Erkrankungen und neurodegenerativen Erkrankungen wie der Parkinsonschen Krankheit und der Alzheimerschen Krankheit.

14. Verbindungen der allgemeinen Formel 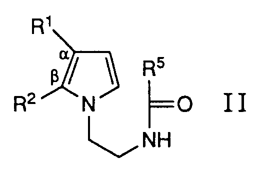 worin R¹, R² und R⁵ die in Anspruch 1 oder 2 angegebene Bedeutung haben, mit der Massgabe, dass R¹ oder R² oder R¹ und R² zusammen keine durch Hydroxy substituierte Gruppe bedeuten.

15. Verbindungen der allgemeinen Formel 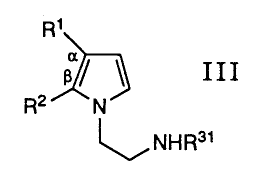 worin R¹ und R² die in Anspruch 1 oder 2 angegebene Bedeutung haben und R³¹ Wasserstoff oder niederes Alkyl bedeutet.

16. Verbindungen der allgemeinen Formel 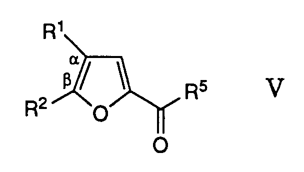 worin R¹, R² und R⁵ die in Anspruch 1 oder 2 angegebene Bedeutung haben.

17. Verbindungen der allgemeinen Formel 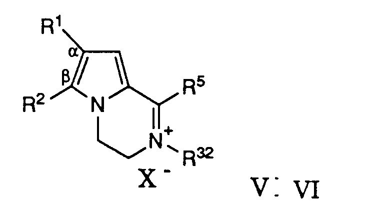 worin R¹, R² und R⁵ die in Anspruch 1 oder 2 angegebene Bedeutung haben und R³² niederes Alkyl und X Halogen bedeutet.
